(19) Europäisches Patentamt
European Patent Office
Office européen des brevets

(11) EP 2 829 880 A1

(12) EUROPEAN PATENT APPLICATION

(43) Date of publication:
**28.01.2015 Bulletin 2015/05**

(51) Int Cl.:
***G01N 33/574*** *(2006.01)*

(21) Application number: **13178291.4**

(22) Date of filing: **26.07.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Energeia Biosciences**
**4931 DL Geertruidenberg (NL)**

(72) Inventor: **Breedveld, Pauline**
**4931 DL Geertruidenberg (NL)**

(74) Representative: **Prüfer & Partner GbR**
**European Patent Attorneys**
**Sohnckestraße 12**
**81479 München (DE)**

# (54) Method for identifying modulators of BCRP/ABCG2-mediated ATP release and use of said modulators for treating diseases

(57) The present invention belongs to the field of pharmaceutical industry and relates to an in vitro method for identifying test compounds which are capable of modulating adenosine triphosphate (ATP) release from cells by inhibiting or stimulating the Adenosine triphosphate-Binding Cassette Transporter (ABC Transporter) Breast Cancer Resistance Protein (BCRP; ABCG2)-mediated transport of ATP. The invention further relates to compounds having the above capability for treating specific diseases.

**Fig. 6**

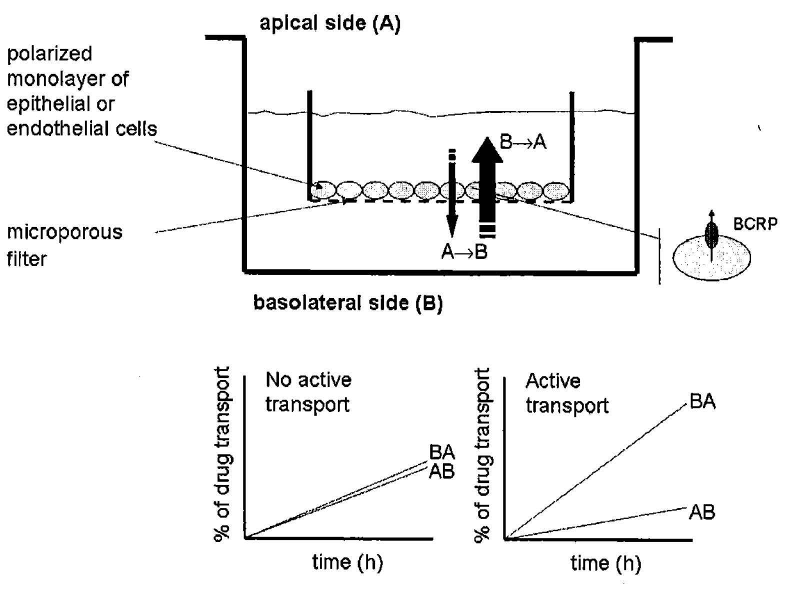


EP 2 829 880 A1

## Description

Field of the invention

The present invention belongs to the field of pharmaceutical industry and relates to an in vitro method for identifying test compounds which are capable of modulating Adenosine triphosphate (ATP) release from cells by inhibiting or stimulating ATP-Binding Cassette Transporter (ABC Transporter)-mediated transport of ATP. Further, the invention relates to compositions comprising said compounds for use in treating specific diseases, and to pharmaceutical compositions comprising said compounds and an antigen.

Description of the background art

The intracellular role of the purine nucleotide adenosine 5'-triphosphate (ATP) has been recognized for many years. ATP is an energy source for many important reactions; a large amount of free energy is liberated when ATP is hydrolyzed to adenosine diphosphate (ADP) or when ATP is hydrolyzed to adenosine monophosphate (AMP). Cells have complex mechanisms for synthesizing ATP and maintaining or rapidly restoring the intracellular level, and one might therefore expect that release of ATP to the extracellular milieu would rarely occur. However, nowadays the role of extracellular ATP in purinergic signaling, i.e. ATP acting directly on cell surface purinergic P2 receptors, or indirectly on P1 receptors via adenosine, is widely accepted (1). Moreover, under pathophysiologic conditions, i.e. in cases of cellular stress, ATP and its metabolites are released in the extracellular environment from several cell types (2).

Cellular stress (leading to ATP release) can be caused by many factors: e.g. low pH, such as exists in the tumor microenvironment, endosomes, lysosomes, mitochondrial matrix, and in blood in case of acidosis (acidic stress) (3); reduced oxygen availability (hypoxia); oxygen deprivation due to a disruption in blood flow (ischemia); cellular invasion of pathogens or toxins (4); crystalline compounds (such as crystalline aluminium oxyhydroxide , cholesterol crystals (5), and uric acid crystals); osmotic changes, such as hypotonic conditions; excessive formation of reactive oxygen species (oxidative stress); heat or cold (thermal stress); mechanical factors (shear stress).

Despite great interest in the mechanisms that regulate cellular ATP permeability, the molecular basis for epithelial ATP transport is currently unknown. This has recently also been addressed in a review about "mechanisms of ATP release and signaling in the blood vessel wall" (6).

ATP release mechanisms

Extracellular nucleotides and nucleosides promote a vast range of physiological responses, via activation of cell surface purinergic receptors. Responses to nucleotides and nucleosides are observed in the CNS and all peripheral tissues, indicating that nucleotide release underlies many important physiological processes. Virtually all tissues and cell types exhibit regulated release of ATP (1).

In the past, several mechanisms of ATP release have been suggested and/or investigated, but firm conclusions could not be drawn thus far, because contradictory results have been found. One obvious source of extracellular ATP is cell lysis, which occurs when massive cell death takes place during injury or inflammation. In this case, ATP originates from injured cells, macrophages or lymphocytes at the inflammation site. A non-lytic source of ATP occurs in some secretory cell types, where ATP is released from secretory granules or vesicles (6). However, ATP release takes place even when cell damage or vesicular exocytosis does not occur. For example, it has been suggested that non-lytic ATP release takes place via gap junction(-like) channels (connexin hemichannels; pannexins) or via plasma lemmal voltage-dependent anion channels. Although many studies thus have addressed ATP release under physiological and pathophysiological conditions, some studies provide evidence for and other studies against the release of ATP by the studied channel (7, 8,9). Also a role for the Cystic Fibrosis Transmembrane Conductance Regulator (CFTR; ABCC7), facilitating ATP release by stimulation of a separate ATP release channel, was suggested (10), but in other studies CFTR failed to conduct ATP (11,12, 6). The same applies to the ABC transporter P-gp (MDR). Abraham et al. showed that P-gp substrates are associated with movement of ATP from the cytoplasm to the extracellular space, and suggested that P-gp co-transports ATP and P-gp substrates. As was shown for the drug doxorubicin, ATP and drug movement were coupled (13). However, also ADP and AMP could both effectively couple to doxorubicin (13). In contrast, Grygorczyk and Hanrahan could find no evidence that ATP release was related to Pgp (or CFTR) expression (11). These studies had a large impact in silencing the notion that ABC transporters may be involved in releasing ATP from cells.

One explanation for these controversial findings may be that ATP is highly sensitive to hydrolysis by ecto-ATPases at the cell surface. Another explanation may be that the firefly luciferase luminescence assay, which is a widely used method to measure ATP content, is a sensitive method, and a number of variables need to be taken into account for reliable results, for example the concentration of luciferin in the reaction mixture has to be appropriate for the levels of ATP present in the cells. Furthermore, quantification of ATP in cell supernatants can easily be confounded by stimuli that induce cell lysis. Because cells contain millimolar amounts of cytosolic ATP (appr. 2-10 mM), rupture of even a single cell in a given preparation can release a sufficient amount of ATP to elicit physiological effects (i.e. Ca2+ wave propagation) and may dramatically skew the quantification and interpretation of ATP released from cells. Another explanation may be that ATP release by the ABC transporters CFTR and P-gp is studied in e.g. Chinese hamster ovary (CHO) cells and red blood cells (RBCs), that express besides the transporter of interest multiple other transporters. However, none of the studies show direct transport of ATP by the transporter or channel, which is a prerequisite to demonstrate that the transported nucleotide is indeed ATP and not ADP or AMP.

More recently, a novel study implicating CFTR in acidosis-induced ATP release from skeletal muscle cells has re-ignited the potential for ABC transporters in releasing ATP. In this study, the authors noted a significant decrease in the amount of ATP release from acidotic skeletal muscle cells upon blockade of CFTR with the selective CFTR inhibitor $CFTR_{inh}$ 172 and by selective knockdown of CFTR with siRNA (7). However, CFTR was unlikely to account for the basal (unstimulated) ATP release: silencing the CFTR gene using RNA interference significantly decreased CFTR protein expression in the myoblasts, but did not reduce the accumulation of ATP in the bathing medium at pH 7.4. The research by Tu et al. demonstrated that CFTR plays an obligatory role in the acidosis-induced efflux of ATP, but they have also not addressed whether or not CFTR itself conducts ATP (7).

In conclusion, ATP release by endothelial cells is until now still a matter of debate (6,7) (8). The exact mechanism of (non-lytic) ATP release has not been elucidated yet.

<u>Role of extracellular ATP in inflammation and apoptosis</u>

Recently, it has been found that ATP release (e.g. from dying, injured and stressed cells) into the extracellular milieu is the first step in the cascade of events leading to NLRP3 (also known as NALP3, cryopyrin, CIAS1, or PYPAF1) inflammasome activation, caspase-1 activation, and IL-1β, IL-18 and IL-33 secretion, which is critical for the inflammatory response. A key function of caspase-1 is the cleavage of inflammatory cytokines IL-1β and IL-18 from their inactive precursor molecules (pro-IL-1β and pro-IL-18) into mature biologically active forms (IL-1β and IL-18) that can be released from cells. IL-1β is one of the most potent proinflammatory cytokines that functions in the generation of systemic and localized responses to infection (14), injury, and inflammatory challenges. The release of these inflammatory mediators leads to recruitment of immature monocytes and dendritic cells. Once released, extracellular ATP serves as a danger signal to alert the immune system by acting on purinergic receptors, i.e. P2Y and P2X receptors, including $P_2X_7$ receptors from dendritic cells (1, 8, 15, 2).

ATP cannot be transported back into the cell but is rapidly degraded to adenosine by several endonucleotidases before re-uptake. Also adenosine has an established role in immunity, in which it may contribute to the engineering of inflammation and immune responses by providing a suppressive tissue-protecting signal in a delayed, negative feedback manner.

In addition, it has recently been reported that ATP modulates activated and regulatory $CD4^+$ T cells (which play an important role in T-cell mediated inflammation) via purinergic P2 receptor activation and this modulation was dose-dependent (16). Physiological concentrations of extracellular ATP (1-50 nM) did not affect activated CD4+ cells and Tregs (regulatory T cells). Conversely, higher ATP concentrations had a bimodal effect on activated CD4+ cells. Whereas 250 nM ATP stimulated proliferation, cytokine release, expression of adhesion molecules, and adhesion, 1 mM ATP induced apoptosis and inhibited CD4+ T cell functions. Extracellular ATP concentration highly increased in fast-growing tumors or hyperinflammed tissues (16). ATP is required for the progression of apoptosis; defects in programmed cell death (apoptosis) mechanisms play important roles in tumor pathogenesis, allowing neoplastic cells to survive beyond their normally intended lifespans, providing protection from hypoxia and oxidative stress as tumor mass expands. Thus, extracellular ATP is a pivotal player in driving CD4+ T cell activity, and modulation of ATP release from cells is an apoptosis-targeted therapy for cancer treatment and may have an important role in inflammation and tumor cell growth.

Thus, by activation of the NLRP3 inflammasome and purinergic receptors present on immune cells such as DCs and regulatory $CD4^+$ T cells, extracellular ATP and its breakdown product adenosine are involved in many inflammatory/immune-mediated diseases, such as rheumatoid arthritis (17), atherosclerosis and ischemia (5), cancer (15), asthma or chronic obstructive pulmonary disease (2), endotoxemia and sepsis (18), graft-versus-host disease (19), and neurodegenerative disorders, including Alzheimer disease, depression (20), and infectious diseases, such as malaria (21)and influenza (22).

With this regard, WO2005/112947 refers to the use of ATP for the manufacture of a medicine comprising ATP as an active ingredient for exerting a preventive or therapeutic pharmacological effect when administered to a mammal.

Although degranulation (i.e. exocytosis) is the process by which nucleotides are released from platelets, neurones and cells of the adrenal medulla, most other cells (which contain ATP in their cytoplasm and mitochondria, but not in storage molecules) must release nucleotides by a different mechanism.

All in all, following from the above, facing the multiple roles ATP plays in multiple diseases such as inflammatory and infectious diseases, it would be of benefit having a test system which is able to identify compounds that are able to modulate ATP release from cells. These compounds may then be used in treating diseases that are related to extracellular ATP concentration.

Hence, it was an object of the present invention to provide an in vitro test system for identifying compounds that are capable of modulating ATP release from cells by inhibiting or modulating the ABC Transporter.

Further, it was an object of the present invention to provide compositions of compounds that are capable of modulating said ATP release, for use in treating certain, ATP-related, diseases.

Summary of the invention

The present invention provides the following aspects, subject-matters and preferred embodiments, which respectively taken alone or in combination, contribute to solving the object of the present invention.

1. In vitro method for identifying test compounds which are capable of modulating adenosine triphosphate (ATP) release from cells by inhibiting or stimulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, comprising the steps of

   a) providing a test system comprising said ABC Transporter, wherein this test system includes at least two compartments and wherein the ABC Transporter is able to transport ATP between said at least two compartments,
   b) contacting at least one of the compartments of the test system of step a) with a test compound in such a manner and for a time period being sufficient so that the test compound comes in contact with said ABC Transporter,
   wherein prior to, during or after the test compound has been brought into contact with at least one of said compartments, ATP is added to at least one of the compartments
   of the test system from which it can be transported to other compartment(s),
   c) determining amount(s) of ATP in at least one of the compartments of the test system to which or from which the ATP is transported,
   d) comparing the determined ATP amount(s) of step (c) with ATP amount(s) determined by using the same test system

      A) at (a) different concentration(s) of the test compound;
      B) in the absence of the test compound; and/or
      C) with a reference compound instead of the test compound;
      and

   e) concluding from the comparison in (d) whether the test compound is capable of modulating ATP release from cells by inhibiting or stimulating, preferably inhibiting, said ABC Transporter-mediated transport of ATP,
   wherein the pH value of at least the compartment to which the test compound is added is about 7 or lower at the time point at which the test compound is added.

The ABC Transporter modulators that are used in the in vitro method according to the present invention can be any ABC Transporter modulator; for example the ABC Modulator Transporter is one or more of:

- BCRP, MRP2, MRP3, MRP4, MRP5, and/or MRP6;
- BCRP, MRP1, MRP2, MRP3, MRP5, and/or MRP6;
- BCRP, MRP1, MRP2, MRP3, MRP4, and/or MRP6;
- BCRP, MRP1, MRP2, MRP3, and/or MRP6;
- BCRP, MRP2, MRP3, MRP5, and/or MRP6;
- BCRP, MRP2, MRP3, MRP4, and/or MRP6;
- BCRP, MRP2, MRP3, and/or MRP6.

In a further embodiment it is also possible that the test compound is capable of modulating ATP release from cells or from intracellular structures or organelles, such as mitochondria,

In a preferred embodiment, the test system contains only the indicated ABC Transporter, i.e. only the ABC transporter whose ATP transport might be influenced by being modulated by the test compound. In other words, in this preferred embodiment for instance only BCRP, only MRP1, only MRP2, only MRP3, only MRP4, only MRP5, or only MRP6 are present. It is also possible to use a test system including a combination of the aforementioned ABC transporters as the only ABC transporters in the test system.

It is however also possible that further structures/cellular elements that are capable of transporting ATP across the compartment(s), such as endogenous transport proteins, are present. In this case, a person skilled in the art takes suitable and known measures to the effect that the eventually determined influenced ATP transport (or at least most of it) can be attributed to the studied ABC transporter only, and/or that the further structures/cellular elements capable of transporting ATP are no longer in a position to transport ATP. This can for instance be achieved by inhibiting said further structures during testing, e.g. if the endogenous transport protein P-gp is present, an inhibitor of P-gp, PSC833 (valspodar), is added.

Within the meaning of the present invention, the term "test compound" denotes any compound that is subjected to the test according to the present invention. Preferred test compounds are disclosed below.

The term "about" in the context of the present invention denotes an interval of accuracy that the person skilled in the art will understand to still ensure the technical effect of the feature in question. The term typically indicates a deviation from the indicated numerical value of $\pm 10\%$, and preferably $\pm 5\%$.

Where the term "comprising" is used in the present description and claims, it does not exclude other elements or steps. For the purposes of the present invention, the term "consisting of" is considered to be a preferred embodiment of the term "comprising". If hereinafter a group is defined to comprise at least a certain number of embodiments, this is also to be understood to disclose a group which optionally consists only of these embodiments.

Where an indefinite or definite article is used when referring to a singular noun e.g. "a" or "an", "the", this includes a plural of that noun unless something else is specifically stated.

The term "compartment" defines a section within a test system. A section can for instance be separated from another section by a spatial separation, e.g. by a membrane, a cell layer, a cell wall, or by a device that is used for cultivation purposes. If for instance the test system comprises vesicles, the compartments are separated from each other by a membrane, i.e. the vesicle membrane, defining the intravesicular compartment and the extravesicular compartment. If the test system comprises cells, then the cell walls define an extracellular compartment and an intracellular compartment. Further, if cells are present in the form of a monolayer, the cells can define an apical extracellular compartment, a basolateral extracellular compartment, and an intracellular compartment.

In each compartment there can be different conditions, e.g. with regard to the medium that is present, or with regard to the pH value.

In the meaning of the present invention, the expression "being capable of modulating ATP release from cells by inhibiting or stimulating the ABC Transporter-mediated transport of ATP" denotes that the test compound interferes with the ABC Transporter-mediated transport of ATP, e.g. by competitive inhibition or allosteric modulation, and thereby influences the function of the ABC Transporter to the effect that the ABC Transporter-mediated transport of ATP is reduced or increased

"Adenosine triphosphate-Binding Cassette Transporter (ABC Transporter)" are members of a protein superfamily that is one of the largest and most ancient families with representatives in all extant phyla from prokaryotes to humans. ABC transporters are transmembrane proteins that utilize the energy of adenosine triphosphate (ATP) hydrolysis to carry out certain biological processes including translocation of various substrates across membranes. They transport a wide variety of substrates across extra- and intracellular membranes, including metabolic products, lipids and sterols, and drugs. Proteins are classified as ABC transporters based on the sequence and organization of their ATP-binding cassette (ABC) domain(s).

2. Method according to item 1, wherein the ATP added in step b) is labelled ATP.

3. Method according to item 2, wherein the labelled ATP is radiolabelled, preferably [$^{32}$P] $\gamma$-ATP or [$^{3}$H]ATP, preferably [$^{32}$P] $\gamma$-ATP.

By using labelled, preferably radiolabelled, such as [$^{3}$H]ATP or [$^{32}$P] $\gamma$-ATP, more preferably by using [$^{32}$P] $\gamma$-ATP it can be made sure that the detected ATP has been transported via the ABC Transporter, however not by processes or mechanisms different therefrom, such as lytic ATP release.

4. Method according to any of the preceding items, wherein the pH value of at least the compartment the test compound is added is about 7 or lower during step b) at the time point at which the test compound is added.

5. Method according to any of the preceding items, wherein the pH value is about 6.8 or less, preferably about 6.5 or less, or in a range of from about 7 to about 4 or about 6.8 to about 4, or in a range of from about 6.5 to about 4, preferably in a range of from about 6.5 to about 5, at the time point at which the test compound is added.

6. Method according to any of the preceding items, wherein the test compound is added to the compartment(s) that is (are) in direct contact with the ABC Transporter.

The compartment(s) that is (are) in direct contact with the ABC Transporter can be the extracellular, intracellular, intravesicular and/or extravesicular compartment(s). In a preferred embodiment, for instance if the compartments are constituted by vesicles such as membrane vesicles to the effect that an intravesicular compartment and an extravesicular compartment are presents, the test compound is added to the extravesicular compartment only. If the compartments are constituted by a cell, or a cell layer such as a cell monolayer, to the effect that an extracellular compartment and an intracellular compartment are present, the test compound is added to the extracellular compartment only.

7. Method according to any of the preceding items, wherein the reference compound in step d) is a compound which is known to inhibit or stimulate the ABC Transporter-mediated transport of ATP to the effect that ATP transport is reduced or increased.

An example of a compound which is known to inhibit the ABC Transporter-mediated ATP release, which in turn results in a reduction of ATP release from cells, is methotrexate (MTX), or a proton pump inhibitor of the "prazole class", for example pantoprazole.

8. Method according to any of the preceding items, wherein the method is carried out under hypoxia, wherein preferably said hypoxia is characterized by an oxygen content in a range of from 0 to 7% $O_2$, preferably of from 0 to 5% $O_2$, or preferably of from 0.5% to 1% $O_2$.

9. Method according to any of the preceding items, wherein the test compound in step b) is contacted with the at least one of the compartments of the test system in a concentration of from about 0.1 nM to about 10 mM, preferably in a concentration of from about 0.1 nM to about 1 mM, more preferably in a concentration of from about 0.1 nM to about 500 $\mu$M, even more preferably in a concentration of from 0.1 nM to about 100 $\mu$M.

10. The method according to any of the preceding items, wherein in step (e) it is concluded that the test compound is capable of modulating ATP release from cells by inhibiting or stimulating the ABC Transporter BCRP of item 1, if

I) at higher concentration(s) of the test compound, the determined amount(s) of ATP
is increased or reduced in the at least one compartment to which the ATP is transported, or
is increased or reduced in the at least one compartment from which the ATP is transported
as compared with the determined amount of ATP at lower concentration(s) of the test compound in the corresponding compartment of the test system;
II) in the presence of the test compound, the determined amount(s) of ATP
is increased or reduced in the at least one compartment to which the ATP is transported, or
is increased or reduced in the at least one compartment from which the ATP is transported
as compared with the determined amount of ATP in the absence of the test compound in the corresponding compartment of the test system; and/or
III) in the presence of a reference compound, the determined amount(s) of ATP is increased or reduced in the at least one compartment to which the ATP is transported, or
is increased or reduced in the at least one compartment from which the ATP is transported
as compared with the determined amount of ATP in the absence of the reference compound in the corresponding compartment of the test system.

11. Method according to any of the preceding items, wherein the test compound is classified as inhibitor of ATP release, if the determined amount(s) of ATP is reduced in the at least one compartment to which the ATP is transported, wherein preferably the reduction is 25% or more, more preferably 30% or more, and/or if the determined amount(s) of ATP is increased in the at least one compartment from which the ATP is transported, wherein preferably the increase is 25% or more, preferably 30% or more, and
wherein the test compound is classified as stimulator of ATP release, if the determined amount(s) of ATP is increased in the at least one compartment to which the ATP is transported, wherein preferably the increase is 25% or more, more preferably 30% or more, and/or if the determined amount(s) of the ATP is reduced in the at least one compartment from which the ATP is transported, wherein preferably the reduction is 25% or more, more preferably 30% or more.
When determining the percentage of stimulation, it is preferred to use the method as defined in item 1 (d)(B).

12. Method according to any of the preceding items, wherein the test system of step a) stably expresses, preferably overexpresses, the ABC transporter(s), and/or wherein step b) is carried out by adding the test compound to at least one of said compartments.

13. Method according to any of the preceding items, wherein the concentration of ATP added is in a range of from about 0.1 nM to about 8 mM, preferably in a range of from about 0.1 nM to about 1 μM and from about 100 μM to about 8 mM.

For instance, if it is desired to test the effect on low dose ATP, the ATP added is in a range of about 0.1 nM to about 1 μM, and if it is desired to test the effect on high dose ATP, the ATP added is in a range of from about 100 μM to about 8 mM.

14. Method according to any of the preceding items, wherein the time period of step (b) ranges in a suitable time period that is known to a person skilled in the art, and which depends on the system and cell type used, preferably the time period ranges from 0.1 minutes to 72 h or even longer, from 0.1 minutes to 48 h or 24 h. If a test system e.g. comprising membrane vesicles or monolayer of cells is used, the time period of step b) ranges from 0.1 minutes to 4 hours, preferably from 0.1 minute to 2 hours.

15. Method according to any of the preceding items, wherein determining the amount of labelled ATP in step (d) is performed at one or more time points after addition of labelled ATP, at which time point(s) a measurable transport of ATP has occurred.

16. Method according to any of the preceding items, wherein the test system comprises:

- membrane vesicles, preferably with an inside-out orientation, comprising, preferably stably expressing, more preferably stably overexpressing, said ABC Transporter, such as membrane vesicles prepared from insect cells such as Sf9 insect cells, or kidney cells, such as HEK293 or MDCKII cells, preferably wherein determining the amount of labelled ATP in step (c) is performed by isolating said membrane vesicles and determining the amount of labelled ATP taken up into and contained in said isolated membrane vesicles;
- polarized monolayer of endothelial cells comprising, preferably stably expressing, more preferably stably overexpressing, said ABC Transporter, such as MDCKII cells, or LLC-PK1 cells or a co-culture of porcine brain endothelial cells and primary rat astrocytes (e.g. CTX-TNA2 cell line); or
- Cells, preferably expressing, more preferably overexpressing, said ABC transporter, comprising, such as side population (SP) stem cells, preferably the SP of hematopoietic stem cells (HSCs) or a side population of a human glioblastoma cell line such as U87-MG; or human or animal (such as bovine, porcine, or rodent) brain capillary endothelial cells (BCECs), or glial cells such as astrocytes, or immune cells such as dendritic cells, natural killer cells, T cells, macrophags, platelets or erythrocytes,
  preferably wherein determining the amount of labelled ATP in step (c) is performed by taking an aliquot of at least one compartment to which labelled ATP is transported to and/or from which labelled ATP is transported, and determining the amount of labelled ATP in said aliquot.

By using the membrane vesicle system, the vesicles can be constructed (designed) and prepared in such a way that only the ABC Transporter whose inhibition or stimulation is desired to be determined. In other words, further structures that may probably influence the test results can be excluded.

By using a system comprising the monolayer of cells, or cells, the in vivo situation can be mimicked in a more realistic way, as the use of cells (or monolayers thereof) is comparably close to in vivo situation.

17. Method according to any of the preceding items, wherein the substance to be tested represents a herbal compound derived from a plant or from any suitable part(s) of a plant, preferably the herbal compound represents an extract, preferably dried extract, of a plant or of any suitable part(s) of a plant.
It is also possible that the herbal compound is not an extract of any suitable part(s) of the plant, but that the herbal compound is derived (obtained) in a different way from any suitable part(s) of the plant, i.e. not by extraction.
Within the meaning of the present invention, "suitable part(s) of the plant" are any parts that can be extracted in order to obtain herbal compound, or any parts that can otherwise be used for obtaining a herbal compound. Such parts can for instance be root, fruit, leaf, fructus, and the like.

18. Method according to any of the preceding items, where the compound to be tested is selected from the group of Traditional Herbal Medicinal Products, such as Traditional Chinese Medicine and Ayurvedic Medicine.
In a further embodiment, the compound to be tested is selected from the group of Traditional Chinese Medicine plants listed in "Chinese Herbal Medicine. Materia Medica, 3rd edition, Bensky, Claver, Stöger, Eastland Press" on pages 1189 - 1217 (Herb and Formula Index), p. 1219-1242 (Pinyin-Pharmaceutical cross reference, p. 1243 - 1251 English-Pharmaceutical Cross Reference, p. 1253-1257 Japanes-Pharmaceutical Cross Reference, p. 1259-1263 Korean Cross Reference, p. 1265-1279 Botanical, Zoological, and Mineral Index.

19. Method according to any of the preceding items, wherein the compound to be tested is derived from a plant or part(s) of the plant selected from the group consisting of Salvia miltiorrhiza Bge. (Chinese name Danshen); Isatis indigotica Fort. (also named Isatis tinctoria; Chinese name Daqingye for dried leaf of Isatis indigotica Fort. and Banlangen for dried root of Isatis indigotica Fort.); a species from the Simaroubaceae family, such as Brucea javanica (L.); and a species from Leonurus, such as Leonurus heterophyllus Sweet or Leonurus japonicas or Leonurus cardiaca (also named Herba Leonuri or Chinese motherworth in English; Chinese name YiMuCao).

20. Method according to any of items 17 to 19, wherein the herbal compound is obtained from an (aqueous) alcohol extract, preferably 70%-100% methanol or ethanol.

21. Method according to any of items 17 to 20, wherein the herbal compound is a hydrophilic phenolic acid or alkaloid compound, or a lipophilic diterpene or triterpene compound.

22. Method according to any of items 17 to 21, wherein the herbal compound is selected from the group consisting of salvianolic acids, such as hydrophilic salvianolic acids A, B, C, D, E; preferably salvianolic acids such as salvianolic acid A or B;
rosmarinic acid or derivatives;
lithospermic acids, preferably hydrophilic lithospermic acids, more preferably magnesium lithospermate such as magnesium lithospermate B;
protocatechuic acids, preferably hydrophilic protocatechuic acids, preferably protocatechualdehyde;
tanshinones, preferably diterpene tanshiones, preferably lipophilic diterpene tanshinones such as cryptotanshinone, tanshinone I, tanshinone IIB, dihydrotanshinone;
alkaloids, preferably hydrophilic alkaloids, such as the indole alkaloids indirubin and indirubin derivatives, or leonurine or stachydrine; and
triterpenes, preferably lipophilic triterpenes, more preferably quassinoids;
preferably, the herbal compound is selected from the group consisting of salvianolic acid B, magnesium lithospermate B, and indirubin.

In a further embodiment, the herbal compounds are selected from the group of compounds as defined in Figure 10 (any of A) to F), or all).

23. Method according to any of the preceding items, wherein the modulated ATP release is a non-lytic ATP release.

A lytic ATP release is for instance an ATP release accomplished via exocytosis, being accompanied by a disruption of the cell membrane. This lytic ATP release is however different from the ATP release which is referred to in the meaning of the present invention: Herein, the ATP release is mediated via the ABC Transporter.

A person skilled in the art knows how the components of the test system that is used in the present invention (e.g. the vesicles, or the cells or cell layers) have to be treated in order to avoid a lytic ATP release.

24. Combination of compounds selected from the group consisting of

a)

(i) one or more anti cancer drugs, preferably selected from the group consisting of temozolomide, bevacizumab, sunitinib, and sorafenib, and
(ii) a prazole selected from the group consisting of omeprazole, lansoprazole, dexlansoprazole, rabeprazole, esomeprazole, tenatoprazole, preferably esomeprazole or pantoprazole; and, optionally,
(iii) compound(s) being capable of modulating adenosine triphosphate (ATP) release from cells by inhibiting or stimulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property; more preferably one or more herbal compound(s) selected from the group of the herbal compounds defined in item 22, even more preferably salvianolic acid B, Mg lithospermate B, or indirubin;

b)

(i) one or more anti cancer drugs, preferably selected from the group consisting of temozolomide, bevacizumab, sunitinib, and sorafenib, and
(ii) compound(s) being capable of modulating adenosine triphosphate (ATP) release from cells by inhibiting or stimulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property; more preferably one or more herbal compound(s) selected from the group of the herbal compounds defined in item 22, even more preferably salvianolic acid B, Mg lithospermate B, or indirubin;

c)

(i) one or more anti cancer drugs, preferably selected from the group consisting of temozolomide, bevacizumab, sunitinib, and sorafenib;
(ii) ATP; and, optionally,
(iii) compound(s) being capable of modulating adenosine triphosphate (ATP) release from cells by inhibiting or stimulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property; more preferably one or more herbal compound(s) selected from the group of the herbal compounds defined in item 22, even more preferably salvianolic acid B, Mg lithospermate B, or indirubin;

d)

(i) methotrexate and/or imatinib, and
(ii) compound(s) being capable of modulating adenosine triphosphate (ATP) release from cells by inhibiting or stimulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property; more preferably one or more herbal compound(s) selected from the group of the herbal compounds defined in item 22, even more preferably salvianolic acid B, Mg lithospermate B, or indirubin; and, optionally,
(iii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole;

e)

(i) ATP, and
(ii) compound(s) being capable of modulating adenosine triphosphate (ATP) release from cells by inhibiting or stimulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property; more preferably one or more herbal compound(s) selected from the group of the herbal compounds defined in item 22, even more preferably salvianolic acid B, Mg lithospermate B, or indirubin; or
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole;

f)

(i) dendritic cell vaccines, and
(ii) compound(s) being capable of modulating adenosine triphosphate (ATP) release from cells by inhibiting or stimulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property; more preferably one or more herbal compound(s) selected from the group of the herbal compounds defined in item 22, even more preferably salvianolic acid B, Mg lithospermate B, or indirubin; and

g)

(i) dendritic cell vaccines,
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole;

h)

(i) dendritic cell vaccines,
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole, and
(iii) compound(s) being capable of modulating adenosine triphosphate (ATP) release from cells by inhibiting or stimulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property; more preferably one or more herbal compound(s) selected from the group of the herbal compounds defined in item 22, even more preferably salvianolic acid B, Mg lithospermate B, or indirubin;

for use in the treatment of cancer occurring in tissue where the respective ABC Transporter Modulator whose transport of ATP is inhibited or stimulated (preferably inhibited) by said compound(s), is expressed, preferably is overexpressed compared to the respective healthy, non-pathologic tissue,
preferably for use in the treatment of brain tumors and/or brain metastases,
provided that the compounds of (i), (ii), and where present, (iii), are different, respectively, in all subitems above.

The respective tissues expressing ABC Transporter are described elsewhere herein.
The compound(s) being capable of modulating adenosine triphosphate (ATP) release from cells are capable of modulating ABC Transporter modulators, wherein said ABC Modulator Transporter is one or more of:

- BCRP, MRP2, MRP3, MRP4, MRP5, and/or MRP6;
- BCRP, MRP1, MRP2, MRP3, MRP5, and/or MRP6;
- BCRP, MRP1, MRP2, MRP3, MRP4, and/or MRP6;
- BCRP, MRP1, MRP2, MRP3, and/or MRP6;
- BCRP, MRP2, MRP3, MRP5, and/or MRP6;
- BCRP, MRP2, MRP3, MRP4, and/or MRP6;
- BCRP, MRP2, MRP3, and/or MRP6.

25. Combination of compounds selected from the group consisting of

aa)

(i) temozolomide; and
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole;

bb)

(i) temozolomide;
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole; and
(iii) a herbal compound selected from the group as defined in item 22, preferably salvianolic acid B or Mg lithospermate B or indirubin;

cc)

(i) temozolomide; and
(ii) a herbal compound selected from the group as defined in item 22, preferably salvianolic acid B or Mg lithospermate B or indirubin;

dd)

(i) temozolomide; and
(ii) ATP;

ee)

(i) temozolomide;
(ii) ATP; and
(iii) a herbal compound selected from the group as defined in item 22, preferably salvianolic acid B or Mg lithospermate B or indirubin;

ff)

(i) methotrexate; and
(ii) a herbal compound selected from the group as defined in item 22, preferably salvianolic acid B or Mg lithospermate B or indirubin;

gg)

(i) methotrexate;
(ii) a herbal compound selected from the group as defined in item 22, preferably salvianolic acid B or Mg lithospermate B or indirubin; and
(iii) a prazole selected from the group consisting of omeprazole, lansoprazole, dexlansoprazole, rabeprazole, esomeprazole, and tenatoprazole;

hh)

(i) ATP;
(ii) a herbal compound selected from the group as defined in item 22, preferably salvianolic acid B or Mg lithospermate B or indirubin;

ii)

(i) ATP; and
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole;

jj)

(i) dendritic cell vaccines; and
(ii) a herbal compound selected from the group as defined in item 22, preferably salvianolic acid B or Mg lithospermate B or indirubin; and

kk)

(i) dendritic cell vaccines, and
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole;

ll)

(i) dendritic cell vaccines,
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole, and
(iii) compound(s) being capable of modulating adenosine triphosphate (ATP) release from cells or from intracellular structures or organelles, such as mitochondria, by inhibiting or stimulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property; more preferably one or more herbal compound(s) selected from the group of the herbal compounds defined in item 22, even more preferably salvianolic acid B, Mg lithospermate B, or indirubin; and

mm)

(i) herbal compound selected from the group as defined in item 22, or from the group as defined in Fig. 10, and
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole,

for use in the treatment of cancer occurring in tissue where the respective ABC Transporter Modulator whose transport of ATP is inhibited or stimulated (preferably inhibited) by said compound(s), is expressed, preferably is overexpressed compared to the respective healthy, non-pathologic tissue,
preferably for use in the treatment of brain tumors and/or brain metastases,
provided that the compounds of (i), (ii), and where present, (iii), are different, respectively, in all subitems above.

The respective tissues expressing ABC Transporter are described elsewhere herein.

26. Combination for use in the treatment of gliomas, preferably high grade glioblastoma multiforme, and/or brain metastases according to items 24 and 25. In a further embodiment, the combination for use in the treatment of gliomas, preferably high grade glioblastoma multiforme, and/or brain metastases, only contains one or more, preferably one, herbal compound, preferably the herbal compound is selected from the group as defined in item 22 and/or in Figure 10, as drug.

Within the meaning of the present invention, the term "drug" refers to an active ingredient which is intentionally included in a combination or composition, and which is effective in treating the respectively indicated pathological state or disease., respectively.

27. Combination for use in the treatment of advanced pancreatic ductal adenocarcinoma or high grade hepatocellular carcinoma according to any of item 24 e), f), or g); and/or according to any of item 25 hh), ii), jj), kk), ll), or mm).

28. Combination for use in the treatment of high grade hepatocellular carcinoma according to any of item 24 a) to c), or according to any of item 25 aa) to ee), wherein (i) respectively is sorafenib.

29. Combination of compounds selected from the group consisting of

A)

(i) antimalarial agent capable of modulating adenosine triphosphate (ATP) release from cells by inhibiting the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably artemisinin or a derivative thereof, and
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole,
esomeprazole, and tenatoprazole;, and, optionally,
(iii) ATP;

B)

(i) antimalarial agent capable of modulating adenosine triphosphate (ATP) release from cells by inhibiting the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably artemisinin or a derivative thereof, and
(ii) compound(s) being capable of modulating adenosine triphosphate (ATP) release from cells by inhibitin the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property; more preferably one or more herbal compound(s) selected from the group of herbal compounds defined in item 22, even more preferably a compound selected from the group consisting of salvianolic acid B, Mg lithospermate B, and a compound from the group of tanshinones; and,
optionally,
(iii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole,
esomeprazole, and tenatoprazole; and, optionally,
(iv) ATP;

C)

(i) antimalarial agent being capable of modulating adenosine triphosphate (ATP) release from cells by inhibiting the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably artemisinin or a derivative thereof, and
(ii) indirubin or derivatives thereof, e.g. indole alkaloids, indirubin-3-oxime, and, optionally,
(iii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole,
esomeprazole, and tenatoprazole; and, optionally,
(iii) ATP;

D)

(i) antimalarial agent being capable of modulating adenosine triphosphate (ATP) release from cells by inhibiting the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably artemisinin or a derivative thereof, and, and
(ii) ATP;

E)

(i) dendritic cell vaccines and
(ii) compound(s) being capable of modulating adenosine triphosphate (ATP) release from cells by inhibiting or stimulating the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property, more preferably one or more herbal compound(s) selected from the group of herbal compounds defined in item 22, even more preferably the compound is selected from the group consisting of salvianolic acid B, Mg lithospermate B, indirubin, and indirubin-derivatives; and

F)

(i) dendritic cell vaccines, and
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole;

G)

(i) dendritic cell vaccines,
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole, and
(iii) compound(s) being capable of modulating adenosine triphosphate (ATP) release from cells by inhibiting or stimulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property; more preferably one or more herbal compound(s) selected from the group of the herbal compounds defined in item 22, even more preferably the compound is selected from the group consisting of salvianolic acid B, Mg lithospermate B, indirubin, and indirubin-derivatives;

for use in the treatment of malaria,
provided that the compounds of (i), (ii), and where present, (iii), are different, respectively, in all subitems above.

Within the meaning of the present invention, the term "tanshinones" refers to all known tanshinones, preferably to the group of all known tanshinones however not including cryptotanshinone, more preferably to the group comprising tanshinone I, IIA, and B.

30. Combination of compounds selected from the group consisting of

AA)

(i) Artemisinin, and
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole;

BB)

(i) Artemisinin, and
(ii) a compound selected from the group consisting of salvianolic acid B, Mg lithospermate B, and a compound from the group of tanshinones;

CC)

(i) Artemisinin and a compound selected from the group consisting of salvianolic acid B, Mg lithospermate B, and a compound from the group of tanshinones, and
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole;

DD)

(i) artemisinin, and
(ii) indirubin or derivatives thereof;

EE)

(i) artemisinin,
(ii) indirubin or derivatives thereof, and
(iii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole;

FF)

(i) artemisinin; and

(ii) ATP;

GG)

(i) dendritic cell vaccines, and
(ii) a herbal compound selected from the group as defined in item 22, preferably salvianolic acid B or Mg lithospermate B, indirubin or indirubin-derivatives;

HH)

(i) dendritic cell vaccines; and
(ii) vacuolar ATPase (v-ATPase) inhibitor, preferably proton pump inhibitor, preferably selected from the group of prazoles, more preferably selected from the group consisting of omeprazole, lansoprazole, dexlansoprazole, rabeprazole, esomeprazole, and tenatoprazole;

II)

(i) dendritic cell vaccines,
(ii) proton pump inhibitors, and
(iii) compound(s) being capable of modulating adenosine triphosphate (ATP) release from cells by inhibiting or stimulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property; more preferably one or more herbal compound(s) selected from the group of the herbal compounds defined in item 22, even more preferably the compound is selected from the group consisting of salvianolic acid B, Mg lithospermate B, indirubin, and indirubin-derivatives;

for use in the treatment of malaria,
provided that the compounds of (i), (ii), and where present, (iii), are different, respectively, in all subitems above.

Within the meaning of the present invention, derivatives of indirubin are for instance indole alkaloids and indirubin-3-oxime.

In a further embodiment, the combination for use in the treatment of malaria only contains one or more, preferably one, herbal compound, preferably the herbal compound is selected from the group as defined in item 22 and/or in Figure 10, as drug.

31. Combination of compounds for use in the treatment of malaria according to item 30, wherein in AA), BB), CC), DD), EE), and HH) additionally ATP is present.

In a further preferred embodiment, the herbal compounds referred to throughout the items are compounds obtained from Danshen (Salvia miltiorrhiza), preferably the compounds obtained from Danshen are the compounds as depicted in Figure 10 A to E.

32. Combination of compounds for use in the treatment of malaria according to any of items 29 to 31, wherein the artemisinin derivatives are artemether, artesunat, arteflene, artemotil, dihydroartemisinin und arteether.
33. Compound capable of activating ATP release, preferably capable of stimulating ATP release as defined in any of items 1 to 23, for use in stimulating the immune system. In a further embodiment, the invention refers to the compound identified as activator of low-dose or high-dose ATP release by using the method as defined in any of items 1 to 23, for use in stimulating the immune system.
34. Compound capable of modulating, preferably of low-dose or high-dose, ATP release, preferably capable of modulating ATP release as defined in any of item 1 to 23, for use in inhibiting hyperinflammation.
35. Compound capable of modulating, preferably of low-dose or high-dose, ATP release, preferably capable of modulating ATP release as defined in any of item 1 to 23, for use in therapeutic vaccination.
36. Compound capable of inhibiting, preferably of low-dose or high-dose, ATP release, preferably capable of inhibiting ATP release as defined in any of item 1 to 23, for use in treating inflammatory diseases, such as rheumatoid arthritis, asthma or chronic obstructive pulmonary disease, atherosclerosis and ischemia, endotoxemia and sepsis, graft-versus-host-disease, neurodegenerative disorders, including Alzheimer disease, depression, preferably rheumatoid arthritis.

37. Compound identified as modulator, preferably of low-dose or high-dose, ATP release by using the method as defined in any of items 1 to 23, for use in DC maturation.

The compounds, as well as combinations of compounds, according to the present invention represent or can be comprised in a pharmaceutical composition and can be formulated as pharmaceutical dosage forms. A person skilled in the art knows which excipients (such as diluents, filler, binder, glidants, buffer, and/or coatings, and the like) are suitable and can be used for preparing the respective formulation. Suitable dosage forms can for instance be tablets, powders, granules, pills, lozenges, sachets, capsules, solutions (e.g. injection or infusion solutions), and the like.
The pharmaceutical composition can be used in a method of treating the diseases listed herein.

38. Pharmaceutical composition comprising

(a) an antigen,
(b) a compound being capable of modulating adenosine triphosphate (ATP) release from cells by stimulating the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably one or more herbal compound(s) possessing said property,

wherein said composition is suitable for eliciting an immune response directed against the antigen (a), preferable for use in vaccination.

The compound(s) being capable of modulating adenosine triphosphate (ATP) release from cells by stimulating the Adenosine triphosphate-Binding Cassette Transporter (ABC Transporter) can for instance be able to stimulate ABC Transporter that can be selected from:

- BCRP, MRP2, MRP3, MRP4, MRP5, and/or MRP6;
- BCRP, MRP1, MRP2, MRP3, MRP5, and/or MRP6;
- BCRP, MRP1, MRP2, MRP3, MRP4, and/or MRP6;
- BCRP, MRP1, MRP2, MRP3, and/or MRP6;
- BCRP, MRP2, MRP3, MRP5, and/or MRP6;
- BCRP, MRP2, MRP3, MRP4, and/or MRP6;
- BCRP, MRP2, MRP3, and/or MRP6.

An antigen can be any suitable antigen that is known by a person skilled in the art and against which vaccination is desired. Just to illustratively mention an example, an antigen useful in influenza vaccination may be influenza-virus-specific haemagglutinin and/or neuraminidase. It can also be that more than one antigen is present. The pharmaceutical composition may further comprise compounds that are commonly present in pharmacological compositions such as vaccination formulations, as known to a person skilled in the art, and can for instance comprise an adjuvant, such as AIOH, an adjuvant comprising squalene and polysorbate 80 (e.g. MF59® or AS03®), CpG oligonucleotide, or adjuvant comprising cholesterol and phospholipids (such as ISCOMATRIX®).

39. Pharmaceutical composition according to item 37, wherein compound (b) has been identified by using the method according to any of items 1 to 23, preferably compound (b) that has been subjected to the method according to any of items 1 to 23 is a herbal compound, such as a herbal compound as defined elsewhere herein., e.g. as defined in citem 22.

40. Combination according to any of items 24 to 32, wherein the compound being capable of modulating adenosine triphosphate (ATP) release from cells by inhibiting or stimulating the Adenosine triphosphate-Binding Cassette Transporter (ABC Transporter) has been identified by using the method according to any of items 1 to 23.

41. Pharmaceutical composition comprising

(i) an antiviral drug, selected from the group consisting of antiviral drugs for the treatment of highly pathogenic RNA virus infections, including for influenza, M2 channel inhibitors, amantadine and rimantadine, and the neuraminidase inhibitors, oseltamivir and zanamivir, and
(ii) a compound being capable of modulating adenosine triphosphate (ATP) release from cells by modulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably a herbal compound possessing said property, wherein said compound is suitable for inhibiting virus replication by inhibiting ATP release by ABC Transporter from the cytosol into the extracellular milieu, thereby limiting the ATP being available for endosomal uptake and replication of the virus.

The compound(s) being capable of modulating adenosine triphosphate (ATP) release from cells by modulating, preferably inhibiting the Adenosine triphosphate-Binding Cassette Transporter (ABC Transporter) can for instance be able to inhibit ABC Transporter that can be selected from:

- BCRP, MRP2, MRP3, MRP4, MRP5, and/or MRP6;
- BCRP, MRP1, MRP2, MRP3, MRP5, and/or MRP6;
- BCRP, MRP1, MRP2, MRP3, MRP4, and/or MRP6;
- BCRP, MRP1, MRP2, MRP3, and/or MRP6;
- BCRP, MRP2, MRP3, MRP5, and/or MRP6;
- BCRP, MRP2, MRP3, MRP4, and/or MRP6;
- BCRP, MRP2, MRP3, and/or MRP6.

42. Pharmaceutical composition comprising

(i) an antiviral drug, selected from the group consisting of antiviral drugs for the treatment of highly pathogenic RNA virus infections, including for influenza, M2 channel inhibitors, amantadine and rimantadine, and the neuraminidase inhibitors, oseltamivir and zanamivir.
(ii) a proton pump inhibitor, and optionally
(iii) a compound being capable of modulating adenosine triphosphate (ATP) release from cells by modulating, preferably inhibiting, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, preferably a herbal compound possessing said property, wherein said compound is suitable for inhibiting virus replication by inhibiting ATP release by ABC Transporter from the cytosol into the extracellular milieu, thereby limiting the ATP being available for endosomal uptake and replication of the virus.

The compound(s) being capable of modulating adenosine triphosphate (ATP) release from cells by modulating, preferably inhibiting the Adenosine triphosphate-Binding Cassette Transporter (ABC Transporter) can for instance be able to inhibit ABC Transporter that can be selected from:

- BCRP, MRP2, MRP3, MRP4, MRP5, and/or MRP6;
- BCRP, MRP1, MRP2, MRP3, MRP5, and/or MRP6;
- BCRP, MRP1, MRP2, MRP3, MRP4, and/or MRP6;
- BCRP, MRP1, MRP2, MRP3, and/or MRP6;
- BCRP, MRP2, MRP3, MRP5, and/or MRP6;
- BCRP, MRP2, MRP3, MRP4, and/or MRP6;
- BCRP, MRP2, MRP3, and/or MRP6.

Within the meaning of the present invention, the compounds listed in (i), (ii) and, where applicable, (iii), that are capable of modulating adenosine triphosphate (ATP) release from cells or from intracellular structures or organelles, such as mitochondria, by stimulating the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP, are used in combination for the treatment of the respective indicated disease (pathological state). A combined use herein means that the specific compounds are used together, e.g. they can be used (administered) in one treatment step substantially at the same time, or timely separated. If administered together, they can be formulated in a common administration form, or formulated in separate but simultaneously applied administration forms. If the compounds are used timely separated, then the time lapse between the administration preferably is chosen such that each component of the combination still exerts its function as modulating ATP release from cells or from intracellular structures or organelles, such as mitochondria, by inhibiting or stimulating ABC transporter.

Further, the prazole being preferred among the list of prazoles is pantoprazole; however, pantoprazole is not used if a composition or combination comprises as active ingredients only methotrexate and a prazole.

Detailed description of the invention

As explained throughout the present description, ATP release from cells plays an important role in pathophysiological processes.

Within the meaning of the present invention it has now surprisingly been found that by inhibiting or stimulating the ABC Transporter defined herein (in particular as defined in the items), by using compounds that are known to be inhibitors or stimulators of ABC Transporters, with the compounds preferably having been identified by the method according to the present invention, the ATP release from the cell is reduced or increased, respectively resulting in a reduced or increased amount of extracellular ATP. As described elsewhere herein, by modulating the release of ATP by inhibiting or stimulating the ABC Transporter defined herein by using a compound capable of inhibiting or stimulating ATP release from cells, certain diseases that are in connection with the extracellular ATP level can be treated. This is in particular the case in cells or tissues, respectively, where ABC Transporters are expressed, preferably overexpressed compared to normal, healthy, non-pathologic tissue.

Thus, in a first aspect, the present invention relates to the modulation of preferably non-lytic ATP release from cells by at least one type of ATP-Binding Cassette Transporter (ABC transporter) selected from the group consisting of ABCG2 (BCRP; Breast Cancer Resistance Protein) and ABCC1-6 (Multidrug Resistance-associated Protein 1-6) under conditions of cellular stress. More in particular, under conditions of low pH and/or hypoxia, as e.g. exists in the microenvironment of tumors. Thereby, the present invention relates to the concomitant modulation of ABC drug transporters (ABCG2 or ABCC1-6) and, preferably, the tumor microenvironment (low pH and/or hypoxia). As to the ABC Transporters that are modulated, these can be any one or more of:

- BCRP, MRP2, MRP3, MRP4, MRP5, and/or MRP6;
- BCRP, MRP1, MRP2, MRP3, MRP5, and/or MRP6;
- BCRP, MRP1, MRP2, MRP3, MRP4, and/or MRP6;
- BCRP, MRP1, MRP2, MRP3, and/or MRP6;
- BCRP, MRP2, MRP3, MRP5, and/or MRP6;
- BCRP, MRP2, MRP3, MRP4, and/or MRP6;
- BCRP, MRP2, MRP3, and/or MRP6.

Whenever it is referred to "ABC Transporter", or "BCRP", a person skilled in the art will understand that also the above (groups of) ABC Transporter is (are) referred to.

In particular, and in accordance with the inter alia above explained concept underlying the invention, the present invention relates to an *in vitro* method ("ABC drug transporter-mediated-ATP release assay") for:

1. The screening of immunomodulatory agents ("test compound", or "compound", respectively) for the treatment of diseases related to an increased level (compared to healthy, non-pathological tissue) of extracellular ATP, such as immune/inflammatory diseases, in particular for the treatment of cancer, more in particular for novel glioma therapies, more in particular for high-grade glioblastoma multiforme and brain metastases. Furthermore, for adenocarcinomas of the digestive tract (including the esophagus, stomach, liver, bile duct, gallbladder, pancreas, small intestine (=duodenal cancer), colon, rectum (=colorectal cancer) and anus, endometrium, and lung, and for melanoma. In particular, for pancreatic ductal adenocarcinoma. Furthermore, for hepatocellular carcinoma (HCC), in particular pathological poorly differentiated G3/G4 grade HCC tissue. Furthermore for the treatment of leukemia.
2. The screening of immunomodulatory agents, e.g. compounds which are capable of modulating adenosine triphosphate (ATP) release from cells by inhibiting or stimulating, the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATPfor treatment of infectious diseases, such as malaria and influenza.
3. The screening of additives (adjuvants) for improving the production of cell-based vaccines, in particular the ex vivo production of dendritic cells or natural killer cells for vaccine production, in particular for the treatment of glioma and malaria.

Recently, it was shown that the apically located ABC drug transporters BCRP (ABCG2) and MRP4 (ABCC4) transport endogenous compounds such as cAMP, cGMP, folic acid and urate as well as nucleobase and nucleoside analogues used in cancer chemotherapy and antiretroviral therapy (23-25). For BCRP (ABCG2) it was shown that substrate drugs could be more efficiently transported at low pH, and this was independent of the dissociation status of the drug (24). In brief, it was shown that the ATP-dependent transport of 1 $\mu$M MTX by BCRP was 7-fold higher at pH 5.1 than at physiological pH, and increased gradually with increasing [$H^+$] concentration. Also the main (toxic) metabolite, 7-OH-MTX was found to be transported more efficiently at low pH. MTX diglutamate was found to be transported by BCRP at low pH but not at physiological pH. Resveratrol, which is a neutral compound at pH $\leq$ 7.4, was efficiently transported by BCRP at pH 6.0 and no transport was observed at pH 7.4. Furthermore, the BCRP substrates mitoxantrone and topotecan were also transported more efficiently at low pH (24).

With the present invention, amongst others it has now been found that the nucleotide ATP is a substrate of e.g. the ABC drug transporter ABCG2 (BCRP). Of clinical importance is that it was found that BCRP transports ATP at physiological pH, but more efficiently at pH 6 (in terms of a pH gradient). This was demonstrated by studying the uptake of radiolabelled ATP, i.e. [$^3$H] radiolabelled ATP (Figure 1 and 2), but also [$^{32}$P]radiolabelled $\gamma$-ATP, into Sf9-BCRP membrane vesicles and wild-type vesicles as control at pH 7.4 and pH 6.0 (Figure 3). This is of importance, because one could argue that the observed transport is not ATP but that its breakdown products (adenosine diphosphate (ADP) or adenosine monophosphate (AMP)) are transported into the vesicles, as ATP is subject to degradation by ecto-ATPases, ecto-ADPases and ecto-nucleotidases at the cell surface in all extracellular spaces. Therefore, it was also demonstrated that BCRP transports [$^{32}$P]-$\gamma$ labeled ATP, as is shown in Figure 3.

In addition, it has now been shown by using Sf9-BCRP vesicles that the BCRP-mediated transport of [$^3$H]ATP at low pH can be inhibited by the BCRP substrate drugs pantoprazole and methotrexate (Fig. 4). However, 10 $\mu$M pantoprazole reduced the BCRP-mediated ATP release at low pH by 15% and high concentrations of pantoprazole (>1 mM) were required to decrease the transport of ATP by 50%. Also with MTX the transport was not completely inhibited yet, 100 $\mu$M, an intermediate-dose, inhibited the ATP transport by appr. 46%.

Furthermore, using the in vitro methods, we tested the effect of various herbal compounds from the group of salvianolic acids, lithospermic acids, tanshinones, indirubines, quassinoids and ginkgolides on the BCRP-mediated transport of ATP at low pH, in the presence and in the absence of pantoprazole. In addition, we tested whether temozolomide is a substrate or inhibitor of BCRP, P-gp and/or MRP1-6 at physiological and/or low pH, and subsequently we tested the effect of temozolomide on the BCRP-mediated transport of ATP at low pH.

Under physiological conditions, intracellular ATP concentration in most cells is 5-10 mM, while extracellular concentrations have been reported to be in the low $\mu$M range. In blood, extracellular ATP concentrations of 0.2 - 2 $\mu$M at 2-4 s and appr. 20 $\mu$M at 3-5 min. after stimulus have been found. Furthermore, it has been shown that the ATP concentration in the tumour interstitium is in the hundreds micromolar range, and was basically undetectable in healthy tissues.

This is in line with the finding of the present invention, where it was demonstrate now for the first time that ATP is transported by BCRP, preferably at pathophysiological (pathogenic) conditions of low pH, and that the transport at physiological pH is minimal.

Recently, it has been found that ATP release (e.g from dying, injured and stressed cells) into the extracellular milieu is the first step in the cascade of events leading to NLRP3 inflammasome activation, and the recruitment and activation of DCs.

Furthermore, as was demonstrated by Trabanelli et al. 250 nM ATP stimulated proliferation, cytokine release, expression of adhesion molecules, and adhesion, 1 mM ATP induced apoptosis and inhibited CD4+ T cell functions (16). Thus, concomitant inhibition of BCRP and modulation of the tumor microenvironment (acidic stress) plays an important role in the induction of apoptosis and reduction of tumor growth.

So far, different assays have been described to study the role of ABC transporters or other pathways in ATP transport and release from cultures of epithelial and nonepithelial cells, i.e.:

1. a radiolabeled [$\gamma$-$^{32}$P]ATP release assay in which the transport and release of loaded radiolabeled ATP is measured and trapped as $^{32}$P-labeled glucose 6-phosphate with the help of the ATP scavenging enzyme, hexokinase. A key part of the design of this assay is the inclusion of hexokinase, an ATP scavenger. In contrast to other ATP scavengers such as the ATPase/ADPase apyrase, hexokinase traps released [$\gamma$-$^{32}$P]ATP as glucose-6-[$^{32}$P]phosphate before the labeled ATP is subject to degradation by the cultured cell themselves (26).
2. A nonradioactive bioassay measuring released ATP as luminescence from the luciferase-luciferin reaction. Each ATP that is released creates a photon of light that is collected by a luminometer. The ATP dependence of firefly luciferase is used to measure the remaining ATP concentration where the luminescence signal is inversely proportional to kinase activity (27).
3. A single-channel patch-clamp analysis of existed membrane patches of cells expressing CFTR in which $Cl^-$ conduction versus $ATP^-$ conduction can be measured (28).

Both the [$\gamma$-$^{32}$P]ATP release/trapping assay (method 1) and the ATP bioluminescence assay (method 2) are measuring the release of intact ATP, but these methods are measuring ATP in an indirect way, i.e. hexokinase, an ATP scavenging enzyme and luciferase, a catalyst, are respectively used in these assays to quantify ATP. Furthermore, it is not possible in the hexokinase assay to study paired analysis of the effect of an agonist on ATP release. Although the luciferase assay is widely used to measure ATP content in isolated mitochondria and in permeabilized whole cells, it is a sensitive assay, in which a number of variables need to be taken into account for reliable results, for example the concentration of luciferin in the reaction mixture has to be appropriate for the levels of ATP present in the cells. The single-channel patch clamp assay of existed membrane patches of cells expressing CFTR (method 3) assesses relative conduction of $Cl^-$ and $ATP^-$. When conductance is measured this indicates activity by CFTR itself, but may also be related to a closely associated channel that conducts the $ATP^-$ anions.

None of the above studies have studied the release of ATP by the drug transporter BCRP at low pH or showed the effect of a modulator on the release of ATP by a drug transporter at low pH.

The present invention provides a novel method to identify immunomodulators by studying the effect of a drug or compound (at different doses) on the release of ATP, preferably [$^{32}$P]γ-ATP, but also [$^{3}$H]ATP or non-labelled ATP, by one type of ABC drug transporter selected from the group of ABC transporters consisting of BCRP (ABCG2) and MRP1-6 (ABCC1-6) under conditions of low pH and/or hypoxia, either by using membrane vesicles or by using a polarized monolayer of cells For further details, see paragraph "*In vitro* methods for identifying modulators of the release of ATP from cells by at least one type of ABC transporter selected from the group consisting of BCRP (ABCG2) and MRP1-6 (ABCC1-6)". Most *in vitro* assays measuring tumor cell function are routinely performed at neutral-to-alkaline pH. This may lead to significant inaccuracy in understanding tumor behaviour *in vivo,* because the tumor microenvironment exhibits a low pH and each pH unit shift represents a 10-fold change in H+ concentration. It has been shown that slight changes in pH can have profound effects on increased transporter activity resulting in increased ATP release from cells. Thus, it is important to study the modulatory effect of compounds under (patho)physiologically relevant conditions. As the current *in vitro* methods take either the tumor microenvironment parameter of low pH or the tumor microenvironment parameter hypoxia into account, the present *in vitro* methods are useful in the translation from preclinical to clinical concept, enabling the preclinical selection of immunomodulators with high(er) potential to succeed in clinical studies.

This is in particular of interest for the treatment of glioma tumors and glioma stem cells for novel glioma therapies, based on both the role of extracellular ATP in triggering the cascade of events leading to NLRP3 inflammasome activation and recruitment and activation of dendritic cells, and the high expression of BCRP in glioma tumors and glioma stem cells, and the low pH and hypoxia existing in the glioma tumor environment.

In the following, *in vitro* methods for identifying modulators of the release of ATP from cells by at least one type of ABC transporter are described.

<u>*In vitro* methods for identifying modulators of the release of ATP from cells by at least one type of ABC transporter selected from the group consisting of BCRP (ABCG2) and MRP1-6 (ABCC1-6)</u>

*Membrane vesicles*

Vesicles of isolated plasma membranes are well suited for the characterization of functional properties of transport systems. Today, membrane vesicles prepared from the sinusoidal (basolateral) and bile canalicular (apical) membrane in the liver and from the brush border (apical) and basolateral membrane in the kidney are readily available for the study of renal and hepatobiliary transport. By using membrane vesicles for drug transport studies, the drug transport across the basolateral and apical membrane can be measured separately, and intracellular binding and/or metabolism can be ignored. ABC drug transporters can be overexpressed in the plasma membrane of cells via transfection (using retrovirus or baculovirus) or drug selection methods. For most applications, the transporter is expressed in insect cell lines, e.g. Sf9 insect cells, taking advantage of the robust baculovirus insect cell system. Isolated membrane vesicles can also be prepared from human cell lines selected for drug resistance overexpressing the transporter, such as HEK293 and MDCKII cells. The advantage of using Sf9 insect cells is that high levels of expression of ABC transporters can be obtained (29). Thus, for the expression of a specific ABC drug transporter in isolated membrane vesicles various cells can be used, preferably Sf9 insect cells, but also HEK293, MDCKII cells.

The membrane vesicles (stably overexpressing one type of ABC transporter selected from the group consisting of BCRP (ABCG2) and MRP1-6 (ABCC1-6)) can be obtained from Solvo Biotechnology or can be prepared as described in literature (29,30). The vesicular buffer and the solutions of the reagents have to be adjusted to the appropriate pH (range pH 5 to pH 7.4) as described in Breedveld et al., Mol Pharmacol 2007 (24).

When an ABC drug transporter, which has its active site oriented towards the cytoplasm, such as MRP1-6 (ABCC1-6) or BCRP (ABCG2), is overexpressed in the plasma membrane, this may result in the ATP-dependent uptake of substrates into inside-out oriented membrane vesicle preparations (30). Subsequently, uptake of substrate can be quantified after filtration over a microporous filter (see Fig. 5). The use of radiolabeled drugs and compounds is required to easily study the uptake into membrane vesicles, and has the advantage that the ABC transporter-mediated uptake of any compound of interest can be measured directly by radiochemical analysis. By testing varying concentrations of the "test compound" the pharmacokinetic parameters, i.e. Km and Vmax, can be obtained. Studying drug uptake into membrane vesicles is best suitable for drugs or compounds, which are hydrophilic, as most lipophilic compounds will easily stick to the membrane.

By using radiolabeled ATP, i.e. preferably [$^{32}$P]radiolabelled γ-ATP, but also [$^{3}$H]ATP, itself as substrate drug, this *in vitro* membrane vesicle assay can now be used to test the capacity of a drug or a compound (at different doses) to directly inhibit or stimulate the transport of ATP (at different doses) by an ABC transporter under conditions of low pH, preferably pH 5 - 6.5, but lower than 7.0 and compared to physiological pH, i.e. 7.3 (which ranges from 7.1 to 7.4). For testing the effect of a compound on ABC transporter-mediated transport of low-dose ATP preferably a concentration of 0.1 - 1 μM radiolabelled ATP is used. For testing the effect of a compound on ABC transporter-mediated transport of high-dose ATP preferably a concentration of 50 μM - 5 mM ATP is used.

*Transwell assay*

While a variety of cells can be used for this assay, the cells must (a) overexpress the drug transporter of interest, and (b) form a functionally polarized cell monolayer, complete with tight extracellular junctions. In this regard, porcine kidney epithelial cells (LLC-PK1) or Madin-Darby canine kidney II epithelial cells (MDCKII) (double-)transfected with various drug transporters have been helpful in demonstrating drug transporter activity. The relatively short culture time required for these cells make the LLC-PK1 and MDCKII cells excellent tools for identifying drug transporter activity, as human ABC transporters can be stably overexpressed in these cell lines by transfection methods. However, endogenous drug transporters expressed in the parental and transfected cell lines may also contribute to the drug transport in these cells. For example, endogenous MDR1 P-gp is present in the MDCKII parental cell lines, and its expression is even higher in MDCKII cells transfected with MRP2. Inhibiting endogenous drug transporters in these cells may be essential for the correct assessment of the role of the studied drug transport protein in drug transport. For example, for studying BCRP-mediated transport, endogenous P-gp needs to be inhibited with valspodar, which can be obtained from a commercial supplier. For studying MRP-mediated drug transport, endogenous P-gp and BCRP need to be inhibited with elacridar, which can be obtained from a commercial supplier.

Drug transport across a monolayer can be studied by using a transwell system (Fig. 6). The (radiolabeled) drug or compound of interest is applied to either the apical or basolateral side of a confluent monolayer of polarized epithelial or endothelial cells and the resulting flux of the compound across the confluent cell monolayer can be measured either by radiochemical analysis or by HPLC analysis. As shown in Fig. 7, apical drug efflux transporters, such as P-gp, MRP2 or BCRP, pump their substrate drug out of the cell, resulting in an increased transport from the basolateral to apical side (BA) compared with the transport from the apical to basolateral side (AB), i.e. active transport. When the compound is not a substrate of the drug efflux transporter, the transport from the basolateral to apical side (BA) is equal to the transport from the apical to basolateral side (AB), i.e. no active transport (Fig. 7). The opposite is observed when the transport of substrate drugs of basolateral drug efflux transporters, such as MRP1 (ABCC1) or MRP3 (ABCC3), is studied: active transport by basolateral drug efflux transporters results in an increased transport from the apical to basolateral side (AB) compared with the transport from the basolateral to apical side (BA) (not shown). Thus, drug efflux activity can be identified by the appearance of bi-directional differences in drug permeability that can be influenced by inhibitors of the drug efflux transporter in question. Studying drug efflux across a monolayer is best suitable for drugs or compounds, which are lipophilic, as most hydrophilic compounds will not easily cross the membrane and reach the drug efflux transporter.

By adjusting the pH of the media in the transwell assay to low pH (pH 5-7, preferably pH 6 - 6.5, but lower than 7.0) as described in Breedveld et al., Mol Pharmacol 2007 (24), the extracellular tumor pH can be simulated. By using radiolabelled ATP, preferably [3$^2$P]ATP, as a substrate, the effect of a test compound on the ABC transporter-mediated ATP release from cells can be measured over time. For testing the effect of a compound on ABC transporter-mediated transport of low-dose ATP preferably a concentration of 0.1 - 1 μM radiolabelled ATP is used. For testing the effect of a compound on ABC transporter-mediated transport of high-dose ATP preferably a concentration of 50 μM - 5 mM ATP is used.

In addition, in a separate experiment, the effect of a test compound on ABC transporter-mediated efflux of intracellular ATP (already present in the cells) at low (extracellular) pH can be determined by both by quantification of the intracellular ATP content and by quantification of the concentration ATP in the apical and basolateral compartment over time. The concentration ATP in these compartments can be measured by HPLC analysis as described by Manfredi et al. (27). Furthermore, these experiments can be done under hypoxic conditions (0.5% - 1 % $O_2$, 5% $CO_2$) by exposing the MDCKII cells to hypoxic conditions for 48 h before the start of the experiment in a hypoxia-athmosphere-controlled incubator, keeping cells unmanipulated for 48h, thereby avoiding $O_2$ pressure changes.

Overall, this assay is an ideal *in vitro* model to study the effect of test compounds on the ABC-transporter mediated ATP release from cells under tumor-like conditions, i.e. low extracellular pH and/or hypoxic cells.

To treat gliomas, it is important that a test compound passes the blood brain barrier (BBB). The BBB consists of specialized brain endothelial cells which, along with astrocytes, neurons and pericytes form the neurovascular unit. The specialised microvascular endothelial cells that form the blood brain barrier are connected by tight junctions serving as physical barriers to restrict paracellular passage. Thereto, it has been shown by Cantrill et al. that a transwell model with a co-culture of porcine brain endothelial cells (PBEC) and primary rat astrocytes (CTX-TNA2 cell line) could be used to study delivery processes at the BBB (31). Furthermore, they showed that P-gp and BCRP activity was present in these cells. Furthermore, it has been shown that ATP is released by astrocytes, mediating the activation of microglia (20)

Thus, when using a co-culture of porcine brain endothelial cells and primary rat astrocytes (CTX-TNA2 cell line) instead of MDCKII cells, our assay is an ideal in vitro model to study the effect of test compounds on the ABC-transporter mediated ATP release from cells under glioma tumor-like conditions, i.e. low extracellular pH and/or hypoxic cells.

Furthermore, studying in addition the effect of test compounds on the ABC-transporter mediated ATP release from glioma cell lines, such as the human U87-MG, or primary pediatric high-grade glioma (pHGG), or diffuse intrinsic pontine glioma (DIPG), or the rat C6 glioma cell line, under conditions of low pH and/or hypoxia will be highly relevant for the further screening of potential immunomodulators for the treatment of glioma.

Summed up, the "ABC drug transporter-mediated-ATP release assays" can be used to test the capacity of a drug or a compound to inhibit or stimulate the transport of ATP, e.g. radiolabelled [$^{32}$P] $\gamma$-ATP, [$^{3}$H]ATP and intracellular ATP, by ABC transporters selected from the group consisting of BCRP (ABCG2) and MRP1-6 (ABCC1-6), preferably under conditions of low pH (pH 5 - 7.0) compared to physiological pH (pH 7.1 - 7.4) and/or under hypoxic conditions (0-1% $O_2$, 5% $CO_2$, 94-95% $N_2$). In case of the ABC drug transporter BCRP (ABCG2), this shows whether a drug or compound may be an effective modulator of BCRP-mediated release of ATP, and thus may be an effective immunomodulator for the development of novel therapies for e.g. inflammatory and infectious diseases, in particular for the treatment of (high-grade) glioblastoma multiforme.

ABC drug transporters: General overview

The ATP-binding cassette (ABC) superfamily of transporters consists of a large number of functionally diverse transmembrane proteins which have been subdivided into seven families designated A through G. These transporters facilitate the unidirectional translocation of chemically diverse substrates over endothelial membranes, including amino acids, lipids, inorganic ions, peptides, saccharides, metals, xenobiotics and proteins. This extrusion from the cell occurs in an active, ATP-dependent manner, and can take place against a steep concentration gradient. These transporters are present in almost all tissues and cell types. To date, sixteen members have been identified to be associated with human disease. However, only three major ABC drug transporters, including P-glycoprotein (P-gp, MDR1, ABCB1), multidrug resistance protein 1 (MRP1; ABCC1) and BCRP (ABCG2; MXR), are thus far believed to seriously affect cancer chemotherapy (32).

The 170 kDa P-glycoprotein was the first of the ABC drug transporters discovered and is the most widely studied ABC transporter. Expression of P-gp in tumor cells converted these cells into multidrug resistant cells. The finding that the clinically applied calcium channel blocker verapamil inhibited drug efflux and restored drug sensitivity in multidrug resistance leukaemia cell lines gave rise to the idea that compounds that inhibit P-gp could reverse P-gp-mediated MDR in patients, and led to the development of P-gp inhibitors. Despite the development of various P-gp (and BCRP) inhibitors, resistance to anticancer agents is nowadays still a problem in cancer treatment.

All members of the ABC transporter family use ATP to translocate substrates and MRP1-2 and MRP4-5 also require physiological concentrations of glutathione (GSH) for their normal transport activity.

Expression of ABCG2 (BCRP)

*BCRP* was first cloned in 1998 based on its overexpression in a highly doxorubicin-resistant MCF7 breast cancer cell line. Later research revealed that the first cloned *BCRP* cDNA represented a mutant BCRP, deviating from the wild-type BCRP at arginine 482. Human *BCRP* encodes a 655 amino acid ABC protein, containing a single N-terminal ATP binding cassette, followed by 6 putative transmembrane segments (TMSs), and therefore often referred to as "half-transporter". *BCRP* belongs to the *ABCG* gene family. Based on analogy with other ABC drug efflux transporters analyzed to date, it seems very likely that the half-transporter BCRP functions as a homodimer.

ABCG2 (BCRP) is mainly expressed in epithelial cells in the body, where it localizes to the apical membrane. As a consequence, BCRP substrates are transported from the basolateral to the apical side of the epithelium. This extrusion from the cell occurs in an active, ATP-dependent manner, and can take place against a steep concentration gradient. Thereby, BCRP has an important barrier function.

*Expression of ABCG2 (BCRP) in normal tissues*

ABCG2 (BCRP) is expressed in a large variety of normal human tissues. In particular, prominent and consistent BCRP expression has been observed in the liver canalicular membrane, in the epithelium of the gastrointestinal tract (small intestine and colon), in kidney proximal tubules, in placental syncytiotrophoblasts, and in ducts and lobules of the breast. Also high expression of the *ABCG2* gene at the apical membrane of alveolar epithelial cells was found in lactating, but not in virgin or nonlactating mammary glands of humans. Furthermore, BCRP is present in venous and capillary endothelial cells, including brain endothelium (i.e., the blood-brain-barrier), but not in arterial endothelium (33,34). BCRP is also expressed in a wide variety of stem cells including muscle, neural, testicular stem cells and those of hematopoietic origin and is the molecular determinant of the side population (SP) phenotype (35) (See Figure 7). Furthermore, BCRP is expressed in immune cells: epidermal Langerhans cells (LC) and dermal interstitial dendritic cells (IDC) were found to express the ABC drug transporter BCRP (ABCG2) (36). Also dendritic cells activated by PPARγ expressed high levels of functional ABCG2 protein (37).

*Expression of ABCG2 (BCRP) in tumor tissues*

Drug resistance is a common phenomenon in cancer treatment. One cause of resistance to chemotherapy is the expression of ABC transporters in tumor cells, which converts these cells into multidrug resistance cells, as multiple compounds of different chemical classes are substrate of ABC drug transporters, such as ABCG2 (BCRP), ABCB1 (P-gp) and ABCC1-6 (MRP1-6). The majority of data concerning the contribution of ABCG2 to drug resistance has been gathered for leukemia. In a study using the anti-ABCG2 monoclonal antibody BXP-21 with 150 specimens from untreated cancer patients with solid tumors, ABCG2 expression was seen in all 21 tumor types, with a relatively increased frequency in adenocarcinomas of the digestive tract, endometrium, and lung, and melanoma. In pancreatic ductal adenocarcinoma high BCRP expression was a significant prognostic factor for early tumor recurrence and poor survival. BCRP expression was also upregulated in hepatocellular carcinoma (HCC) and the expression was higher in pathological poorly differentiated G3/G4 grade than in G1/G2 HCC tissue (38). In some solid tumors, such as ovarian and breast cancer, ABCG2 expression frequently showed no correlation with clinical outcomes. Of relevance, ABCG2 expression in many other human solid tumors, such as lung and esophageal cancers, is associated with negative prognosis (39). Furthermore, ABCG2 showed strong expression in glial tumors. ABCG2 (BCRP) displayed higher expression levels in high-grade compared with low-grade tumors (34). More recently, ABCG2 expression has been detected in glioma stem cells, and the expression level of ABCG2 correlates well with the increasing pathological grade of glioma, i.e. positive immunostaining of ABCG2 was observed in less than 10% of low-grade gliomas (grade I + II) and in more than 40% of high-grade gliomas (grade III + IV), suggesting that ABCG2 may be a marker of glioma progression (40). The same phenomenon was observed in a recent study that investigated the expression of ABCG2 in esophageal squamous cancer cells: ABCG2 expression was significantly increased with the increasing pathological grade and TNM stage. Furthermore, ABCG2 expression was significantly higher in the lymphatic metastasis group than in the non-metastatic group (39).

*Expression of ABCG2 (BCRP) in stem cells*

Stem cells have the capacity for both self-renewal (i.e., proliferation without a change in phenotype) and differentiation (i.e., changing into a new phenotype). Some stem cells have already undergone considerable differentiation, so further differentiation is restricted to a single cell type or lineage. Other stem cells are multipotent and differentiate into a variety of cell types (i.e., hematopoietic stem cells).

Side population (SP) cells are a population of primitive bone marrow-derived stem cells with long-term repopulating capacities. The SP phenotype can be determined by efflux of the fluorescent dye Hoechst 33342. The ABC transporter BCRP (ABCG2) is mainly responsible for the SP phenotype (i.e. the transport of the Hoechst dye), at least in bone marrow. Besides BCRP, P-gp is also able to efflux the Hoechst dye, but to a lesser extent than BCRP does. None of the MRPs present in SP cells (i.e. MRP1, 3 and 4) were able to efflux the Hoechst dye (35). Whereas bone marrow cells from *Mdr1al1b* knockout mice contained a normal number of SP cells, a significant reduction of SP cells in bone marrow and skeletal muscle was observed in *Bcrp1*-/- mice, suggesting *Bcrp1* as molecular phenotype of SP (35). SP cells are highly enriched for hematopoietic stem cell (HSC) activity. Approximately 70% of the classic stem cell population KLS (ckit/Sca-1/ Lin), known for its repopulation capacities, also has the SP phenotype. Zhou et al. discovered that ABCG2 (BCRP) null hematopoietic cells were significantly more sensitive to the BCRP substrate mitoxantrone. This result suggests that the physiological function of ABCG2 expression in HSCs is to provide protection from cytotoxic substrates. Scharenberg et al. showed that BCRP is expressed at relatively high levels in putative hematopoietic stem cells (isolated as SP, 341/382 or 341/KDR1 populations) and drops sharply in committed progenitors (341/381, 341/331, or 341/101). Expression remains low in most maturing populations, but rises again in natural killer cells and erythroblasts.

In recent years, it has become clear that the SP population not only resides within the bone marrow but also in other non-hematopoietic organs, such as spleen tissue, umbilical cord blood, brain, kidney, heart, intestine, skin, and lungs. In these organs, they appear to have the ability to differentiate into many cell types following reintroduction in vivo, including skeletal and cardiac muscle, neurons, and epithelial cells like hepatocytes, but also in hematopoietic cells in lethally irradiated mice. This multipotent differentiation capacity of HSCs suggests that these cells contribute significantly to tissue remodelling. A loss of expression of BCRP leads to cell differentiation, indicating that BCRP might determine stem cell-induced tissue remodelling through differential expression. During hematopoietic differentiation, ABCG2 level is initially down-regulated, but the protein is expressed again (at a low level) in some mature cell types, *e.g.* of the erythroid lineage (35). The same phenomenon also occurs in human neural, retinal and embryonic stem cells. When ABCG2 is overexpressed in retinal progenitors by using a retrovirus-mediated transduction, differentiation is blocked. Si-RNA-mediated silencing of ABCG2 expression in retinal progenitors depletes the SP population and promotes differentiation.

*Expression of BCRP in cancer stem cells*

It is generally believed that conventional chemotherapy does not kill all tumor cells, and a small subpopulation of cells, which are resistant to the chemotherapy and have the capability of initiating tumors, might be the source of recurrence and metastasis. These cells are referred to as cancer stem cells (CSCs), which have been found in a variety of hematopoietic and solid tumor types including leukemia, breast cancer, prostate cancer, glioma, medulloblastoma, pancreatic cancer, gastric cancer, hepatocarcinoma, colorectal and ovarian carcinoma. According to the CSC model, minimal residual disease and tumour recurrence after treatment would result from a remaining, therapy-resistant CSC fraction, whereas metastatic potential would be a CSC-specific property.

CSCs might share many properties of normal stem cells, such as self-renewal and unlimited proliferative potential. Like normal tissues, cancer cell lines and primary tumor cells contain an SP. The existence of SP cells has been proven to play an important role in tumor growth and relapse in many solid tumors. For example, SP from pancreatic cancer cell line had high capacity of the epithelial to mesenchymal transmission, invasion and metastasis. Therefore it is desired to isolate and to characterize CSCs for effective therapeutic strategies of targeting these cells. As to the methods of detecting CSCs, identification of specific markers for CSCs is the preferred one. The tumor initiating and chemotherapy-resistant capability of CSCs may be attributed to the expressions of several ATP-binding cassette (ABC) transporters, such as ABCG2 and MDR1. The ABC transporter BCRP (ABCG2) is mainly responsible for the SP phenotype.

In any case, in several experimental systems, malignant SP cells have been shown to be endowed with higher clonogenic and tumorigenic potential than non-SP cells. For example, in a study in NOD/SCID mice the SP population of cancer stem-like cells from SK-MG-1, a human glioma cell line, generated tumors in the brains of these mice at 8 weeks after implantation, whereas the non-SP cells did not generate any tumors in the brain. Another example, SP cells sorted from HCC cell lines HCCLM3, MHCC97-H, MHCC97-L and Hep3B harboured cancer stem cells, were related to metastatic potential and therapeutic resistance. Due to these features, SP cells can be responsible for the aggressive behavior of certain tumors as well as for the development of drug resistance; thus, strategies targeting this subset may have therapeutic implications.

*Expression of BCRP in immune cells*

Antigen-presenting cells like dendritic cells are important regulators of immune responses by presenting their captured antigens to specific T cells. In general, the maturation status of DCs is a key determinant of how the immune response will evolve. Of relevance for this patent application, epidermal Langerhans cells (LC) and dermal interstitial dendritic cells (IDC) were found to express the ABC drug transporter BCRP (ABCG2). Also low BCRP expression was present on CD34+ blood DC precursors and expression was increased upon their differentiation to LC (36). The nuclear hormone receptor peroxisome proliferator-activated receptor gamma (PPARγ) directly and transcriptionally induces ABCG2 expression in a cell type of the human myeloid lineage, monocyte-derived dendritic cells. Dendritic cells activated by PPARγ express high levels of functional ABCG2 protein, and gain an enhanced capacity to extrude xenobiotics. These features may have important consequences to the survival and drug resistance of human dendritic cells during immune regulation and also have therapeutic ramifications (37). Recently, it has been shown by Kooij et al. that P-gp, which is also expressed on a variety of immune cells like monocytes, DCs, T and B cells and is involved in the efflux of inflammatory molecules such as steroids, prostaglandins and cytokines, plays a role in the immune response by controlling DC maturation and subsequent DC-induced T cell responses. The authors showed that P-gp knockout mice (tvtdr1a/1b-/-) displayed reduced clinical signs of experimental autoimmune encephalomyelitis (EAE) compared to wild type animals. Observed differences were associated with decreased brain inflammation, CNS demyelination and an overall reduced T cell response in tvtdr1a/1b-/- animals during disease. They postulated that P-gp mediates DC maturation by influencing the excretion of proinflammatory cytokines like TNF-α and IFN-γ as lower levels of these cytokines were detected during DC-T cell interactions. However, a controversial issue remains whether P-gp itself is capable of transporting cytokines or that P-gp is involved of the secretion of other relevant physiological substrates like platelet activating factor that in turn may affect cytokine secretion like IFN-γ as a secondary effect (41).

With the present finding, it is now clear that the nucleotide ATP is the relevant physiological substrate which is released by the ABC drug transporter BCRP under pathophysiological conditions, such as low pH and hypoxia, which exists amongst others in the tumor microenvironment, in particular in metastatic cells (42).

*Expression of BCRP in the brain and central nervous system: blood-brain barrier, blood-cerebrospinal fluid barriers and brain parenchyma*

Thus far, the treatment of primary or metastatic brain tumors with chemotherapy is limited because of a low distribution of anticancer agents into brain tissue. An important reason for this low efficacy is the efficient protection of the brain against drugs involving two drug-permeability barriers (1) the blood-brain barrier (BBB) and the blood-cerebrospinal fluid (CSF) barrier. The BBB is primarily formed by the endothelium of the blood capillaries in the brain. The endothelial cells at their adjacent margins form tight junctions, preventing the diffusional entry of polar solutes via the paracellular pathway. The absence of fenestrations, vesicular traffic, and pinocytosis in brain capillary endothelia further restricts free flow between brain interstitium and blood. Diffusion of some hydrophobic drugs through the endothelial cell membrane is counteracted by transporters such as P-gp and BCRP, present in the apical (luminal) membranes of these cells. It has recently been shown that endogenous substrates for ABC transporters may include inflammatory mediators, such as prostaglandins, leukotrienes, cytokines, suggesting a potential role for ABC transporters in immunological responses, including inflammatory brain disorders. The blood-CSF barrier is formed by the epithelium of the choroid plexus, which also contains transport proteins, like MRP1, that act as a barrier for certain drugs entering from the blood.

Drug transport in the central nervous system is highly regulated not only by the blood-brain and the blood-cerebrospinal fluid barriers but also in brain parenchyma. Cellular membranes of parenchyma cells, such as microglia and astrocytes, act as a second "barrier" to drug permeability and express transporters whose properties appear similar to those localized at the conventional brain barriers. The brain parenchyma is made up of neurons and the surrounding neuroglia cells. The macroglia consist of astrocytes and oligodendrocytes, and proliferate throughout life, particularly in response to injury. Microglia are considered to be the resident immune cells of the brain. In the brain, astrocytes play active roles in cell-to-cell signaling by releasing gliotransmitters, such as glutamate and ATP, and thus forming neuron-glia and glia-glia networks. Astrocytic ATP release is involved in modulating a variety of neuronal activities, affecting excitability, synaptic transmission, cell death, growth and survival. Release of ATP from astrocytes is required for $Ca^{2+}$ wave propagation among astrocytes and for feedback modulation of synaptic functions. Extracellular ATP was shown to induce partial exocytosis of lysosomes, which contain abundant ATP. Regulated lysosomal exocytosis in astrocytes may contribute to intercellular signalling. Calcium wave propagation is the most prominent form of intercellular signalling among astrocytes, and this propagation is mediated mainly by ATP release. All types of glial cells express purinergic receptors. ATP released from astrocytes also modulates the activities of astrocytes and microglia through the stimulation of purinergic receptors; P2X7 receptors are widespread in astrocytes. ATP can activate P2X7 receptors in astrocytes to release glutamate, GABA, and also ATP which might regulate excitability of neurons in certain pathological conditions. It has been suggested that astrocytes can sense the severity of damage in the CNS by the amount of ATP released from damaged cells and that extracellular ATP concentration modulates the TNF-$\alpha$-mediated inflammatory response. Astrocytes possess a number of nutrient and drug transport proteins including several nucleoside transporters as well as P-gp and MRP1. Besides lytic ATP release, astrocytes have been shown to release ATP in a non-lytic manner in response to osmotic swelling, mechanical stimulation, deprivation of extracellular $Ca^{2+}$, and stimulation with glutamate, UTP, noradrenaline or NO. However, the precise pathway for non-lytic ATP release remains controversial (43). Swelling-induced ATP release from mouse cortical astrocytes was insensitive to blockers of Pgp and MRP1 transporters (glibenclamide, probenecid, verapamil) and the P2X7 receptor (brilliant blue G). In addition, it was found that blockers of connexin and pannexin hemichannels (1-octanol, carbenoxolone) and VSOR anion channels (phloretin, DCPIB) did not significantly affect swelling-induced ATP release from mouse astrocytes (43). Furthermore, also exocytosis did not seem to play a role in swelling-induced ATP release from astrocytes (43).

BCRP has been shown to be expressed in the BBB, i.e. the brain microvessel endothelium (33). The report by Aronica et al. (34) confirms the presence of BCRP protein in human brain tissue. BCRP is found to be consistently expressed in lysates of human temporal cortex and hippocampus and its expression is relatively high compared with other ABC transporters, such as P-gp. In glial tumor tissue from patients with refractory epilepsy a strong BCRP protein expression was found. BCRP displayed higher expression levels in high-grade tumors, compared with the low-grade. However, in control brain tissue, BCRP was exclusively expressed in blood vessels, and no glial or neuronal BCRP immunoreactivity was found. The strong BCRP expression in the microvasculature of the gliomas (astrocytoma, anaplastic astrocytoma, glioblastoma multiforme, anaplastic oligogendroglioma) and glioneuronal tumors could critically influence the transport and bioavailability of anticancer agents in the epileptogenic areas within the tumor (34). Furthermore, Bhatia et al. showed that BCRP is expressed in the nuclear extracts of glioblastoma multiforme and astrocytoma cells (44).

<u>Multidrug Resistance-Associated Proteins 1-6 (ABCC1-6; MRP1-6)</u>

The multi-drug resistance associated protein (MRP) family contains at least nine members (MRP1-9) with sizes from 1325 to 1545 amino acids. All MRP members have hydrophobic transmembrane domains and cytoplasmic nucleotide binding domains. MRPs can be categorized according to the presence or absence of a third (NH2-terminal) membrane-spanning domain in their structure. This topological feature can be found in MRP1, MRP2, MRP3, MRP6 and MRP7, while it is not possessed by MRP4, MRP5, MRP8, and MRP9. MRPs with this structural feature have the ability to transport conjugates, while MRPs without it are able to transport cyclic nucleotides.

*Expression in the Central Nervous System (CNS)*

As described above for the ABC drug transporter BCRP (ABCG2), the primary barriers to the CNS are the blood-brain-barrier (BBB) and the blood-cerebrospinal fluid barriers. The blood-CSF barrier is anatomically defined by the chorid plexus, located in the lateral, third, and fourth ventricles. The cells that comprise these barriers express multiple ATP-dependent, membrane-bound, efflux transporters, besides P-glycoprotein (P-gp; ABCB1) and BCRP (ABCG2) also the multidrug resistance-associated protein (MRP) family, which contribute to lowered drug accumulation. The prevention of accumulation of compounds in the CNS by the ABC transporters depends on the selective expression of the transporters on the apical versus basolateral surface of the cells. For example, the ABC transporter MRP1 localizes in the basolateral membrane of choroid plexus epithelial cells, whereas P-gp and BCRP are expressed at the apical membrane of endothelial cells of the brain capillaries forming the blood-brain-barrier. Mrp4 expression is in the basolateral membrane of the choroid plexus epithelium and in the apical membrane of the brain capillary endothelium, thereby limiting the movement of drugs or compounds from the blood into the brain or into the CSF, thereby protecting the brain from chemotherapy, as was shown for topotecan. Cellular components of the brain parenchyma could act as a second barrier to brain permeation of pharmacological agents via expression of many of the same transporters. In table 2 an overview is given of the protein and/or gene expression of MRP/Mrp isoforms in various brain compartments in human, in mouse and in rat (45).

*Expression of MRPs in glioma tumors*

Calatozollo et al. investigated the expression of P-gp, and the MRPs 1, 3 and 5 in malignant glioma patients. Cytofluorimetric analysis of 48 glioma specimens and 21 primary cultures showed high levels of MRP1, moderate levels of MRP5 and low levels of P-gp and MRP3. Immunohistochemistry of 25 glioma specimens showed expression of MRP1 (51.3%), MRP5 (45.8%), P-gp (34.8%) and MRP3 (29.9%) in tumor cells. Moreover, analysis of tumor samples by real time quantitative PCR showed mRNA expression of all investigated MRPs and P-gp. Tumor vasculature, analyzed in glioma specimens and in tumor derived endothelial cells, showed expression of all investigated proteins. Non-tumor brain samples and normal human astrocytes and endothelial cells also expressed MRP1 and MRP5. No significant differences in protein expression were detected between primary or recurrent gliomas, suggesting that glioma chemoresistance is mostly intrinsic (46).

Also Declèves et al. investigated the functional expression of MRPs in 2 distinct glioma cells (GL15 and 8MG) from patients with glioblastoma multiforme. MRP1 was detected at both mRNA and protein level in both cell lines, with a higher expression in the 8MG cells that occur predominantly at the cell membrane. MRP3,4 and 5 were detected by RT-PCR in both cell lines, whereas MRP2 was not expressed.In addition, MRP3 protein was aslo detected by immunohistochemistry in both cell lines. In contrast, P-gp expression was not detected in either cell line .

Furthermore, immunohistochemical staining on primary pediatric glioma tissues indicated the presence of P-gp, MRP1 and BCRP in the endothelial cells of the tumor vasculature, while only MRP1 was also expressed in glioma tumor cells. However, for BCRP possibly oligomers were formed in the pediatric glioma cultures (46).

<u>Tumor microenvironment</u>

In tumor drug resistance, also the tumor microenvironment of cells plays an important role. The tumor environment is characterized by regions of fluctuating and chronic hypoxia, low pH, and nutrient deprivation, which occurs as a consequence of inadequate blood flow, inflammation, enhanced glycolysis (the so-called Warburg effect), and the production of acidic metabolites (lactic acid production). In addition, excessive $CO_2$ release caused by altered glycolysis leads to increased tumor expression of carbonic anhydrase IX (CA-IX), thus contributing to further acidification of the extracellular tumor environment. Hypoxia also activates the hypoxia-inducible factor (HIF) pathway, which in turn upregulates glucose transporters and CA-IX and leads to additional exacerbation of tumor acidosis. As a result, the extracellular pH in tumor tissue can drop to values of 6.0 or less and is on average 0.2 to 1.0 units lower than that of normal tissues. The intracellular pH of mammalian cells is normally maintained within a narrow range, i.e. from 7.1 to 7.3, and is tightly regulated by specific ion exchangers (see Table 1) (3, 47). As a consequence, tumor cells are exposed to a pH gradient. This biochemical shift has been shown to provide a selective growth advantage to tumor cells at the expense of infiltrating host cells, including immune cells. It is becoming increasingly evident that the chronic destruction of cellular homeostasis occurring in cancer cells by metabolic alterations including glycolysis and intracellular alkalinisation associated with microenvironment acidification is a main factor driving tumor progression, invasion and metastases.

Furthermore, hypoxia arises early in the process of tumor development because rapidly proliferating tumor cells outgrow the capacity of the host vasculature. Tumors with low oxygenation have a poor prognosis, and strong evidence suggest this is because of the effects of hypoxia on malignant progression, angiogenesis, metastasis, and therapy resistance. The hypoxic microenvironment selects cells capable of surviving in the absence of normal oxygen availability (hypoxia tolerance).

*Tumor microenvironment and pH: the expression of proton pumps in tumor tissues*

Targeting and exploiting features of the tumor microenvironment to kill cancer cells provides a more integrated view of cancer therapy. Acidity of human solid tumors is involved in tumor progression and malignancy mostly by inducing a selection of cells adapted through an altered metabolism to survive in hostile conditions. Recent *in vivo* studies on animal models and also in patients with oral cancer have shown an acidic extracellular pH (pHe) and neutral-to-alkaline intracellular pH (pHi) in tumor lesions. The acidic tumor pHe drives proliferation, favors chemoresistance and promotes metastatic potential whereas maintenance of alkaline pHi sustains resistance to cytotoxicity.

The abnormal pH gradient characterizing tumor cells is finely tuned by different ion/proton pumps including the vacuolar ATPase (v-ATPase) whose expression and activity are enhanced in human tumors. Gene knock-down of the v-ATPase subunit c reduced growth of hepatocellular carcinoma and sensitized breast cancer cells to chemotherapeutics, and provided synergistic effects on tumor growth. Hence, pharmacological targeting of v-ATPase to regulate tumor pH or overcome chemoresistance has been recently proposed as a novel anticancer strategy (42).

Thus, for the maintenance of an appropriate relatively neutral intracellular pH, an acidic luminal pH, and an acidic extracellular pH, vacuolar $H^+$-ATPases play a critical role by actively pumping protons either through ion exchange mechanisms or by segregating $H^+$ within cytoplasmic organelles that are subsequently expelled (for an overview of ion exchange pumps, see Table 1). In a study by de Milito et al. the proton pump inhibitor (PPI) esomeprazole (and omeprazole) inhibited proliferation of melanoma cells *in vitro* and induced a cytotoxicity strongly boosted by low pH culture conditions. Esomeprazole induced cell death was significantly increased in cells cultured in medium at pH 5.0 while no cytotoxic effect was detectable at neutral pH. Interestingly, esomeprazole-induced cytotoxic effect in melanoma cells was accompanied by a time-dependent activation of several caspases. Their data indicated that blockade of proton pumps through esomeprazole induced caspase-dependent cell death in human melanoma cells. Esomeprazole administration (2.5 mg/kg) to SCID mice engrafted with human melanoma reduced tumor growth, consistent with decrease of proliferating cells and clear reduction of pH gradients in tumor tissue. Moreover, systemic esomeprazole administration dramatically increased survival of human melanoma-bearing animals, in absence of any relevant toxicity. These data show preclinical evidence supporting the use of PPI as novel therapeutic strategy for melanoma, providing the proof of concept that PPI target human melanoma modifying tumor pH gradients (42).

Furthermore, Calcinotto et al. showed that buffering of low pH at the tumor site by in vivo administration of the proton pump inhibitor (PPI) esomeprazole improved tumor-infiltrating T-lymphocytes (TIL) efficacy and delayed cancer progression in tumor-bearing mice (47). These data highlight tumor acidity as a mechanism of immune escape that could be targeted for rescuing effective tumor immunity and achieving disease control in patients with cancer. Some reports have shown that metastatic tumors are more acidic than primary tumors, but also that solid tumors, either carcinoma or melanomas or sarcomas, are more acidic than systemic tumors (i.e. leukemia). It appears therefore conceivable that proton pump inhibitors, such as esomeprazole, might be more active against very malignant tumors, which are often entirely unresponsive to current therapy (42). In addition to this it has also been shown that metastatic melanoma cells may be grown in acidic condition while cells deriving from primary tumors die when cultured in the same condition, needing longer periods of adaptation to select acid-resistant cells. In fact, acidic condition increases susceptibility of metastatic melanoma cells to proton pump inhibitors.

Of interest, it was recently shown in esophageal squamous cancer cells that - besides ABCG2 - also v-ATPase expression significantly increased with the increasing pathological grade and TNM stage. Furthermore, v-ATPase expression was also significantly higher in the lymphatic metastasis group than in the non-metastatic group (39).

*Tumor microenvironment, stem cell environment and hypoxia: the expression of ABCG2 (BCRP)*

The microenvironment of solid tumors is characterized by heterogeneity in oxygenation. Hypoxia arises early in the process of tumor development because rapidly proliferating tumor cells outgrow the capacity of the host vasculature. Tumors with low oxygenation (1-7% oxygen content) have a poor prognosis, and strong evidence suggest this is because of the effects of hypoxia on malignant progression, angiogenesis, metastasis, and therapy resistance. The hypoxic microenvironment selects cells capable of surviving in the absence of normal oxygen availability (hypoxia tolerance). Hypoxia tolerance arises through the increased ability to regulate ATP supply during hypoxic exposure. Hypoxic tumor cells are able to decrease their requirements for ATP. Tumors with a low oxygenation have a poor prognosis, and strong evidence suggests this is because of the effects of hypoxia on malignant progression, angiogenesis, metastasis and therapy resistance. Of importance, Krishnamurthy et al. reported that *Bcrp* expression is up-regulated by hypoxia in several human lineages (placental/choriocarcinoma (JAR) cells, osteosarcoma (Saos-2) cells, and myeloid leukemia cells (OCI-AML3 cell line) and in blast cells from patients with acute myeloid leukemia (48) (49). Hypoxia induced increased BCRP activity, as was shown by a 7-fold increase in the efflux of the dye Hoechst 33342 from cells under conditions of low oxygen (49). The increased activity correlated with the increased expression of BCRP mRNA in primary murine bone marrow cells and in human cell lines of diverse lineages (49).

Not only the tumor microenvironment, but also stem cells have a characteristic feature with their ability to thrive under conditions of low oxygen (hypoxia). Notably, hematopoietic stem cells are concentrated in hypoxic areas, and, under hypoxic conditions, myeloid cells have an improved ability to repopulate the bone marrow of lethally irradiated mice.

As shown by Krishnamurthy et al., ABCG2 enhanced the survival of hematopoietic stem cells in hypoxia through its interactions with heme. Specifically, ABCG2 binds heme and diminishes the cellular accumulation of porphyrins (49). The accumulation of heme within the cell can ultimately lead to the accumulation of iron and the production of cell-damaging reactive oxygen species by the Fenton reaction. Ahmed et al. demonstrated that constitutive expression of ABCG2 enhances the proliferative capacity of early human hematopoietic progenitors both in vitro and in vivo (50). Taken together, ABCG2 plays a role in protecting stem cells by increasing their survival capacity and proliferation potential, processes which are fundamental for stem cell maintenance and renewal.

Thus, the expression of ABCG2 (BCRP) plays a role in both tumor cell and stem cell survival strategy under hypoxic conditions.

*Concomitant modulation of tumor microenvironment and BCRP (ABCG2): inhibition of A TP release*

Extracellular ATP accumulates in the supernatant of dying tumor cells, while the intracellular concentration of ATP declines before and during the entry of cells into the step-wise process that leads to apoptosis and secondary necrosis. Vesicular exocytosis has been shown to mediate ATP release in various cell types responding to glutamate, hypotonic exposure, mechanical stress or hypoxia. Reportedly, tumor tissues contain much higher amounts of ATP in their interstitium (in the hundreds micromolar range) than healthy tissues do, where ATP would be basically undetectable. It has been shown by Martins et al. that chemotherapeutic agents induce ATP release from cancer cells. The intracellular concentration of ATP was reduced before and during the manifestation of apoptotic characteristics such as the dissipation of the mitochondrial transmembrane potential and the exposure of phosphatidylserine residues on the plasma membrane (4).

As described before, the extracellular (i.e. interstitial) pH of solid tumors is substantially more acidic than that of normal tissues, and the pH gradient between the cytoplasm and the intracellular organelles may also be involved in resistance to antitumor drugs. Luciani et al. showed that pretreatment of tumor cells with proton pump inhibitors inhibited the v-$H^+$-ATPase activity and increased both extracellular pH and the pH of lysosomal organelles. PPI pretreatment induced a marked increase in the cytoplasmic retention of the cytotoxic drugs, with clear targeting to the nucleus in the case of the anticancer agent doxorubicin. The authors also showed that the subcellular localization of v-$H^+$-ATPase changed upon treatment with the proton pump inhibitor omeprazole: v-$H^+$-ATPase-expressing-like structures accumulated in perinuclear regions of the omeprazole-treated melanoma cells, whereas in untreated cells v-$H^+$-ATPase staining appeared widely diffuse in the cytoplasm and beneath the cell membrane. This change in localization was accompanied by a change in levels of intracellular ATP. Cells treated with omeprazole or esomeprazole had higher levels of intracellular ATP than untreated cells, suggesting potent inhibition of v-$H^+$-ATPase activity. However, the current invention demonstrates that the proton pump inhibitor pantoprazole inhibited ATP release from cells by inhibition of BCRP (ABCG2) (51). Thus, resistance to antitumor drugs can be reduced by concomitant inhibition of v-$H^+$-ATPase and the ABC drug transporter BCRP leading to a reduced release of ATP from tumor cells, thus increased intracellular levels of ATP (51). This is in particular important for targeting brain metastases, which are even more acidic than tumor cells.

<u>Cancer types</u>

*Brain tumors and current treatment*

High-grade gliomas, in particular glioblastoma multiforme (GBM), are the most common primary brain tumors in adults and among the most deadliest of human cancers. Patients with a malignant glioma have poor prognosis owing to local recurrence, with a 5-year survival of only 9.8% despite surgery, radiotherapy and temozolomide chemotherapy. Gliomas are classified from grade 1 (more benign) to grade 4 (highly malignant; GBM) according to the WHO classification, which is based on histological features (e.g. mitostatic activity, microvascular endothelial proliferation). GBMs can be distinguished into primary GBMs, which rapidly arise without clinical evidence of a previously existing lower-grade lesion, and secondary GBMs, which develop through progression from low-grade astrocytomas or anaplastic astrocytomas.

GBM is characterized by diffuse infiltration into surrounding healthy brain tissue. The location of GBM and the extensive infiltration of tumor cells into surrounding pivotal brain structures are an impediment for all therapeutic interventions. The real challenge in improving the survival of GBM patients is to design therapies against numerous malignant cells that cannot be removed by surgical resection, as they have migrated (deeply) into the normal surrounding brain parenchyma. Radiation therapy shows too many neurotoxic side effects to enlarge the high-dose target volume to include these glioma cells. Furthermore, most chemotherapeutic agents cannot reach the invasive tumor cells because these reside in regions where the vasculature is largely intact with functional BBB properties.

Furthermore, high-grade gliomas in humans consist of a highly angiogenic region that harbors leaky blood vessels. However, there are also many invasive glioma cells that use the preexisting vasculature, which has an intact BBB. Capillaries in the tumor express the drug efflux transporter BCRP, often at increased levels, indicating that these tumor vessels may restrict drug uptake. Induction of angiogenesis is an essential prerequisite for tumor growth and progression. Once tumors reach a certain size (appr. 1 $mm^3$), simple diffusion of oxygen and nutrients is not sufficient to supply all tumor cells with necessary factors. This results in hypoxic areas, which trigger the formation of tumor vasculature, termed the angiogenic switch. This way the tumor established its own independent blood supply, which consequently facilitates its further expansion. GBMs are one of the most vascularized human cancers, and the formation of tumor-associated vessels occurs early during tumor progression. In fact, endothelial proliferation is one of the pathological criteria for grading a glioma as high-grade, indicative of poor prognosis. In addition to supplying tumor cells with oxygen and nutrients, endothelial cells are believed to form a niche-type entity in GBM. Endothelial cells closely interact with CSCs and maintain them in a stem-like state. Vice versa, GBM CSCs are able to recruit endothelial cells by secreting VEGF, stimulating the formation of their own niche. Moreover, GBM CSCs can differentiate into cells that very closely resemble tumor-associated vascular endothelial cells, suggesting that GBM CSCs can differentiate into niche-type cells. Therefore, endothelial cells have a major role in tumor growth. Tumor microvascular endothelial cells are capable of stimulating growth and stemness of GBM cells despite radiation and TMZ treatments, which is enabled by their extensive cell death resistance.

Recently, it was reported that the long term culture of glioma cells in medium at pH 6.5 induces a stem cell phenotype. Glioma stem cells (GSCs) are not uniformly distributed, but rather located in specialized niches, suggesting that the cancer stem cell phenotype is regulated by the tumor microenvironment. Hjelmeland et al. recently demonstrated that acidic conditions, independent of restricted oxygen, promote the expression of GSC markers, self-renewal and tumor growth (52). GSCs exert paracrine effects on tumor growth through elaboration of angiogenic factors, and low pH conditions augment this expression associated with induction of hypoxia inducible factor 2a (HIF2a), a GSC-specific regulator. Induction of HIF2a and other GSC markers by acidic stress can be reverted by elevating pH in vitro, suggesting that raising intratumoral pH may be beneficial for targeting the GSC phenotype. Therefore, anticancer therapies based on targeting both tumor pH regulation and the efflux transporter BCRP may be an effective approach for targeting GSCs, thereby reducing tumor growth and drug resistance.

Concomitant radiation therapy and temozolomide therapy after surgery is the standard treatment for glioblastoma multiforme. A potentially promising alternate approach for glioblastoma multiforme, brain metastases and primary central nervous system lymphoma is immune therapy (53). Active immunotherapy, which aims to stimulate or amplify the patient's own immune response, has shown promise. A common strategy is to provide antigen-presenting cells, usually dendritic cells, that have been made to express the target antigen (DC vaccine), but is still under development (53).

Furthermore, the tyrosine kinase inhibitor imatinib mesylate (Gleevec), which is used in the treatment of chronic myeloid leukemia and gastro-intestinal stromal tumors, has been shown to effectively inhibit PDGF-induced glioblastoma cell growth in preclinical studies. However, in clinical studies the effect is less pronounced and the results of Razis et al. (54) do not suggest a benefit from imatinib as a single agent in unselected glioblastoma multiforme patient populations, although imatinib was shown to be present in glioblastoma multiforme tissue and showed molecular effects in a subset of patients (54). In preclinical in vitro and in vivo studies it was shown that imatinib is a substrate drug of both P-gp and BCRP. Coadministration of BCRP and P-gp inhibitors, such as pantoprazole and elacridar, resulted in an increased brain penetration of imatinib (54).

Other treatments which are currently under investigation for recurrent and high-grade glioblastoma in adults and/or children are bevacizumab, and/or sunitinib and/or sorafenib, all three inhibitors of VEGF and/or PDGFR, kinases which are involved in angiogenesis and glioblastoma cell growth. However, these treatments have shown varying degrees of success, and encounter development of resistance. The brain penetration of both sunitinib and sorafenib has been shown to be restricted by P-gp (ABCB1) and BCRP (ABCG2). BCRP was the dominant transporter for sorafenib transport across the BBB. Brain accumulation of sunitinib was markedly (23-fold) increased in Abcb1a/b/Abcg2(-/-) mice, but only slightly (2.3-fold) in Abcb1a/b(-/-) mice, and not in Abcg2(-/-) mice. Importantly, a clinically realistic coadministration of oral elacridar and oral sunitinib to wild-type mice resulted in markedly increased sunitinib brain accumulation, equaling levels in Abcb1a/1b/Abcg2(-/-) mice.

Given the presence of BCRP in the blood brain barrier, in (high-grade) brain tumors, and in cancer stem cells, the development of more potent BCRP inhibitors that concomitantly inhibit the tumor microenvironment and the immune response mediated by the release of ATP by BCRP may be an important therapeutic application in the treatment of brain tumors, in particular high-grade gliomas and brain metastases, in cancer patients

*Pancreatic cancer, in particular pancreatic ductal adenocarcinoma*

Pancreatic cancer is a malignant neoplasm of the pancreas. Early detection is difficult, as patients seldom exhibit disease-specific symptoms at the early stages of pancreatic cancer, resulting in late diagnosis. The majority of pancreatic cancers are, upon diagnosis, advanced and unresectable. Pancreatic cancer has the lowest survival rate amongst all malignancies, with a 5-year survival rate of less than 5%. The current treatment for pancreatic cancer is very limited and ineffective. Gemcitabine and a combination of gemcitabine and erlotinib are the only FDA approved standard therapies for unresectable pancreatic cancer. Several gemcitabine-based combination therapies have been studied for the treatment of pancreatic cancer, none of which have shown any significant benefit over gemcitabine monotherapy.

| Cancer type | Anticancer agent(s) | ABC transporter(s) involved in drug resistance |
|---|---|---|
| Pancreas | Gemcitabine | MRP5 (and polymorphisms) P-gp, MRP1 |
| | Gemcitabine + erlotinib | P-gp, BCRP |

*Hepatocellular carcinoma (HCC) and current treatment*

Hepatocellular carcinoma (HCC) is the fifth most common type of cancer worldwide. HCC constitutes 85-90% of primary liver cancers and it is the third leading cause of cancer-related mortality. The overall survival rate remains less than 10%. Viral hepatitis caused by hepatitis B or C infections is the main cause of HCC. The standard treatment for HCC patients without cirrhosis is partial hepatectomy, but this is possible for only less than 20% of the patients, and several minimally invasive ablation methods have been developed. There are only limited therapeutic options, and resistance to conventional chemotherapy and radiotherapy is a major problem.

Until recently, there was no effective systemic chemotherapy available to treat this aggressive disease. Recently, sorafenib, a multikinase inhibitor (amongst others VEGFRs and PDGFR-$\beta$), has recently a breakthrough in HCC treatment and is now considered standard treatment for advanced HCC.

Sorafenib has been shown to be transported by both P-gp and BCRP in in vitro and in vivo models. Agarwal and Elmquist showed that P-gp and BCRP together limit the brain distribution of sorafenib with BCRP being the dominant transporter.

<u>Therapeutic application: Concomitant modulation of ATP release by ABCG2 (BCRP) and vacuolar ATPase as target for the development of dendritic cell vaccines</u>

The field of immunotherapy for the treatment of cancer, including high-grade gliomas, is moving into the direction of active specific immunotherapy using autologous dendritic cells as vehicle for immunization.

Furthermore, novel strategies for treating cancer, including glioma, pancreatic carcinoma and esophageal carcinoma, are dendritic cell therapy combined with anticancer drugs, as was shown for example for temozolomide and gemcitabine (55,56).

*Dendritic cells*

Dendritic cells (DCs) are bone marrow-derived cells that populate all lymphoid and nonlymphoid organs. They have a central role in immune regulation, ranging from tolerance induction and the prevention of autoimmunity to the induction of antitumor immunity and the protection against infectious agents.

DCs are seen as nature's adjuvant and have the potential to recognize foreign antigen, process it into small peptides for presentation onto MHC molecules to the T cell receptor (TCR), and to provide the essential costimulatory molecules for activation of naive CD4+ and CD8+ T cells. Subsequently, CD4+ T cells provide help for B cell class-switching and induction of long-lived plasma cells. DCs have an immature phenotype in peripheral tissues, specialized for antigen uptake, but upon recognition of foreign material migrate to the lymph node T cell paracortex where they arrive as mature cells, expressing all costimulatory molecules and having lost the capacity to take up antigens. The response of DCs to exposure to foreign antigens is part of the innate immune response, and by providing a link between antigen recognition and antigen processing for presentation to naïve T cells, these cells bridge innate and adaptive immunity.

Human DCs originating from pluripotent hematopoietic stem cells can be divided into at least three subpopulations: interstitial DCs residing in the skin and lymphoid organs and two subsets of blood DCs, the CD11 c+ myeloid and CD11 c- plasmacytoid DC. The two subsets of blood DCs not only differentially express CD11c and CD123 (IL-3R $\alpha$-chain), but also show distinct patterns of Toll-like receptor expression and regulation by cytokines. Circulating blood DCs are rare (they account for <1% of human PBMC) and are difficult to maintain in culture.

Improved understanding of the biology of DCs has revealed that three interactive signals impinge on lymphocyte responses and are important for consideration in vaccine development in order to achieve optimal activation of both innate and adaptive antitumor immunity: (i) adequate DC presentation of MHC-peptide complexes for induction of antigen-specific T cells, with simultaneous expression of activation ligands for stimulation of innate natural killer (NK) cells; (ii) dominant positive costimulation via molecules such as CD40, CD80, and CD86 and (iii) secretion of cytokines that polarize immune responses in a Th1/Tc1 direction to create optimal antitumor responses.

The way by which DCs are activated by pathogens can be direct, through recognition of pathogen-associated microbial patterns (PAMPS) like endotoxin, peptidoglycan, or unmethylated CpG motifs on Toll-like receptors (TLRs), or indirect, through recognition of damage associated molecular patterns (DAMPs) like ATP, uric acid, or HMGB-1, released upon cellular stress or tissue damage inflicted by pathogens.

*DC vaccines and vaccine adjuvants*

The broad range of powerful immune stimulatory and regulatory functions has made DCs targets for vaccine development strategies. This includes cellular vaccination for treatment of cancer or infectious diseases, as well as "negative vaccination" for the treatment of autoimmune diseases and prevention of allograft rejections.

The outcome of the first DC-based clinical trials emphasizes the importance of the quality of DCs, especially their migratory capacity and ability to induce potent T-cell responses. Notably, only a small percentage of the DCs injected in current trials actually migrate from the injection site into the draining lymph node to present the antigen to T cells.

Vaccination strategies aimed at inducing immunity to fight cancer or infectious diseases need to include means to mature the targeted DC. Maturation and activation stimuli can be applied systemically, separate from the targeting vector.

It is now clear that many agents with adjuvant activity on the immune response such as bacterial endotoxin, monophosphoryl lipid A, Freund's adjuvant, bacterial CpG motifs, MF59, and agalactosylceramide boost immunity through induction of dendritic cell (DC) maturation.

*Dendritic cell (DC) activation by extracellular ATP as mechanism* of *action of vaccine adjuvants: aluminum adjuvant and inflammasome activation*

The most widely used adjuvants in both veterinary and human vaccines are aluminum compounds (57). Understanding the mechanism of currently used adjuvants, such as aluminum, will be beneficial for the development of new adjuvants. Although several mechanisms have been described, there is still a need for better understanding of the adjuvant mechanism of aluminum compounds. As aluminum adjuvants have limitations, including local reactions, augmentation of IgE responses, ineffectiveness for some antigens and inability to augment cell-mediated immune responses, especially cytotoxic T-cell responses, there is a need to discover new adjuvants (57).

One explanation commonly used is that aluminum delays clearing from the injection-site. However, recent work showed that immunopotentiation by aluminum also occurred without adsorption of antigen to aluminum phosphate. Another explanation is that soluble antigens may upon adsorption be presented to immunocompetent cells in a particulate manner which could facilitate antigen targeting, i.e., aluminum favouring antigen uptake by antigen presenting cells.

Recent research showed that the adjuvant aluminum hydroxide activates inflammatory dendritic cells through activation of the NALP3 inflammasome. This activation can occur directly, via triggering of the NALP3 inflammasome by alum crystals, or indirectly through the release of one of the two danger signals known to activate NALP3, namely ATP and uric acid, upon cellular stress and tissue damage inflicted by pathogens (58). Alum immunization with antigen resulted in strong Th2 skewed immune responses in mice, including the induction of IgE and IgG1 antibody isotypes. An exciting application derived from these studies is in the development of new vaccine adjuvants, where TLR-based adjuvants have thus far proven to be too toxic and a molecular understanding of how to target and modulate adaptive immunity has been challenging. Although alum will likely prove to have multiple immunostimulatory properties, nucleotide oligomerization domain (NOD)-like receptor (NLR)-based activation appears to be an effective alternate strategy in the innate-adaptive paradigm to generate immunity-one that can be built upon to tailor a new generation of adjuvants.

*Aluminum adjuvant and vacuolar $H^+$-ATPase and the cellular microenvironment*

Traditionally, aluminum hydroxide (Alhydrogel, Malgeldrate) has been applied as gastric antacid, based on the acid-neutralizing capacity.

In root apices, it has been shown that aluminum inhibits plasma membrane $H^+$-ATPase activity by permanently altering the plasma membrane surface potential. In contrast, Hamilton et al. showed that vacuolar ATPase and mitochondrial ATP synthase were induced by aluminum.

Aluminum hydroxide adjuvant exhibits very low solubility in aqueous media, but the solubility increases in acidic or alkaline media. The adsorbent properties of aluminum hydroxide adjuvant are related to the surface area as well as the surface groups. The surface is composed of hydroxyl groups coordinated to aluminum. Such hydroxyl groups, known as metallic hydroxyls, can accept a proton to produce a positive site or can donate a proton to produce a negative site at the surface. Thus, the surface charge of aluminum hydroxide adjuvant is pH dependent.

Aluminum adjuvant is crystalline. It is a basic principle of colloid chemistry that charged particles electrostatically attract ions of opposite charge (counterions) to form the Gouy-Chapman double layer. Negatively charged surfaces attract cations including protons to form the double layer. Thus, the pH of the double-layer region (i.e., microenvironmental pH) will be lower than the bulk pH. Similarly, the double layer surrounding positively charged particles will be rich in anions, including hydroxyls, and the microenvironment pH will be higher than the bulk pH. Antigens that are adsorbed to aluminum-containing adjuvants may be exposed to a pH that is different from the bulk pH, especially if the adjuvant has a surface charge. The differences in pH between microenvironment pH and bulk pH may be in the order of 2 pH units, thereby influencing the rate of acid-catalyzed hydrolysis.

*Concomitant modulation of ATP release and the intracellular pH* as *target for immunemodulation in vaccine development for infectious diseases*

In malaria, an infectious disease, which is caused by protozoan parasites of the genus Plasmodium that infect and destroy red blood cells of its host, the intraerythrocytic parasite exploits the purinergic signaling of its host to adapt the erythrocyte to its requirements. Having entered a red cell, the invading parasite lies dormant for some hours (the ring stage), after which it begins a period of rapid growth (the trophozoite stage) followed by division (schzogony), resulting in the generation of 20-30 new parasites. The metabolic and biosynthetic activity of the malaria trophozoite is intense. The parasite is wholly reliant on glycolysis as its energy source, and it consumes glucose and produces lactic acid at a rate some 100 times higher than does a normal, uninfected erythrocyte. The high metabolic activity of the parasite generates a substantial intracellular acid load. In addition, in the in vivo situation, malaria infection commonly gives rise to a pronounced extracellular acidosis. For the parasite to remain viable, it must therefore have an effective means of protecting its intracellular pH (pHi) from both intra- and extracellular acid loads.

Upon invasion of the erythrocyte, the malaria parasite establishes a parasitophorous vacuole inside which it develops. In addition, the parasite exports a membrane network into the host cytoplasm. One of proteins that are inserted into the erythrocyte host membrane is a functional malaria parasite-encoded (V)-H+-ATPase. This pump has a role in the maintenance of the intracellular pH (pHi) of the infected erythrocyte (59).

Mature human erythrocytes express functional ionotropic $P_2X$ purinoceptors. It has been shown that ATP release by anion channels in the host erythrocyte membrane was involved in the infection with the malaria parasite (21). Furthermore, it has recently been shown that the "danger signal" uric acid, which - like ATP - is released from injured or dying cells and stimulates the maturation of dendritic cells, accumulated within *P. falciparum* infected erythrocytes within the parasitophorous vacuole of replicating *P. falciparum*, and was subsequently released upon rupture of infected erythrocytes (60). The uric acid precipitates induced the maturation of human dendritic cells *in vitro,* and this response is significantly inhibited when uric acid is depleted. It is assumed by the inventor that this effect of uric acid precipitate might be the result of lowering the pH at the side of the drug transporter BCRP. Thus, modulation of the release of the danger signals ATP and uric acid by BCRP at low pH provides a novel target in malaria treatment. Of importance, ABC transporters are also expressed in *Plasmodium falciparum*, leading to resistance to antimalarial drugs, such as artemisinin.

In addition, enveloped viruses, such as influenza virus, penetrate their cell targets following the merging of their membrane with that of the cell. After this interaction, these virus particles are internalized in endosomes. Endosomes maintain a slightly acidic internal pH. For efficient entry of influenza virus into cells a pH gradient is necessary. Acidification of endosomes is regulated by a variety of factors, such as proton leak, CIC chloride channels, or Na+/K+-ATPases, but is primarily determined by the activity of the ATP-dependent vacuolar ATPase (22). Notably, the Na+/K+-ATPase is the most ATP-consuming process in cells; Na+/K+ transport has been reported to utilize > 30% of the basal ATP concentration. For an efficient viral cycle it is necessary to maintain a fairly constant intracellular ATP level. After viral replication, influenza viruses bud from the plasma membrane of the virus-infected cells. Budding is a critical step in virus replication. Influenza virus budding is dependent on the supply of energy from ATP hydrolysis; reduction in ATP level corresponded to the inhibition of virus release. Thus, the intracellular ATP level is of relevance for an efficient virus release from cells. Thus, by concomitant inhibition of vacuolar ATPase and BCRP both the pH gradient and the ATP uptake into the endosome is reduced, thus endosomal uptake of virus is inhibited.

In conclusion, the current finding of an *in vitro* method for the screening of new immunomodulators that modulate the ATP transport by the drug transporter ABCG2 (BCRP) or multidrug resistance-associated transporters ABCC1-6 (MRP1-6) at low pH (with or without concomitant inhibition of v-ATPase) contributes to the development of vaccines and/or immunotherapeutics for cancer, in particular high-grade gliomas and brain metastases, and for infectious diseases, such as malaria and influenza.

*Herbal products and the search for novel vaccine adjuvants*

Nowadays, it has been recognized that herbal products, such as traditional Chinese medicines (TCM), Ayurvedic medicines, plant extracts (root or leaf extracts), and plant-derived compounds (phytochemicals), are potential sources for offering clinically relevant therapeutics or leads. A typical example of this is the development of the anti-malarial drug artimisinin, which was isolated from the plant *Artimisia annua*, a herb that has been used for more than two thousand years by Chinese herbalists. Other prominent examples of plant-derived compounds are the anticancer drugs paclitaxel, isolated from *Taxus brevifolia*, and camptothecin, isolated from the Chinese Happy Tree (Xi Su, *Camptotheca accumulata*) whose derivatives irinotecan and topotecan are clinically established in cancer treatment. Thus far, only a few adjuvants have been licensed for use in humans. The main reason for the lack of effective adjuvants is the toxicity of immune stimulatory compounds, as for example is the case with lipopolysaccharide (LPS). Besides being non-toxic, an optimal adjuvant should also be cheap to produce, biodegradable, biocompatible, immunologically inert, stable, and with long shelf life. It should promote not only humoral immunity, but also induce cellular immune response without inducing IgE antibodies (i.e., be non-allergenic).

It has already been suggested in literature that herbal products could be used as (source for) immunological adjuvants and may fulfill the criteria for an optimal adjuvant (61). For example, Quan et al. showed that intranasal co-administration of Korean red ginseng (Panax ginseng) or Salviae radix extract with inactivated influenza virus A (PR8) increased the levels of influenza specific antibodies and neutralizing activities compared to immunization with PR8 alone. Co-administration of Salviae radix extract significantly enhanced Th1 type cytokines IFN-$\gamma$ and IL-2, whereas Ginseng augmented Th2 type cytokine responses (IL-4, IL-5). Interestingly, Rivera et al. showed that Ginseng extract and aluminum hydroxide act synergistically as vaccine adjuvants when combined with inactivated porcine parvovirus in guinea pigs (62). Another example, ginsenoside Re isolated from the root of Panax ginseng co-administrated with split inactivated H3N2 influenza virus antigen in mice significantly enhanced serum specific IgG, IgG1, IgG2a and IgG2b responses, HI titers, lymphocyte proliferation responses as well as IFN-$\gamma$ and IL-5 secretions, indicating that both Th1 and Th2 were activated. Furthermore, it has been shown that saponins derived from TCM such as Panax ginseng (root, stem and leaf), Astragalus species (root), Panax notoginseng (root), Mormordica (seed), Glycerrhiza (root and rhizome), Achyranthes bidentatae (root) and Platycodon grandiflorum (root) had an immune-modulatory effect.

Another example, it was shown recently that the anti-tumorigenic mushroom Agaricus blazei Murill (AbM) enhances transcription of IL-1$\beta$ and triggers NLRP3 inflammasome-mediated IL-1$\beta$ secretion in human THP-1 macrophages. AbM-mediated IL-1$\beta$ secretion was markedly reduced in macrophages deficient in NLRP3 and ASC, demonstrating that the NLRP3 inflammasome is essential for AbM-induced IL-1$\beta$ secretion. In addition, caspase-1 was activated and involved in proteolytic cleavage and secretion of IL-1$\beta$ in AbM-treated macrophages. Importantly, it was shown that AbM-induced inflammasome activation requires the release of ATP, binding of extracellular ATP to the purinergic receptor $P_2X_7$, the generation of reactive oxygen species, and efflux of potassium.

Interestingly, Ginkgo biloba extract and one of its constituents, bilobalide, were shown to inhibit the hypoxia-induced decrease in ATP content in endothelial cells in vitro probably by preserving ATP regeneration by mitochondria. Furthermore, Eckert et al. showed that Ginkgo biloba extract reduced the levels of reactive oxygen species, including the superoxide anion radical, were reduced in brains from GBE-treated mice compared with controls. In older mice, GBE resulted in a protective effect by increasing production of adenosine triphosphate in neurons.

Furthermore, it was very recently demonstrated, that a nano-formulation of curcumin significantly decreased the proliferation of esophageal adenocarcinoma cells, significantly upregulated the expression of the co-stimulatory molecules CD86 in DCs, and significantly decreased the secretion of pro-inflammatory cytokines from in vitro activated T cells (56).

*The role of ABC transporters in the transport of herbal compounds*

As described before, P-gp (ABCB1), MRPs (MRP1-5, ABCC1-5) and BCRP (ABCG2) may act as barriers (in intestine, brain etc.) limiting the absorption and elimination of many drugs and endogenous compounds, including many herbal constituents.

Thus far, most studies only address inhibition of P-gp function or stimulation of P-gp expression by phytochemicals (63). For example, it has been shown that P-gp plays a role in the active transport of tanshinone IIA, tanshinone IIB and cryptotanshinone, three compounds present in *Salvia miltiorrhiza* extract (64). This implies that P-gp inhibitors could potentially be identified from Chinese herbs. Only a few studies investigated the role of BCRP and/or MRP1 and/or MRP2 in the transport of plant-derived compounds.

BCRP (ABCG2) was shown to be significantly inhibited by flavonoids (apigenin, biochanin A, chrysin, daidzein, daidzin, fisetin, genistein, hesperetin, kaempferol, luteolin, naringenin, phloretin, quercetin, silybin and silymarin) in in vitro studies (65), which are present in many herbs, such as in Ginkgo biloba (65). Enokizono et al. also showed that Bcrp limits the oral bioavailability and penetration into the brain, testis, and epididymis of genistein and the penetration of the phytoestrogens daidzein and coumestrol into brain and testis in mice. Also Van de Wetering et al. showed that ABCG2 limits the systemic exposure to many different phytoestrogens by directing their sulfate conjugates for excretion via the feces.

Also resveratrol, a dietary stilbenic phenolic compound, present mainly in grape and grape products, was shown to be transported by BCRP at low pH, but not at physiological pH (24). Furthermore, the polyphenolic compound curcumin, a diferuloylmethane derived from dried rhizomes of the perennial herb *Curcuma longa* Linn (commonly known as turmeric), has been shown to be a BCRP inhibitor in mice and in humans and was shown to have chemosensitizing, radiosensitizing, wound healing, antimicrobial, antiviral, antifungical, cholekinetic, anti-inflammatory, and immunomodulatory activities.

Furthermore, metabolites of ginsenosides (derived from Panax Ginseng) were shown to inhibit BCRP in MCF-7/MX cells (66).

*Use of mature DCs for vaccination purposes*

The development of techniques to generate large numbers of dendritic cells (DCs) *ex vivo* resulted in a number of studies exploring DC-based vaccination strategies. The first clinical study involved B-cell lymphoma patients and used immature DCs (iDCs) that were administered intravenously. The iDCs were loaded with the immunoglobulin protein produced by each tumour (idiotype protein), which is tumour specific as the malignant cells are monoclonal and will produce identical immunoglobulin receptors with unique antigenic variable regions. Subsequent studies varied in the way DCs were generated, loaded with antigen or administered. Initial studies used iDCs until it was found that they induced tolerance instead of immunity, resulting in a switch to the use of mature DCs (mDCs).

Mature DCs exhibit reduced phagocytic activity and increased expression of MHC co-stimulatory molecules, and secrete large amounts of immunostimulatory cytokines. Maturing DCs also change their pattern of chemokine receptor expression, rendering them sensitive to lymphoid chemokines. Thereby, mature DCs acquire the capacity to migrate to the T cell areas of draining secondary lymphoid organs, where they encounter naïve T cells and initiate an adaptive immune response.

The types of DC used in vaccination studies included FLT3L-expanded DCs, CD34+ DCs, DCs generated with IFN$\alpha$ or IFN$\beta$ and allogeneic DCs. DCs were pulsed with peptides, loaded with proteins or transfected with RNA encoding specific antigens (defined RNA). To increase the range of tumour-specific antigens that were presented, DCs were transfected with tumour-derived RNA or fused with tumour cells to generate DC-tumour hybrids. Furthermore, DC-derived exosomes were used for vaccination purposes. Exosomes are membrane vesicles of endocytic origin that are secreted by many cell types. DC-derived exosomes pulsed with peptides are capable of inducing peptide-specific T-cell responses. Several clinical trials evaluated combinations of DC-based therapy with other therapies, such as depletion of regulatory T cells, chemotherapy or administration of IFN$\gamma$. Novel strategies that are currently in clinical trials include the use of TLR-ligand-activated DCs, use of various DC subsets and DC-based therapy in combination with strategies that target co-stimulation molecules, such as CTLA4, OX40, 4-1BB or PD1. 4-1BB, tumour-necrosis factor receptor superfamily, member 9.

*Ex vivo production of mature DCs*

Ex vivo generated monocyte-derived DCs are used as a cellular vaccine against cancer in clinical trials. In order to be able to induce an efficient tumour-specific CTL response during immunotherapy, DCs have to be able to migrate to the lymph node and produce the Th1 polarizing cytokine, IL-12p70, upon encounter of T cells in the lymph node. However, most clinically used DCs do not produce IL-12p70 upon T cell contact, and lack the capacity to induce type 1 immunity (67).

The current "gold standard" DC maturation cocktail for clinical trials is a monocyte-conditioned medium-mimic consisting of pro-inflammatory mediators TNF$\alpha$, IL-1$\beta$, IL-6 and $PGE_2$ first reported by Jonuleit et al., which induces high migratory potential toward the lymph node expressed CCR7 ligands. This cocktail, however, does not induce the production of IL-12p70, important for the production of a proper CTL response (68). Commonly, monocytes are cultured for 5-7 days with GM-CSF and IL-4 to generate immature DCs that have to be activated for another 2-3 days with microbial, proinflammatory, or T-cell-derived stimuli to obtain mature DCs with full T stimulatory capacity (69).

Dauer et al. showed that when pro-inflammatory mediators (TNF$\alpha$, IL-1$\beta$, IL-6 and $PGE_2$) were added to monocyte DC after 24h culturing with GM-CSF and IL-4 to accelerate DC maturation, cells displayed a fully mature DC immunophenotype ($CD83^+$ $CD40^+$ $CCR7^+$ CD14- $CD80^+$ $CD86^{high}$ $MHCII^{high}$) after only 48 h of total culture period (69).

Frankenberger and Schendel investigated the use of TLR agonists to regulate specific cytokine production in young mDCs, allowing them to provide appropriate signals for polarization of CD4+ T cell responses in a Th1-direction, to efficiently stimulate CD8+ CTL responses and to activate natural killer cells. They demonstrated that 3 day cultured mDCs matured with TLR agonists, displayed a much higher stimulatory capacity for naïve T cells than 3d mDC matured with TNFα, IL-1β, IL-6 and $PGE_2$ alone.

Now, with the present invention it is possible to screen for modulators of ABC transporter-mediated ATP release that could be used for the production of mature DCs for the development of DC vaccines or could be combined with DC vaccines, in particular for the treatment of high-grade glioma or malaria.

Description of the figures

**Figure 1: Transport of ATP by BCRP (ABCG2).**

A) Time course of uptake of 100 μM [$^3$H]ATP at pH 7.4 and pH 6 into membrane vesicles containing BCRP compared to wild-type (WT) control vesicles (n=4);
B) Net uptake of 100 μM [$^3$H]ATP at pH 7.4 and pH 6 into BCRP membrane vesicles over time (corrected for transport in wild-type vesicles).

**Figure 2: Concentration dependency of ATP transport by BCRP (ABCG2) at pH 6 (A) and pH 7.4 (B).**
Sf9-BCRP membrane vesicles were incubated with the concentrations of [$^3$H]ATP and at the pH indicated at 37°C for 5 min. The uptake of ATP is plotted in pmol/mg protein/min. Values shown are means ± SE of each experiment in triplicate.

**Figure 3: Transport of ATP by BCRP (ABCG2).**
Time course of net uptake of 100 μM [$^{32}$P]γ-ATP at pH 7.4 and pH 6 into BCRP membrane vesicles (corrected for transport in wild-type vesicles).

**Figure 4: Effect of pantoprazole (4A) and methotrexate (4B) on the transport of ATP by BCRP (ABCG2).**
A) Net uptake of 89 μM [$^3$H]ATP at pH 6 into membrane vesicles containing BCRP (corrected for transport in wild-type (WT) control vesicles) in the absence and presence of 10 μM and 1 mM pantoprazole (n=3); B) Net uptake of 89 μM [$^3$H]ATP at pH 6 into membrane vesicles containing BCRP (corrected for transport in wild-type (WT) control vesicles) in the absence and presence of 100 μM and 1 mM methotrexate (n=3).

**Figure 5: Schematic representation of the ABC drug transporter mediated-ATP-release assay.**
The uptake of a [3H] or [32P]-radiolabelled ATP by an ABC drug transporter selected from the group consisting of BCRP (ABCG2) or MRP1-6 (ABCC1-6) into membrane vesicles (stably overexpressing one type of ABC transporter) under conditions of low pH (compared with physiological pH) can be studied by radiochemical analysis. Subsequently, the effect of a test compound on the ABC drug transporter-mediated ATP release can be studied at different pH values (not shown in figure).

**Figure 6: Schematic representation of the ABC drug transporter mediated-ATP-release assay.**
The transport of a [3H] or [32P]-radiolabelled ATP by an ABC drug transporter selected from the group consisting of BCRP (ABCG2) or MRP1-6 (ABCC1-6) into MDCKII cells (stably overexpressing one type of ABC drug transporter) under conditions of low extracellular pH (compared with physiological pH) and/or hypoxic conditions (0-1% $O_2$, 5% $CO_2$, 94-95% $N_2$) (compared with normoxic conditions (20% $O_2$, 5% $CO_2$, 75% $N_2$)) can be studied by radiochemical analysis or HPLC. Subsequently, the effect of a test compound on the ABC drug transporter-mediated ATP release can be studied at different pH values (not shown in figure).

**Figure 7: Schematic representation of ABCG2 expression in normal tissues.**
Arrows indicate direction of substrate transport. In the brain and testis, ABCG2 is expressed mainly in the blood vessel endothelium, forming part of the blood-brain and blood-testis barrier, respectively. (from: Robey et al. Curr Pharm Biotechnol 2011)

**Figure 8: Table 1:**
Cellular localization and function of ion exchange pumps

**Figure 9: Table 2:**
Protein and/or gene expression of MRP/Mrp isoforms in various brain compartments in human and in mouse and in rat. Table adapted from Dallas et al. (45).

**Figure 10: Chemical structure of Danshen compounds**

Fig. 10 A: Chemical structures of compounds from Danshen (Salvia miltiorrhiza)
Fig. 10 B: Chemical structures of main lipophilic compounds from Danshen
Fig. 10 C: Chemical structure of main hydrophilic compounds from Danshen
Fig. 10 D: Chemical structure of Magnesium lithospermate B
Fig. 10 E: Chemical structure of salvianolic acid B
Fig. 10 F: Chemical structure of Indirubin

Examples

Material and Methods

ABC Transporter studies

Materials

[$\gamma$-$^{32}$P]-ATP was obtained from Perkin Elmer Inc. (Boston, USA). [$^{3}$H]-ATP was obtained from Moravek Biochemicals (Brea, CA, USA). Pantoprazole (Pantozol® 40 mg i.v.) was from Altana Pharma, Hoofddorp, The Netherlands and MTX (Emthexate PF® 25 mg/ml) was from Pharmachemie, the Netherlands. PSC333 was from Novartis (Basel, Switzerland). Creatine phosphate and creatine kinase were obtained from Boehringer Mannheim (Almere, the Netherlands) and OE67 filters were from Schleicher and Schuell (Dassel, Germany). All other chemicals and reagents were of analytical grade and were purchased from Sigma (St. Louis, MO).

Preparation of membrane vesicles and vesicular transport assays

Membrane vesicles from (ABC transporter-transfected) Sf9 cells were prepared in isotonic vesicle buffer consisting of 50 mM Tris and 250 mM sucrose adjusted to pH 7.4. Vesicular transport assays were performed in vesicular transport assay buffer (50 mM HEPES/KOH, 100 mM KCl) as described before (Breedveld et al., 2004, 2007). The vesicular transport assay buffer and the solutions of all other reagents used in transport assays were adjusted to the appropriate pH (pH 6 or pH 7.4) by adding aliquots of HCl (37% w/w) for low pH. The pH of the final solution was controlled (Breedveld et al., 2007). The time- and concentration-dependent uptake of radiolabelled substrates into membrane vesicles was studied following the rapid filtration method as previously described (Breedveld et *al.,* 2004).

Transport across MDCKII monolayer

MDCKII cells (stably expressing ABC transporter of interest) were cultured as described before (Breedveld *et al.*, 2004) (Pavek *et al.*, *2005*). Transepithelial transport assays were performed as described previously (Breedveld *et al.*, 2004). Media were adjusted to pH 6.0 or 7.4 with HCl (37% w/w) and buffered with 25 mM HEPES, immediately before addition of radiolabeled substrate.

Kinetic and statistical analysis

Curve fitting was performed by nonlinear regression analysis using the software package Graphpad Prism version 4 (GraphPad Software, San Diego, USA). Data from concentration-dependent transport assays were analyzed according to the classical Michaelis-Menten model (eq. 1) (Rowland and Tozer, 1995):

$$\nu = \frac{V_{max} \times [S]}{K_m + [S]} \qquad (1)$$

where v is the rate of transport, $V_{max}$ is the maximum velocity, S is the substrate concentration, and $K_m$ is the substrate concentration at half-maximum velocity. The two-sided unpaired Student's *t*-test was used throughout to assess the statistical significance of the difference between two sets of data. Results are presented as means ± standard error. Differences were considered to be statistically significant when $P < 0.05$.

Results

The effect of low pH on transport of ATP by BCRP (ABCG2).

Recently, it was reported that BCRP (ABCG2) transports substrate drugs more efficiently at low pH. This was shown for MTX, its major metabolite 7-OH-MTX, MTX diglutamate, folic acid, mitoxantrone, topotecan and resveratrol (24).

Despite great interest in the mechanisms that regulate cellular ATP permeability, the molecular basis for (non-lytic) ATP release from endothelial cells is still a matter of debate. Therefore, we investigated whether ATP is released by BCRP due to low pH, which is a pathophysiological condition, as exists in the tumor microenvironment.

By using Sf9-BCRP membrane vesicles, we studied the effect of pH (pH 6 and pH 7.4) on the BCRP-mediated transport of [$^3$H]ATP, and compared this to the transport of [$^3$H]ATP at the same chosen pH values in Sf9 wild-type vesicles (Fig 1A). In Sf9 wild-type membrane vesicles, there was no significant difference in the uptake of 100 $\mu$M [$^3$H]ATP at pH 7.4 compared with the uptake at pH 6 (Fig. 1A). The transport of 100 $\mu$M [$^3$H]ATP by BCRP was 3.8-fold higher at pH 6 than at physiological pH at 0.5 min. These results were corrected for the uptake of [$^3$H]ATP into Sf9 wild-type membrane vesicles (Fig. 1B). At pH 6, the transport of [$^3$H]ATP is saturable, with apparent $K_m$ and $V_{max}$ values of 2.0 mM and 1.5 nmoles/mg protein/min. (Fig. 2A). At pH 7.4, the transport rate increased linearly with the ATP concentration up to 5 mM (Fig. 2B).

One could argue that the observed transport is not ATP but that its breakdown products (adenosine diphosphate (ADP) or adenosine monophosphate (AMP)) are transported into the vesicles, as ATP is subject to degradation by ecto-ATPases, ecto-ADPases and ecto-nucleotidases at the cell surface in all extracellular spaces. Therefore, it was also demonstrated that BCRP transports [$^{32}$P]-$\gamma$ labeled ATP, as is shown in Figure 3. In contrast to the [$^3$H]-ATP transport by BCRP, the BCRP-mediated transport of [$^{32}$P]-$\gamma$-labeled-ATP was minimal at physiological pH, but at pH 6 the uptake was 80 pmol/mg protein at 0.5 min., which increased linearly to 194 pmol/mg protein at 10 min. (Fig. 3). Thus, BCRP transports ATP at low pH, whereas the transport at physiological pH is minimal.

The effect of BCRP inhibitors on the BCRP-mediated transport of ATP at low pH.

It has been shown before that both the proton pump inhibitor pantoprazole and the antifolate drug methotrexate are transported by BCRP at physiological pH (Breedveld et al., 2004). For MTX it was demonstrated that the transport at low pH was increased compared with physiological pH (24).

It has now been shown by using Sf9-BCRP vesicles that the BCRP-mediated transport of [$^3$H]ATP at low pH can be inhibited by the BCRP substrate drugs pantoprazole and methotrexate (Fig. 4). However, 10 $\mu$M pantoprazole reduced the BCRP-mediated ATP release at low pH by appr. 15% and high concentrations of pantoprazole (>1 mM) were required to decrease the transport of ATP by 50%. Also with MTX the transport was not completely inhibited yet, 100 $\mu$M, an intermediate-dose, inhibited the ATP transport by appr. 46%.

ABC Transporter studies

Materials.

Salvianolic acid A and B, tanshinone I and IIA, rosmarinic acid, protocatechuic acid (ethyl ester), 2'z-indirubin, and indirubin-3'-oxime are purchased from Sigma (Sigma-Aldrich, Zwijndrecht, The Netherlands). Salvia mithiorrhiza root extract and Radix isatidis extract are obtained from NatuurApotheek (Pijnacker, the Netherlands). Temozolomide is obtained from Schering-Plough, bevacizumab from Genentech/Hoffmann-laRoche, sunitinib and sorafenib are purchased from Sequoia Research Products (Pangbourne, UK). Pantoprazole (Pantozol® 40 mg i.v.) was from Altana Pharma, Hoofddorp, the Netherlands and MTX (Emthexate PF® 25 mg/ml) was from Pharmachemie, the Netherlands. Heparin (5000 IU/ml) is obtained from Leo Pharma BV (Breda, the Netherlands). Lithium heparinized microvettes and dipotassium-EDTA microvettes are obtained from Sarstedt (Numbrecht, Germany). EDTA disodium salt pH 8.0 is from Cambrex BioScience Inc. (Rockland, ME). Bovine serum albumin (BSA), fraction V, was purchased from Roche (Mannheim, Germany). Isoflurane (Forane) is from Abbott Laboratories (Queenborough, Kent, UK). All other chemicals and reagents are obtained from Sigma-Aldrich (Steinheim, Germany).

*In vitro* ABC transporter experiments

The effect of herbal compounds on the BCRP-mediated transport of ATP at low pH.

By using Sf9-BCRP membrane vesicles, we study the effect of herbal compounds selected from the group of salvianolic acids, lithospermic acids, tanshinones, protocatechuic acids, indirubins on the BCRP-mediated transport of 1 and 100 μM [$^{32}$P]ATP at pH 6 and pH 7.4, and compare this to the transport of [$^{32}$P]ATP at the same chosen pH values in Sf9 wild-type vesicles. In addition, we test the effect of co-administration of selected herbal compounds and the BCRP and proton pump inhibitor pantoprazole (10 μM) or esomeprazole on the transport of [$^{32}$P]ATP at the same conditions.

The effect of anticancer drugs on the BCRP-mediated transport of ATP at low pH.

By using Sf9-BCRP membrane vesicles, we study the effect of the anticancer agents temozolomide, bevacizumab, sunitinib, and sorafenib on the BCRP-mediated transport of 1 and 100 μM [$^{32}$P]ATP at pH 6 and pH 7.4, and compare this to the transport of [$^{32}$P]ATP at the same chosen pH values in Sf9 wild-type vesicles. We use methotrexate as control. In addition, we test the effect of co-administration of the BCRP and proton pump inhibitor pantoprazole (10 μM) or esomeprazole and/or a herbal compound (5 μM, 50 μM, 500 μM) selected from the group of salvianolic acid B, Mg lithospermate B and indirubin on the transport of [$^{32}$P]ATP at the same conditions.

In addition to the membrane vesicle studies, we investigate whether low pH also affects BCRP-mediated efflux of ATP from intact cells. For this purpose, we assess in the transwell model the transport of ATP in MDCKII-BCRP cells at pH 7.4 and at pH 6.5 and compare this with the transport of ATP in control cells at the same pH values, and we measure the intracellular ATP concentrations. In addition, we test the effect of the BCRP and proton pump inhibitor pantoprazole (20 or 100 μM) or esomeprazole and/or a herbal compound selected from the group of salvianolic acid B, Mg lithospermate B and indirubin on the transport of [$^{32}$P]ATP at the same conditions, and we compare this to the administration of 5 μM elacridar (as control). In addition, we test the concomitant administration of the anticancer agent imatinib (1 μM).

These experiments in the Transwell model will also be performed with a co-culture of porcine brain endothelial cells (PBEC) and primary rat astrocytes (CTX-TNA2 cell line) as intact cells.

The effect of low pH on transport of ATP by MRP1-6 (ABCC1-6).

By using Sf9-MRP1,2,3,4, 5 or 6 membrane vesicles, we study the effect of pH (pH 6 and pH 7.4) on the MRP1-6-mediated transport of 1 and 100 μM [$^{32}$P]ATP, and compare this to the transport of [$^{32}$P]ATP at the same chosen pH values in Sf9 wild-type vesicles. We study the effect of herbal compounds selected from the group of salvianolic acid B, Mg lithospermate B, tanshinone I, and indirubin on the MRP-mediated transport of 1 and 100 μM [$^{32}$P]ATP at pH 6 and pH 7.4.

The effect of herbal compounds on chemotherapy-induced ATP release from brain tumor cell lines.

We expose a panel of (side population of) brain tumor cell lines (human U87-MG, the rat C6 glioma cell line or from patient biopsies) to temozolomide or chemotherapeutic agents that are currently under investigation for the treatment of glioblastoma multiforme, i.e. bevacizumab, sunitinib, sorafenib, or imatinib. In addition, we test the effect of concomitant administration of various herbal compounds from Salvia miltiorrhiza root extract (water soluble phenolic compounds, salvianolic acid A and B and magnesium lithospermate B, and lipophilic tanshinones) and from Radix isatidis extract (indole alkaloids). We assess short and long term morphological change, growth inhibition and cell death (by MTT assay) after respectively overnight exposure or 72 h exposure to the compounds, and measure both intracellular and extracellular ATP concentrations.

*In vivo* ABC transporter experiments

Animals

Mice were housed and handled according to institutional guidelines complying with Dutch legislation. Animals used were male Bcrp1-/- (Bcrp1 knockout) and wild-type mice of a comparable genetic background (FVB or mixed 129/Ola and FVB) between 9 and 14 weeks of age. Animals were kept in a temperature-controlled environment with a 12-h light/12-h dark cycle and received a standard diet (AM-II; Hope Farms, Woerden, The Netherlands) and acidified water *ad libitum*.

Drug preparation

Sorafenib tosylate is dissolved in DMSO (25 mg/ml) and 25-fold diluted with Cremophor EL/ethanol/water (1:1:6, v/v/v). Sunitinib malate is dissolved in DMSO at a concentration of 25 mg/ml and further diluted with 50 mM sodium acetate buffer (pH 4.6) to yield a concentration of 1 mg/ml. Sorafenib and sunitinib are administered orally at 10 mg/kg body weight (10 ml/kg). A vial of pantoprazole is diluted with NaCl 0.9% to a final concentration of 8 mg/ml. Salvianolic acid B is dissolved in Cremophor EL/ethanol/water (1:1:6, v/v/v) and indirubin is dissolved in DMSO at a concentration of 25 mg/ml and further diluted with 50 mM sodium acetate buffer (pH 4.6) to yield a concentration of 1 mg/ml.

Plasma pharmacokinetics and relative brain accumulation of sorafenib and sunitinib in mice.

To minimize variation in absorption upon oral administration, mice were fasted for 3 h before sorafenib or sunitinib were administered with or without concomitant administration of either pantoprazole (120 mg/$m^2$) and/or salvianolic acid B or indirubin by gavage into the stomach, using a bluntended needle. To prevent blood from coagulating, heparin was used for the sunitinib pharmacokinetic experiments, whereas EDTA was used for the sorafenib pharmacokinetic experiment. Tail vein blood sampling was performed at 0.25, 0.5, 1, 2 and 4 h time-points after oral administration, using either microvettes containing dipotassium-EDTA or lithium heparin. Six hours after oral administration, mice were anesthetized with isoflurane and blood was collected by cardiac puncture, in which 0.5 M disodium-EDTA or 5000 IU/ml heparin were used as anticoagulants. Immediately thereafter, mice were sacrificed by cervical dislocation and brains were rapidly removed. Plasma was isolated from the blood by centrifugation at 2,100 g for 6 min at 4°C, and the plasma fraction was collected and stored at -20°C until analysis. Brains were homogenized with 1 ml of 4% BSA and stored at -20°C until analysis. Relative brain accumulation after oral administration was calculated by determining the drug brain concentration at 6 h relative to the plasma AUC0-6h, as the AUC better reflects the overall drug exposure of the brain over time than the plasma concentration at 6 h after oral administration.

Drug analyses

Sorafenib and sunitinib concentrations in plasma and brain homogenates are analyzed by liquid chromatography coupled to tandem mass spectrometry. Lower limit of quantification (LLQ) values for sunitinib were 5 ng/ml and 15.6 ng/g for the plasma and brain homogenates, respectively. LLQ values for sorafenib were 10 ng/ml and 31.2 ng/g for the plasma and brain homogenates, respectively.

Statistical analysis

Pharmacokinetic parameters were calculated by non-compartmental methods. The area under the plasma concentration-time curve was calculated using the trapezoidal rule, without extrapolating to infinity. The maximum drug concentration in plasma (Cmax) and the time to reach maximum drug concentration in plasma (Tmax) were determined directly from mean concentration-time data. Data are presented as means ± SD. For parametric statistical analysis, all the data except for plasma concentrations and AUC0-6h values were log-transformed to obtain equality in variances. One-way analysis of variance (ANOVA) was used to determine statistical significance of differences between groups, after which post-hoc tests with Bonferroni correction were performed for comparison between individual groups. Differences were considered statistically significant when $P < 0.05$.

The effect of herbal BCRP modulators and proton pump inhibitors on the treatment of high-grade glioblastoma multiforme with temozolomide using a genetically engineered mouse model of high-grade glioma.

A high-grade glioma mouse model that is very suitable for intervention studies is described by de Vries et al. (70). This was achieved by intracranial injection of Cre lentiviral vectors into Ink4a/Arf;K-Rasv12, Pten;Ink4a/Arf;K-Rasv12 and p53;Ink4a/Arf;K-Rasv12 LoxP conditional adult mice. Tumor incidence and grade of malignancy were highest in the latter genotype. Tumors reproduced many of the histologic features that are characteristic of human gliomas, including tumor cell heterogeneity, invasiveness, vessel permeability, and BBB characteristics. Crossing in of the conditional LucR gene allowed monitoring of tumor growth for a sufficiently long period of time to have a time window for intervention. In particular, the short latency time and the very high incidence seen in p53;Ink4a/Arf;K-Rasv12 mice when using lentiviral CMVCre make this a practical model for use in therapy intervention studies, which was shown in an efficacy study using oral temozolomide 100 mg/kg for 9 d. We test the effect of co-administration of herbal extracts from Salvia miltiorrhiza or Radix Isatidis, or herbal BCRP modulators (salvianolic acid B, Mg lithospermate B or indirubin) and/or pantoprazole or esomeprazole, and temozolomide (4 week treatment) on the reduction of glioblastoma tumor growth.

The effect of herbal BCRP modulators on activation of caspase-1, cytokine release from immune cells and on maturation of DCs under conditions of low pH and/or hypoxia.

Aluminum hydroxide is a widely used adjuvant, and has been shown activate caspase-1, to induce the secretion of chemokines and to enhance monocyte differentiation towards dendritic cells.

In this study, we investigate the effect of herbal BCRP modulators, selected from the group of salvianolic acids, lithospermic acids and indirubins, on the BCRP-mediated release of ATP from immune cells, the activation of caspase-1, the induction of cytokine secretion, and on monocyte differentiation towards dendritic cells under conditions of low pH and/or hypoxia, and we compare this with or without concomitant administration of the proton pump inhibitor pantoprazole or aluminium hydroxide. Lipopolysaccharide (LPS) is used as control, as cytokine release can be directly evoked by the gram-negative bacterial coat component LPS.

Materials, cell culturing and isolation of cells

Reagents

LPS (*Escherichia coli* 055.B5), Alhydrogel (Alum gel, 13 mg/mL), salvianolic acid A and B, tanshinone I and IIA, 2'z-indirubin, indirubin-3'-oxime, are purchased from Sigma (Sigma-Aldrich, Zwijndrecht, The Netherlands). Salvia mithiorrhiza root extract and Radix isatidis extract are obtained from NatuurApotheek (Pijnacker, the Netherlands).

RPMI 1640, Tyrode's buffer, fetal bovine serum, and non-essential amino acids were purchased from GibCo BRL Life Technologies (GIBCO-BRL-Invitrogen Corporation, Carlsbad, CA, USA). Rabbit polyclonal antibodies against IL-1$\beta$, caspase-1 and GAPDH were obtained from Cell signaling (Bioke, Leiden, The Netherlands) and Stressgen (Enzo Life Sciences BV, Zandhoven, Belgium), respectively. The precision protein standards and PVDF membrane were purchased from Bio-Rad (Bio-Rad Laboratories, Veenendaal, The Netherlands). Horseradish peroxidase (HRP)-conjugated rabbit anti-mouse IgG and goat anti-rabbit IgG were purchased from Dako Diagnostics (Dako B.V. Heverlee, Belgium).

Isolation and stimulation of PBMC, monocytes, DCs and neutrophils

Buffy coats are obtained from healthy blood donors upon informed consent and fractionated by Ficoll density centrifugation.

The PBMC layer is recovered, washed three times with RPMI 1640 medium, and resuspended in complete medium (RPMI 1640 supplemented with I-glutamine and 25 mM HEPES, containing 10% FCS (HyClone Laboratories) and 1% penicillin/streptomycin/glutamine). PBMCs are cultured in 96-well flat-bottom plates at a density of $4 \times 10^5$ cells/well.

Monocytes are isolated from PBMCs by magnetic cell separation with anti-CD14 beads using the MACS system (Miltenyi Biotec). Cells are cultured in complete medium in 96-well flat-bottom plates at a density of 2 x $10^5$ cells/well.

Monocyte-derived DCs are obtained by culturing monocytes in DC medium (RPMI 1640 supplemented with 10% FCS (HyClone Laboratories), 1% nonessential amino acid supplement, 1% pyruvate, 1% Glutamax, IL-4 (10 ng/ml), and GM-CSF (10 ng/ml)) in cell-culture dishes at a density of $3 \times 10^6$ cells/ml. At the end of the incubation period, cells are detached by gentle pipetting and cultured in complete medium in 96-well plates. For time course experiments of DC differentiation, the monocytes are directly cultured in 96-well flat-bottom plates with DC medium in the presence of herbal extract (Salvia miltiorrhiza root or Radix isatidis), herbal compound (salvianolic acid, Mg lithospermic acid, or indirubin), and/or pantoprazole (10 $\mu$M) or alum (13 $\mu$g/ml), with or without ATP (1 $\mu$M, 250 $\mu$M or 1 mM), or in the presence of LPS (100 pg/ml). These DC differentiation experiments are performed either at physiological conditions or at pathophysiological conditions (low pH and/or hypoxia). Where indicated, fluorescent latex beads (1 $\mu$m, Fluoresbrite YG Microspheres; Polysciences) were added to monocytes at a concentration of 5 beads/cell, and bead uptake by the cells was visualized by FACS. Cells are analyzed without further maturation as immature DCs (iDCs) or are matured by addition of an inflammatory mix (10 ng/ml IL-1$\beta$, IL-6, and TNF-$\alpha$ and 1 $\mu$M $PGE_2$) during the last 24 h of incubation.

Myeloid DCs are isolated from PBMCs by FACS sorting for lineage negative ($CD3^-$, CD14,$CD19^-$, $CD56^-$)$CD11c^{bright}$ cells. Sorted cells are cultured in complete medium at a density of 1 x$10^4$ cells/well.

To analyze cells from fresh total blood, venous blood is collected from healthy volunteers upon informed consent. RBCs are lysed with ACK lysis buffer (150 mM Nh4Cl, 10 mM KHCO3, 0.1 mM EDTA (pH 7.3), for 5-10 min., and the remaining cells are washed and cultured in complete medium in 15-ml Falcon tubes.

Plasmacytoid dendritic cells are isolated from PBMCs by Diamond Plasmacytoid Dendritic Cell Isolation Kit by using CD304 MicroBeads (Magnetic cell sorting/MACS/; Miltenyi Biotech, Utrecht, the Netherlands). The purity is assessed by the flow cytometric analysis of CD123. Human pDCs were cultured with various stimuli at 37°C under a humidified atmosphere with 5% $CO_2$ in a V-bottom 96-well plate for 8 h for assessing cytokine production ($10^6$/ml).

Human neutrophils are obtained from heparinized venous blood buffy coat by Ficoll-Hypaque centrifugation, followed by sedimentation in 5% dextran/0.9% saline. Neutrophils are separated from erythrocytes by lysis in a solution of 0.15M $NH_4C1$, 0.01 M $NaHCO_3$ and 0.01M tetra EDTA. The recovered neutrophils are resuspended in RPMI 1640 medium supplied with 10% fetal calf serum and essential amino acids and 10 mM 4-(2-hydroxyethyl)-1-piperazineethanesulfonic acid (HEPES), pH 7.2 and washed three times. The purity of neutrophils preparations is determined by Wright's staining of cytospin preparations.

T cells for mixed lymphocyte reaction assay (MLR) are isolated from PBMCs by magnetic cell separation using the MACS Naive $CD4^+$ T Cell Isolation kit (Miltenyi Biotec). $CD4^+$ T cells are cultured in RPMI 1640 medium supplemented with 10% heat-inactivated FBS, 2 mM L-glutaine, 100U/mL penicillin, and 100 $\mu$g/mL streptomycin at 37% in 5% $CO_2$. $CD4^+$ T cells are activated for 3 d in flat-bottom 96-well plates pre-coated with anti-CD3 mAb in presenc of soluble anti-CD28 mAb.

For stimulation experiments, 5 x $10^6$ PBMC, or 1 x $10^6$ DC are plated in 24-well plates in 0.5 ml RPMI 1640/10% FCS and stimulated with the various test compounds (Salvia miltiorrhiza root extract, Radix isatidis extract, salvianolic acid, Mg lithospermic acid, and/or pantoprazole, and/or alum and/or LPS) at varying doses and time spans.

Cytokine secretion

Cell-free culture supernatants were collected and stored at -20°C until analysis. Cytokine secretion was measured by Bio-Plex analysis (27 plex panel) (Bio-Rad), according to manufacturer's instructions. The following soluble proteins are assayed: IL-1$\beta$, IL-6, IL-8, IL-10, IL-12p70, IFN-$\gamma$, IP-10, MCP-1 (CCL2), MIP-1$\alpha$ (CCL3), MIP-1$\beta$ (CCL4), TNF-$\alpha$. Furthermore, IL-18 is measured in supernatants by ELISA.

Flow cytometric phenotypic analyses

After 48 h of incubation with or without ATP, DCs were immunophenotyped using fluorescein isothiocyanate (FITC)-, phosphatidylethanolamine (PE)-,or allophycocyanin-conjugated monoclonal antibody: anti-CD1a (1:25), anti-CD54 (1:25), anti-CD86 (1:25), anti-CD40 (1:10; BD Pharmingen, San Diego, CA), anti-CD14 (1:25), anti-HLA-DR (1:25; BD Biosciences, San Jose, CA), anti-langerin (CD207), anti-CD83 (1:10; Immunotech, Marseilles, France), anti-CD80, anti-CD86 (BD Pharmingen, San Diego, CA) and anti-CD34 (1:10; Sanquin, Amsterdam, The Netherlands); 2.5 to 5 x$10^4$ cells are washed in phosphate buffered saline (PBS) supplemented with 0.1% bovine serum albumin and 0.02% NaN3 and incubated with specific or corresponding control Mabs for 30 minutes at 4°C. Chemokine receptor 7 (CCR7) expression is determined by incubation with rat anti-CCR7 mAb, followed by incubation with ant-rat IgG2a-biotynylated mAb) and incubation with streptavidin-allophycocyanin (BD PharMingen). Cells are analyzed on a FACS calibur flow cytometer (BD Biosciences, San Jose, CA) equipped with CellQuest analysis software.

Cell migration assays

Cell culture supernatants or chemokine containing medium is placed in the lower chamber of a 96-well Transwell plate (pore size: 5$\mu$m; Millipore). Cells are added to the upper chamber at a density of $10^4$ cells/well and allowed to transmigrate for 3 h. The number of transmigrated cells is assessed by FACS.

BCRP expression on monocyte-derived DC

Monocyte-derived DC (immature moDC and mature moDC) are analysed for the expression of BCRP by lightcycler RT-PCR and western blot analysis as described by van de Ven et al. (36)

MLR analysis

APCs are placed in serial dilutions (1/3) into 96-well flat-bottom plates at a starting cell density of $4 \times 10^4$ cells/well. A total of $2 \times 10^5$ naive $CD4^+$ T cells are added per well, and cells were cocultured for 5 days. At 24 h before the end of the incubation period, 50 $\mu$l of culture supernatant was drawn for cytokine analysis, and the cell cultures are pulsed with 0.5 $\mu$Ci of [$^3$H]thymidine (Amersham Biosciences). The incorporation of [$^3$H]thymidine in proliferating cells is measured by a liquid scintillation counter (Packard).

Endocytosis assays

PBMCs are cultured with herbal extract or compound and/or pantorpazole or alum, or LPS for 24 h. Phagocytosis of fluorescent latex beads (1 $\mu$m, Fluoresbrite YG Microspheres; Polysciences) are analyzed after cocultivation with 10 beads/cell throughout the total cultivation period. Cells are stained for CD14 with anti-CD14-allophycocyanin, and the mean fluorescence intensity of $CD14^+$ monocytes in the FITC channel (Fl-1) is analyzed. Endocytosis is assessed likewise by analysis of $CD14^+$ monocytes in PBMCs cocultured with herbal extract or compound and/or pantorpazole or alum, or LPS together with the fluid phase dye Lucifer Yellow CH (Invitrogen Life Technologies) or Alexa Fluor 488-conjugated dextran for 24 h.

Analysis of dead or apoptotic cells

To identify dead or apoptotic cells at the end of the culture period, cells are stained with Annexin V-PE Apoptosis Detection kit I (BD Pharmingen), according to the manufacturer's instructions. Apoptotic cells stain positive for Annexin V, whereas dead cells are Annexin V/7-aminoactinomycin D double positive.

Western blot analysis of inflammasome signalling

The protein concentration is determined by BCA protein assay kit (Pierce). The lysate (25 μg) is subjected to SDS/PAGE [10% (w/v) gel]. The separated proteins are electroblotted on PVDF membranes (Bio-Rad). Membranes are then washed once with Tris/HCl, pH 7.4, containing 159 mM NaCl and 1% Tween 20 (TBS-T), and then blocked in super-blocking buffer (Pierce) for 1 h. After washing the membranes with TBS-T, antibodies against IL-1β and caspase-1 are added for 24h at 4 C as indicated by the supplier. After three washes with TBST, membranes are treated for 1 h with HRP-conjugated goat anti-rabbit IgG for IL-1β and rabbit anti-goat IgG for caspase-1 diluted to 1:20,000 in TBS-T. After three washes with TBS-T, immunoreactive protein bands are revealed with an enhanced chemiluminescence Western blot analysis system (Amersham Pharmacia Biotech). Films are scanned and analyzed on a GS7-10 Calibrated Imaging Densitometer equipped with Quantity One v. 4.0.3 software (Bio-Rad). For detection of equal loaded protein on the gel, the membranes were stripped with stripping buffer (Pierce), incubated with antibody to GAPDH as a housekeeping protein and visualized by ECL.

References

1. Burnstock, G. 2006. Purinergic signalling. Br. J. Pharmacol. 147 Suppl 1: S172-S181.
2. Idzko, M., H. Hammad, M. van Nimwegen, M. Kool, M. A. Willart, F. Muskens, H. C. Hoogsteden, W. Luttmann, D. Ferrari, F. Di Virgilio, J. C. Virchow, Jr., and B. N. Lambrecht. 2007. Extracellular ATP triggers and maintains asthmatic airway inflammation by activating dendritic cells. Nat. Med. 13: 913-919.
3. Tannock, I. F., and D. Rotin. 1989. Acid pH in tumors and its potential for therapeutic exploitation. Cancer Res. 49: 4373-4384.
4. Martins, I., A. Tesniere, O. Kepp, M. Michaud, F. Schlemmer, L. Senovilla, C. Seror, D. Metivier, J. L. Perfettini, L. Zitvogel, and G. Kroemer. 2009. Chemotherapy induces ATP release from tumor cells. Cell Cycle 8: 3723-3728.
5. Duewell, P., H. Kono, K. J. Rayner, C. M. Sirois, G. Vladimer, F. G. Bauernfeind, G. S. Abela, L. Franchi, G. Nunez, M. Schnurr, T. Espevik, E. Lien, K. A. Fitzgerald, K. L. Rock, K. J. Moore, S. D. Wright, V. Hornung, and E. Latz. 2010. NLRP3 inflammasomes are required for atherogenesis and activated by cholesterol crystals. Nature 464: 1357-1361.
6. Lohman, A. W., M. Billaud, and B. E. Isakson. 2012. Mechanisms of ATP release and signalling in the blood vessel wall. Cardiovasc. Res. 95: 269-280.
7. Tu, J., G. Le, and H. J. Ballard. 2010. Involvement of the cystic fibrosis transmembrane conductance regulator in the acidosis-induced efflux of ATP from rat skeletal muscle. J. Physiol 588: 4563-4578.
8. Schwiebert, L. M., W. C. Rice, B. A. Kudlow, A. L. Taylor, and E. M. Schwiebert. 2002. Extracellular ATP signaling and P2X nucleotide receptors in monolayers of primary human vascular endothelial cells. Am. J. Physiol Cell Physiol 282: C289-C301.
9. Qu, Y., S. Misaghi, K. Newton, L. L. Gilmour, S. Louie, J. E. Cupp, G. R. Dubyak, D. Hackos, and V. M. Dixit. 2011. Pannexin-1 is required for ATP release during apoptosis but not for inflammasome activation. J. Immunol. 186: 6553-6561.
10. Braunstein, G. M., R. M. Roman, J. P. Clancy, B. A. Kudlow, A. L. Taylor, V. G. Shylonsky, B. Jovov, K. Peter, T. Jilling, I. I. Ismailov, D. J. Benos, L. M. Schwiebert, J. G. Fitz, and E. M. Schwiebert. 2001. Cystic fibrosis transmembrane conductance regulator facilitates ATP release by stimulating a separate ATP release channel for autocrine control of cell volume regulation. J. Biol. Chem. 276: 6621-6630.
11. Reddy, M. M., P. M. Quinton, C. Haws, J. J. Wine, R. Grygorczyk, J. A. Tabcharani, J. W. Hanrahan, K. L. Gunderson, and R. R. Kopito. 1996. Failure of the cystic fibrosis transmembrane conductance regulator to conduct ATP. Science 271: 1876-1879.
12. Li, C., M. Ramjeesingh, and C. E. Bear. 1996. Purified cystic fibrosis transmembrane conductance regulator (CFTR) does not function as an ATP channel. J. Biol. Chem. 271: 11623-11626.
13. Abraham, E. H., B. Shrivastav, A. Y. Salikhova, K. M. Sterling, N. Johnston, G. Guidotti, S. Scala, T. Litman, K.

C. Chan, R. J. Arceci, K. Steiglitz, L. Herscher, and P. Okunieff. 2001. Cellular and biophysical evidence for interactions between adenosine triphosphate and P-glycoprotein substrates: functional implications for adenosine triphosphate/drug cotransport in P-glycoprotein overexpressing tumor cells and in P-glycoprotein low-level expressing erythrocytes. Blood Cells Mol. Dis. 27: 181-200.

14. Osawa, R., K. L. Williams, and N. Singh. 2011. The inflammasome regulatory pathway and infections: role in pathophysiology and clinical implications. J. Infect. 62: 119-129.

15. Ghiringhelli, F., L. Apetoh, A. Tesniere, L. Aymeric, Y. Ma, C. Ortiz, K. Vermaelen, T. Panaretakis, G. Mignot, E. Ullrich, J. L. Perfettini, F. Schlemmer, E. Tasdemir, M. Uhl, P. Genin, A. Civas, B. Ryffel, J. Kanellopoulos, J. Tschopp, F. Andre, R. Lidereau, N. M. McLaughlin, N. M. Haynes, M. J. Smyth, G. Kroemer, and L. Zitvogel. 2009. Activation of the NLRP3 inflammasome in dendritic cells induces IL-1beta-dependent adaptive immunity against tumors. Nat. Med. 15: 1170-1178.

16. Trabanelli, S., D. Ocadlikova, S. Gulinelli, A. Curti, V. Salvestrini, R. P. Vieira, M. Idzko, V. F. Di, D. Ferrari, and R. M. Lemoli. 2012. Extracellular ATP exerts opposite effects on activated and regulatory CD4+ T cells via purinergic P2 receptor activation. J. Immunol. 189: 1303-1310.

17. Spies, C. M., R. H. Straub, and F. Buttgereit. 2012. Energy metabolism and rheumatic diseases: from cell to organism. Arthritis Res. Ther. 14: 216.

18. Ramakers, B. P., N. P. Riksen, J. G. van der Hoeven, P. Smits, and P. Pickkers. 2011. Modulation of innate immunity by adenosine receptor stimulation. Shock 36: 208-215.

19. Wilhelm, K., J. Ganesan, T. Muller, C. Durr, M. Grimm, A. Beilhack, C. D. Krempl, S. Sorichter, U. V. Gerlach, E. Juttner, A. Zerweck, F. Gartner, P. Pellegatti, V. F. Di, D. Ferrari, N. Kambham, P. Fisch, J. Finke, M. Idzko, and R. Zeiser. 2010. Graft-versushost disease is enhanced by extracellular ATP activating P2X7R. Nat. Med. 16: 1434-1438.

20. Cao, X., L. P. Li, Q. Wang, Q. Wu, H. H. Hu, M. Zhang, Y. Y. Fang, J. Zhang, S. J. Li, W. C. Xiong, H. C. Yan, Y. B. Gao, J. H. Liu, X. W. Li, L. R. Sun, Y. N. Zeng, X. H. Zhu, and T. M. Gao. 2013. Astrocyte-derived ATP modulates depressive-like behaviors. Nat. Med. 19: 773-777.

21. Akkaya, C., E. Shumilina, D. Bobballa, V. B. Brand, H. Mahmud, F. Lang, and S. M. Huber. 2009. The Plasmodium falciparum-induced anion channel of human erythrocytes is an ATP-release pathway. Pflugers Arch. 457: 1035-1047.

22. Guinea, R., and L. Carrasco. 1995. Requirement for vacuolar proton-ATPase activity during entry of influenza virus into cells. J Virol 69.

23. van Aubel, R. A., P. H. Smeets, J. J. van den Heuvel, and F. G. Russel. 2005. Human organic anion transporter MRP4 (ABCC4) is an efflux pump for the purine end metabolite urate with multiple allosteric substrate binding sites. Am. J. Physiol Renal Physiol 288: F327-F333.

24. Breedveld, P., D. Pluim, G. Cipriani, F. Dahlhaus, M. A. van Eijndhoven, C. J. de Wolf, A. Kuil, J. H. Beijnen, G. L. Scheffer, G. Jansen, P. Borst, and J. H. Schellens. 2007. The effect of low pH on breast cancer resistance protein (ABCG2)-mediated transport of methotrexate, 7-hydroxymethotrexate, methotrexate diglutamate, folic acid, mitoxantrone, topotecan, and resveratrol in in vitro drug transport models. Mol. Pharmacol. 71: 240-249.

25. de Wolf. C., R. Jansen, H. Yamaguchi, H. M. de, W. K. van de, J. Wijnholds, J. Beijnen, and P. Borst. 2008. Contribution of the drug transporter ABCG2 (breast cancer resistance protein) to resistance against anticancer nucleosides. Mol. Cancer Ther. 7: 3092-3102.

26. Schwiebert, E. M., M. E. Egan, and W. B. Guggino. 1998. Assays of dynamics, mechanisms, and regulation of ATP transport and release: implications for study of ABC transporter function. Methods Enzymol. 292: 664-675.

27. Manfredi, G., L. Yang, C. D. Gajewski, and M. Mattiazzi. 2002. Measurements of ATP in mammalian cells. Methods 26: 317-326.

28. Higashi, T., A. Isomoto, I. Tyuma, E. Kakishita, M. Uomoto, and K. Nagai. 1985. Quantitative and continuous analysis of ATP release from blood platelets with firefly luciferase luminescence. Thromb. Haemost. 53: 65-69.

29. Ozvegy, C., T. Litman, G. Szakacs, Z. Nagy, S. Bates, A. Varadi, and B. Sarkadi. 2001. Functional characterization of the human multidrug transporter, ABCG2, expressed in insect cells. Biochem. Biophys. Res. Commun. 285: 111-117.

30. Breedveld, P., N. Zelcer, D. Pluim, O. Sonmezer, M. M. Tibben, J. H. Beijnen, A. H. Schinkel, O. van Tellingen, P. Borst, and J. H. M. Schellens. 2004. Mechanism of the Pharmacokinetic Interaction between Methotrexate and Benzimidazoles: Potential Role for Breast Cancer Resistance Protein in Clinical Drug-Drug Interactions. Cancer Res 64: 5804-5811.

31. Cantrill, C. A., R. A. Skinner, N. J. Rothwell, and J. I. Penny. 2012. An immortalised astrocyte cell line maintains the in vivo phenotype of a primary porcine in vitro blood-brain barrier model. Brain Res. 1479: 17-30.

32. Szakacs, G., J. K. Paterson, J. A. Ludwig, C. Booth-Genthe, and M. M. Gottesman. 2006. Targeting multidrug resistance in cancer. Nat. Rev. Drug Discov. 5: 219-234.

33. Maliepaard, M., G. L. Scheffer, I. F. Faneyte, M. A. van Gastelen, A. C. Pijnenborg, A. H. Schinkel, M. J. van de Vijver, R. J. Scheper, and J. H. Schellens. 2001. Subcellular localization and distribution of the breast cancer resistance protein transporter in normal human tissues. Cancer Res. 61: 3458-3464.
34. Aronica, E., J. A. Gorter, S. Redeker, E. A. van Vliet, M. Ramkema, G. L. Scheffer, R. J. Scheper, d. van, V, S. Leenstra, J. C. Baayen, W. G. Spliet, and D. Troost. 2005. Localization of breast cancer resistance protein (BCRP) in microvessel endothelium of human control and epileptic brain. Epilepsia 46: 849-857.
35. Zhou, S., J. D. Schuetz, K. D. Bunting, A. M. Colapietro, J. Sampath, J. J. Morris, I. Lagutina, G. C. Grosveld, M. Osawa, H. Nakauchi, and B. P. Sorrentino. 2001. The ABC transporter Bcrp1/ABCG2 is expressed in a wide variety of stem cells and is a molecular determinant of the side-population phenotype. Nat. Med. 7: 1028-1034.
36. van de. Ven., J. J. Lindenberg, A. W. Reurs, R. J. Scheper, G. L. Scheffer, and T. D. de Gruijl. 2012. Preferential Langerhans cell differentiation from CD34(+) precursors upon introduction of ABCG2 (BCRP). Immunol. Cell Biol. 90: 206-215.
37. Szatmari, I., G. Vamosi, P. Brazda, B. L. Balint, S. Benko, L. Szeles, V. Jeney, C. Ozvegy-Laczka, A. Szanto, E. Barta, J. Balla, B. Sarkadi, and L. Nagy. 2006. Peroxisome proliferator-activated receptor gamma-regulated ABCG2 expression confers cytoprotection to human dendritic cells. J. Biol. Chem. 281: 23812-23823.
38. Sukowati, C. H., N. Rosso, D. Pascut, B. Anfuso, G. Torre, P. Francalanci, L. S. Croce, and C. Tiribelli. 2012. Gene and functional up-regulation of the BCRP/ABCG2 transporter in hepatocellular carcinoma. BMC. Gastroenterol. 12: 160.
39. Huang, L., Q. Lu, Y. Han, Z. Li, Z. Zhang, and X. Li. 2012. ABCG2/V-ATPase was associated with the drug resistance and tumor metastasis of esophageal squamous cancer cells. Diagn. Pathol. 7: 180.
40. Jin, Y., Z. Q. Bin, H. Qiang, C. Liang, C. Hua, D. Jun, W. A. Dong, and L. Qing. 2009. ABCG2 is related with the grade of glioma and resistance to mitoxantone, a chemotherapeutic drug for glioma. J. Cancer Res. Clin. Oncol. 135: 1369-1376.
41. Kooij, G., R. Backer, J. J. Koning, A. Reijerkerk, H. J. van, S. M. van der Pol, J. Drexhage, A. Schinkel, C. D. Dijkstra, J. M. den Haan, T. B. Geijtenbeek, and H. E. de Vries. 2009. P-glycoprotein acts as an immunomodulator during neuroinflammation. PLoS. ONE. 4: e8212.
42. De Milito. A., R. Canese, M. L. Marino, M. Borghi, M. lero, A. Villa, G. Venturi, F. Lozupone, E. lessi, M. Logozzi, M. P. Della, M. Santinami, M. Rodolfo, F. Podo, L. Rivoltini, and S. Fais. 2010. pH-dependent antitumor activity of proton pump inhibitors against human melanoma is mediated by inhibition of tumor acidity. Int. J. Cancer 127: 207-219.
43. Liu, H. T., A. H. Toychiev, N. Takahashi, R. Z. Sabirov, and Y. Okada. 2008. Maxianion channel as a candidate pathway for osmosensitive ATP release from mouse astrocytes in primary culture. Cell Res. 18: 558-565.
44. Bhatia, P., M. Bernier, M. Sanghvi, R. Moaddel, R. Schwarting, A. Ramamoorthy, and I. W. Wainer. 2012. Breast cancer resistance protein (BCRP/ABCG2) localises to the nucleus in glioblastoma multiforme cells. Xenobiotica 42: 748-755.
45. Dallas, S., D. S. Miller, and R. Bendayan. 2006. Multidrug resistance-associated proteins: expression and function in the central nervous system. Pharmacol. Rev. 58: 140-161.
46. Calatozzolo, C., M. Gelati, E. Ciusani, F. L. Sciacca, B. Pollo, L. Cajola, C. Marras, A. Silvani, L. Vitellaro-Zuccarello, D. Croci, A. Boiardi, and A. Salmaggi. 2005. Expression of drug resistance proteins Pgp, MRP1, MRP3, MRP5 and GST-pi in human glioma. J. Neurooncol. 74: 113-121.
47. Calcinotto, A., P. Filipazzi, M. Grioni, M. lero, M. A. De, A. Ricupito, A. Cova, R. Canese, E. Jachetti, M. Rossetti, V. Huber, G. Parmiani, L. Generoso, M. Santinami, M. Borghi, S. Fais, M. Bellone, and L. Rivoltini. 2012. Modulation of microenvironment acidity reverses anergy in human and murine tumor-infiltrating T lymphocytes. Cancer Res. 72: 2746-2756.
48. Ross, D. D., J. E. Karp, T. T. Chen, and L. A. Doyle. 2000. Expression of breast cancer resistance protein in blast cells from patients with acute leukemia. Blood 96: 365-368.
49. Krishnamurthy, P., D. D. Ross, T. Nakanishi, K. Bailey-Dell, S. Zhou, K. E. Mercer, B. Sarkadi, B. P. Sorrentino, and J. D. Schuetz. 2004. The stem cell marker Bcrp/ABCG2 enhances hypoxic cell survival through interactions with heme. J. Biol. Chem. 279: 24218-24225.
50. Ahmed, F., N. Arseni, H. Glimm, W. Hiddemann, C. Buske, and M. Feuring-Buske. 2008. Constitutive expression of the ATP-binding cassette transporter ABCG2 enhances the growth potential of early human hematopoietic progenitors. Stem Cells 26: 810-818.
51. Luciani, F., M. Spada, A. De Milito, A. Molinari, L. Rivoltini, A. Montinaro, M. Marra, L. Lugini, M. Logozzi, F. Lozupone, C. Federici, E. lessi, G. Parmiani, G. Arancia, F. Belardelli, and S. Fais. 2004. Effect of Proton Pump Inhibitor Pretreatment on Resistance of Solid Tumors to Cytotoxic Drugs. J Natl Cancer Inst 96: 1702-1713.
52. Hjelmeland, A. B., Q. Wu, J. M. Heddleston, G. S. Choudhary, J. MacSwords, J. D. Lathia, R. McLendon, D. Lindner, A. Sloan, and J. N. Rich. 2011. Acidic stress promotes a glioma stem cell phenotype. Cell Death. Differ. 18: 829-840.
53. Aarntzen, E. H., I. J. de Vries, W. J. Lesterhuis, D. Schuurhuis, J. F. Jacobs, K. Bol, G. Schreibelt, R. Mus, J. H. De Wilt, J. B. Haanen, D. Schadendorf, A. Croockewit, W. A. Blokx, M. M. Van Rossum, W. W. Kwok, G. J.

Adema, C. J. Punt, and C. G. Figdor. 2013. Targeting CD4(+) T-helper cells improves the induction of antitumor responses in dendritic cell-based vaccination. Cancer Res. 73: 19-29.
54. Razis, E., P. Selviaridis, S. Labropoulos, J. L. Norris, M. J. Zhu, D. D. Song, T. Kalebic, M. Torrens, A. Kalogera-Fountzila, G. Karkavelas, S. Karanastasi, J. A. Fletcher, and G. Fountzilas. 2009. Phase II study of neoadjuvant imatinib in glioblastoma: evaluation of clinical and molecular effects of the treatment. Clin. Cancer Res. 15: 6258-6266.
55. Fritzell, S., E. Sanden, S. Eberstal, E. Visse, A. Darabi, and P. Siesjo. 2013. Intratumoral temozolomide synergizes with immunotherapy in a T cell-dependent fashion. Cancer Immunol. Immunother.
56. Milano, F., and K. K. Krishnadath. 2008. Novel therapeutic strategies for treating esophageal adenocarcinoma: the potential of dendritic cell immunotherapy and combinatorial regimens. Hum. Immunol. 69: 614-624.
57. Gupta, R. K. 1998. Aluminum compounds as vaccine adjuvants. Adv. Drug Deliv. Rev. 32: 155-172.
58. Li, H., S. Nookala, and F. Re. 2007. Aluminum hydroxide adjuvants activate caspase-1 and induce IL-1 beta and IL-18 release. J. Immunol. 178: 5271-5276.
59. Marchesini, N., M. Vieira, S. Luo, S. N. Moreno, and R. Docampo. 2005. A malaria parasite-encoded vacuolar H(+)-ATPase is targeted to the host erythrocyte. J. Biol. Chem. 280: 36841-36847.
60. van de Hoef, D. L., I. Coppens, T. Holowka, M. C. Ben, O. Branch, and A. Rodriguez. 2013. Plasmodium falciparum-derived uric acid precipitates induce maturation of dendritic cells. PLoS. ONE. 8: e55584.
61. Rey-Ladino, J., A. G. Ross, A. W. Cripps, D. P. McManus, and R. Quinn. 2011. Natural products and the search for novel vaccine adjuvants. Vaccine 29: 6464-6471.
62. Rivera, E., S. Hu, and C. Concha. 2003. Ginseng and aluminium hydroxide act synergistically as vaccine adjuvants. Vaccine 21: 1149-1157.
63. Eichhorn, T., and T. Efferth. 2012. P-glycoprotein and its inhibition in tumors by phytochemicals derived from Chinese herbs. J. Ethnopharmacol. 141: 557-570.
64. Yu, X. Y., S. G. Lin, Z. W. Zhou, X. Chen, J. Liang, P. Q. Liu, W. Duan, B. Chowbay, J. Y. Wen, C. G. Li, and S. F. Zhou. 2007. Role of P-Glycoprotein in the intestinal absorption of tanshinone IIA, a major active ingredient in the root of Salvia miltiorrhiza Bunge. Current Drug Metabolism 8: 325-340.
65. Morris, M. E., and S. Zhang. 2006. Flavonoid-drug interactions: effects of flavonoids on ABC transporters. Life Sci. 78: 2116-2130.
66. Jin, J., S. Shahi, H. K. Kang, H. W. van Veen, and T. P. Fan. 2006. Metabolites of ginsenosides as novel BCRP inhibitors. Biochem. Biophys. Res. Commun. 345: 1308-1314.
67. ten, B. A., M. L. Karsten, M. C. Dieker, J. J. Zwaginga, and S. M. van Ham. 2007. The clinical grade maturation cocktail monophosphoryl lipid A plus IFNgamma generates monocyte-derived dendritic cells with the capacity to migrate and induce Th1 polarization. Vaccine 25: 7145-7152.
68. Jonuleit, H., U. Kuhn, G. Muller, K. Steinbrink, L. Paragnik, E. Schmitt, J. Knop, and A. H. Enk. 1997. Pro-inflammatory cytokines and prostaglandins induce maturation of potent immunostimulatory dendritic cells under fetal calf serum-free conditions. Eur. J. Immunol. 27: 3135-3142.
69. Dauer, M., B. Obermaier, J. Herten, C. Haerle, K. Pohl, S. Rothenfusser, M. Schnurr, S. Endres, and A. Eigler. 2003. Mature dendritic cells derived from human monocytes within 48 hours: a novel strategy for dendritic cell differentiation from blood precursors. J. Immunol. 170: 4069-4076..
70. de Vries, N. A., S. W. Bruggeman, D. Hulsman, H. I. de Vries, J. Zevenhoven, T. Buckle, B. C. Hamans, W. P. Leenders, J. H. Beijnen, L. M. van, A. J. Berns, and T. O. van. 2010. Rapid and robust transgenic high-grade glioma mouse models for therapy intervention studies. Clin. Cancer Res. 16: 3431-3441.

## Claims

**1.** In vitro method for identifying test compounds which are capable of modulating adenosine triphosphate (ATP) release from cells by inhibiting or stimulating the Adenosine triphosphate-Binding Cassette Transporter (ABC Transporter) Breast Cancer Resistance Protein (BCRP; ABCG2)-mediated transport of ATP, comprising the steps of

a) providing a test system comprising said ABC Transporter, wherein this test system includes at least two compartments and wherein the ABC Transporter is able to transport ATP between said at least two compartments,
b) contacting at least one of the compartments of the test system of step a) with a test compound in such a manner and for a time period being sufficient so that the test compound comes in contact with said ABC Transporter,
wherein prior to, during or after the test compound has been brought into contact with at least one of said compartments, ATP is added to at least one of the compartments of the test system from which it can be transported to other compartment(s),

c) determining amount(s) of ATP in at least one of the compartments of the test system to which or from which the ATP is transported,
d) comparing the determined ATP amount(s) of step (c) with ATP amount(s) determined by using the same test system

A) at (a) different concentration(s) of the test compound;
B) in the absence of the test compound; and/or
C) with a reference compound instead of the test compound;
and

e) concluding from the comparison in (d) whether the test compound is capable of modulating ATP release from cells by inhibiting or stimulating said ABC Transporter-mediated transport of ATP,
wherein the pH value of at least the compartment to which the test compound is added is about 7 or lower at the time point at which the test compound is added.

**2.** Method according to claim 1, wherein the ATP added in step b) is labelled ATP, preferably radiolabelled ATP, more preferably [$^{32}$P] γ-ATP.

**3.** Method according to any of the preceding items, wherein the pH value is about 6.8 or less, preferably about 6.5 or less, or in a range of from about 7 to about 4 or about 6.8 to about 4, or in a range of from about 6.5 to about 4, preferably in a range of from about 6.5 to about 5.

**4.** Method according to any of the preceding claims, wherein the method is carried out under hypoxia, wherein preferably said hypoxia is **characterized by** an oxygen content in a range of from 0 to 7% $O_2$, preferably of from 0 to 5% $O_2$, or preferably of from 0.5% to 5% $O_2$.

**5.** Method according to any of the preceding claims, wherein the test compound is classified as inhibitor of ATP release, if the determined amount(s) of ATP is reduced in the at least one compartment to which the ATP is transported, wherein preferably the reduction is 25% or more, and/or if the determined amount(s) of ATP is increased in the at least one compartment from which the ATP is transported, wherein preferably the increase is 25% or more, and wherein the test compound is classified as stimulator of ATP release, if the determined amount(s) of ATP is increased in the at least one compartment to which the ATP is transported, wherein preferably the increase is 25% or more, and/or if the determined amount(s) of the ATP is reduced in the at least one compartment from which the ATP is transported, wherein preferably the reduction is 25% or more.

**6.** Method according to any of the preceding items, wherein the test system of step a) stably expresses, preferably overexpresses, the ABC transporter(s), and/or wherein step b) is carried out by adding the test compound to at least one of said compartments.

**7.** Method according to any of the preceding claims, wherein the substance to be tested represents a herbal compound derived from a plant or from any suitable part(s) of a plant, preferably the herbal compound represents an extract, preferably dried extract, of a plant or of any suitable part(s) of a plant.

**8.** Method according to any of claim 7, wherein the herbal compound is obtained from an (aqueous) alcohol extract, preferably 70%-100% methanol or ethanol.

**9.** Method according to claim 7 or 8, wherein the herbal compound is selected from the group consisting of
salvianolic acids, such as hydrophilic salvianolic acids A, B, C, D, E; preferably salvianolic acids such as salvianolic acid A or B;
lithospermic acids, preferably hydrophilic lithospermic acids, more preferably magnesium lithospermate such as magnesium lithospermate B;
protocatechuic acids, preferably hydrophilic protocatechuic acids such as rosmarinic acid, also preferred protocatechualdehyde;
tanshinones, preferably diterpene tanshiones, preferably lipophilic diterpene tanshinones such as cryptotanshinone, tanshinone I, tanshinone IIB, dihydrotanshinone;
alkaloids, preferably hydrophilic alkaloids, such as the indole alkaloids indirubin and indirubin derivatives, or leonurine or stachydrine; and
triterpenes, preferably lipophilic triterpenes, preferably quassinoids;

preferably, the herbal compound is selected from the group consisting of salvianolic acid B, magnesium lithospermate B, and indirubin.

**10.** Combination of compounds selected from the group consisting of

a)

(i) one or more anti cancer drugs, and
(ii) a prazole selected from the group consisting of omeprazole, lansoprazole, dexlansoprazole, rabeprazole, esomeprazole, tenatoprazole, and, optionally,
(iii) compound(s) being capable of modulating adenosine triphosphate (ATP) release from cells by inhibiting or stimulating the Adenosine triphosphate-Binding Cassette Transporter (ABC Transporter) Breast Cancer Resistance Protein (BCRP; ABCG2)-mediated transport of ATP;

b)

(i) one or more anti cancer drugs, and
(ii) compound(s) being capable of modulating adenosine triphosphate (ATP) release from cells by inhibiting or stimulating the Adenosine triphosphate-Binding Cassette Transporter (ABC Transporter) Breast Cancer Resistance Protein (BCRP; ABCG2)-mediated transport of ATP;

c)

(i) one or more anti cancer drugs;
(ii) ATP; and, optionally,
(iii) compound(s) being capable of modulating adenosine triphosphate (ATP) release from cells by inhibiting or stimulating the Adenosine triphosphate-Binding Cassette Transporter (ABC Transporter) Breast Cancer Resistance Protein (BCRP; ABCG2)-mediated transport of ATP;

d)

(i) methotrexate and/or imatinib, and
(ii) compound(s) being capable of modulating adenosine triphosphate (ATP) release from cells by inhibiting or stimulating the Adenosine triphosphate-Binding Cassette Transporter (ABC Transporter) Breast Cancer Resistance Protein (BCRP; ABCG2)-mediated transport of ATP, and, optionally,
(iii) a prazole selected from the group consisting of omeprazole, lansoprazole, dexlansoprazole, rabeprazole, esomeprazole and tenatoprazole;

e)

(i) ATP, and
(ii) compound(s) being capable of modulating adenosine triphosphate (ATP) release from cells by inhibiting or stimulating the Adenosine triphosphate-Binding Cassette Transporter (ABC Transporter) Breast Cancer Resistance Protein (BCRP; ABCG2)-mediated transport of ATP; or
(ii) a prazole selected from the group consisting of omeprazole, lansoprazole, dexlansoprazole, rabeprazole, esomeprazole and tenatoprazole;

f)

(i) dendritic cell vaccines, and
(ii) compound(s) being capable of modulating adenosine triphosphate (ATP) release from cells by inhibiting or stimulating the Adenosine triphosphate-Binding Cassette Transporter (ABC Transporter) Breast Cancer Resistance Protein (BCRP; ABCG2)-mediated transport of ATP; and

g)

(i) dendritic cell vaccines, and
(ii) proton pump inhibitors

for use in the treatment of cancer occurring in tissue where the respective ABC Transporter Modulator whose transport of ATP is inhibited or stimulated by said compound(s) is expressed,
provided that the compounds of (i), (ii), and where present, (iii), are different, respectively, in all subitems above.

**11.** Combination for use in the treatment of brain tumors and/or brain metastases, preferably gliomas, more preferably high grade glioblastoma multiforme, according to claim 10.

**12.** Combination for use in the treatment of advanced pancreatic ductal adenocarcinoma or high grade hepatocellular carcinoma, according to claim 10 e), f), and/or g).

**13.** Combination of compounds selected from the group consisting of

A)

(i) antimalarial agent capable of modulating adenosine triphosphate (ATP) release from cells by inhibiting the Adenosine triphosphate-Binding Cassette Transporter (ABC Transporter) Breast Cancer Resistance Protein (BCRP; ABCG2)-mediated transport of ATP, and
(ii) proton pump inhibitors, and, optionally,
(iii) ATP;

B)

(i) antimalarial agent capable of modulating adenosine triphosphate (ATP) release from cells by inhibiting the Adenosine triphosphate-Binding Cassette Transporter (ABC Transporter) Breast Cancer Resistance Protein (BCRP; ABCG2)-mediated transport of ATP, and
(ii) compound(s) being capable of modulating adenosine triphosphate (ATP) release from cells by inhibiting the Adenosine triphosphate-Binding Cassette Transporter (ABC Transporter) Breast Cancer Resistance Protein (BCRP; ABCG2)-mediated transport of ATP, and a compound from the group of tanshinones; and, optionally,
(iii) a proton pump inhibitor, and, optionally, (iv) ATP;

C)

(i) antimalarial agent being capable of modulating adenosine triphosphate (ATP) release from cells by inhibiting the Adenosine triphosphate-Binding Cassette Transporter (ABC Transporter) Breast Cancer Resistance Protein (BCRP; ABCG2)-mediated transport of ATP, and
(ii) indirubin or derivatives thereof, and, optionally,
(iii) a proton pump inhibitor, and, optionally,
(iii) ATP;

D)

(i) antimalarial agent being capable of modulating adenosine triphosphate (ATP) release from cells by inhibiting the Adenosine triphosphate-Binding Cassette Transporter (ABC Transporter) Breast Cancer Resistance Protein (BCRP; ABCG2)-mediated transport of ATP, and
(ii) ATP;

E)

(i) dendritic cell vaccines, and
(ii) compound(s) being capable of modulating adenosine triphosphate (ATP) release from cells by inhibiting the Adenosine triphosphate-Binding Cassette Transporter (ABC Transporter) Breast Cancer Resistance Protein (BCRP; ABCG2)-mediated transport of ATP; and

F)

(i) dendritic cell vaccines, and

(ii) proton pump inhibitors;
for use in the treatment of malaria,
provided that the compounds of (i), (ii), and where present, (iii), are different, respectively, in all subitems above.

**14.** Pharmaceutical composition comprising

(a) an antigen,
(b) a compound being capable of modulating adenosine triphosphate (ATP) release from cells by stimulating the Adenosine triphosphate-Binding Cassette Transporter (ABC Transporter) Breast Cancer Resistance Protein (BCRP; ABCG2)-, MRP1-, MRP2-, MRP3-, MRP4-, MRP5-, and/or MRP6-mediated transport of ATP,
wherein said composition is suitable for eliciting an immune response directed against the antigen (a).

**15.** Compound capable of inhibiting ATP release as defined in any of claims 1 to 9, for use in DC maturation.

**Fig. 1**

1A)

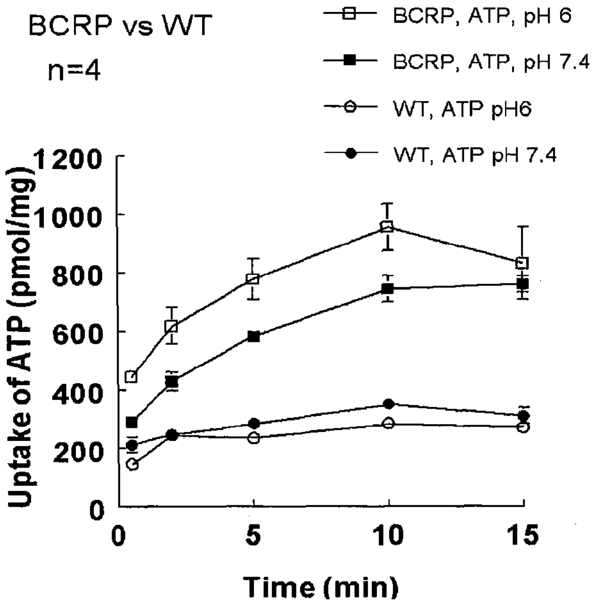
BCRP vs WT
n=4
BCRP, ATP, pH 6
BCRP, ATP, pH 7.4
WT, ATP pH6
WT, ATP pH 7.4
Uptake of ATP (pmol/mg)
1200
1000
800
600
400
200
0
0
5
10
15
Time (min)

1B)

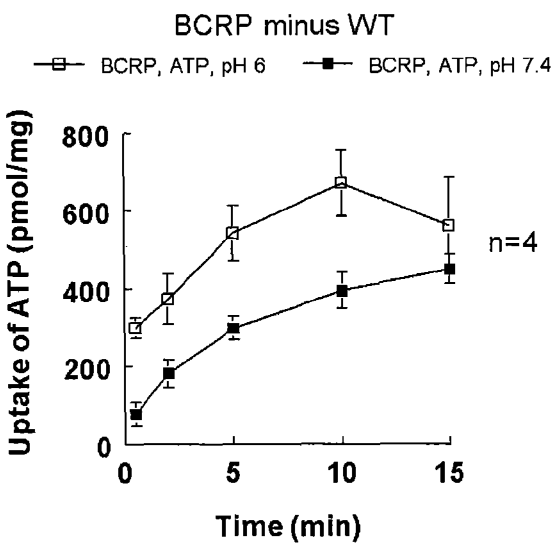
BCRP minus WT
BCRP, ATP, pH 6
BCRP, ATP, pH 7.4
Uptake of ATP (pmol/mg)
800
600
400
200
0
0
5
10
15
n=4
Time (min)

**Fig. 2**

2A)

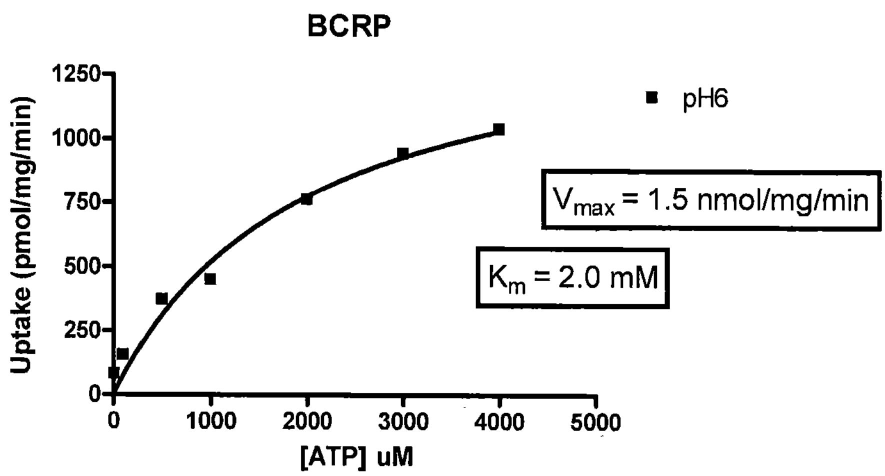
BCRP
Uptake (pmol/mg/min)
1250
1000
750
500
250
0
0
1000
2000
3000
4000
5000
[ATP] uM
pH6
$V_{max}$ = 1.5 nmol/mg/min
$K_m$ = 2.0 mM

2B)

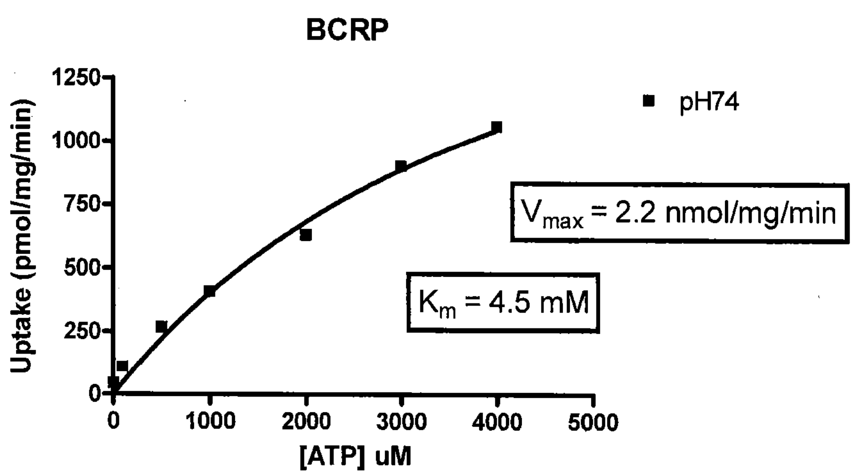
BCRP
Uptake (pmol/mg/min)
1250
1000
750
500
250
0
0
1000
2000
3000
4000
5000
[ATP] uM
pH74
$V_{max}$ = 2.2 nmol/mg/min
$K_m$ = 4.5 mM

Fig. 3

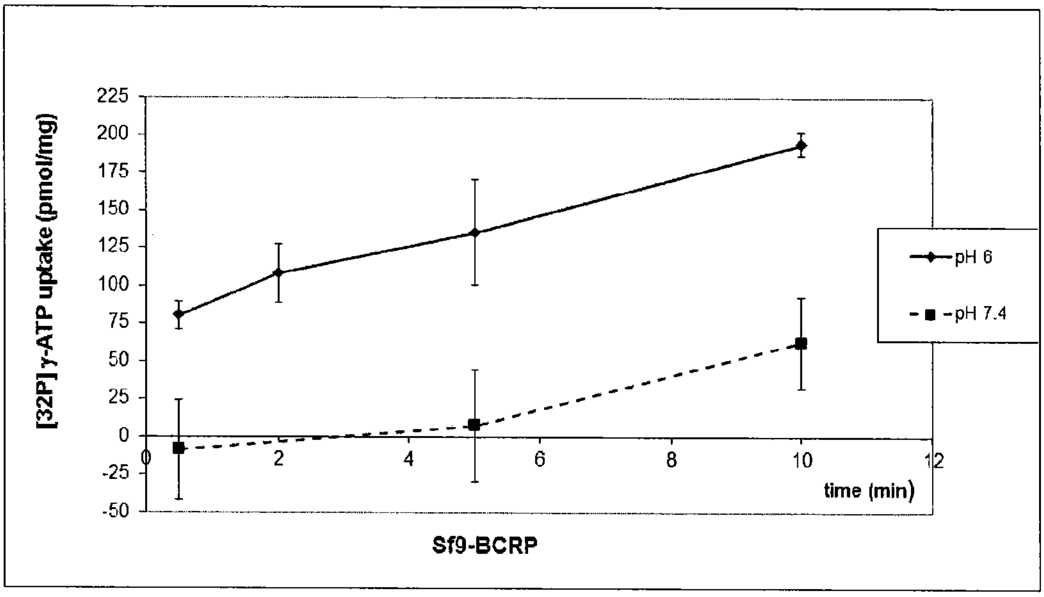
[32P] γ-ATP uptake (pmol/mg)
225
200
175
150
125
100
75
50
25
0
-25
-50
0
2
4
6
8
10
12
time (min)
pH 6
pH 7.4
Sf9-BCRP

Fig. 4

4A)

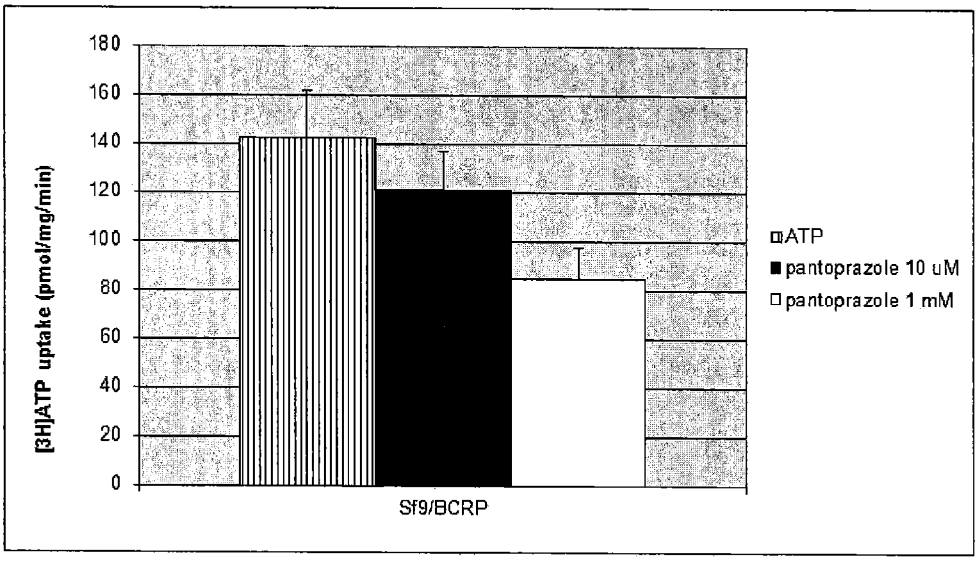
180
160
140
120
100
80
60
40
20
0
[3H]ATP uptake (pmol/mg/min)
Sf9/BCRP
ATP
pantoprazole 10 uM
pantoprazole 1 mM

4B)

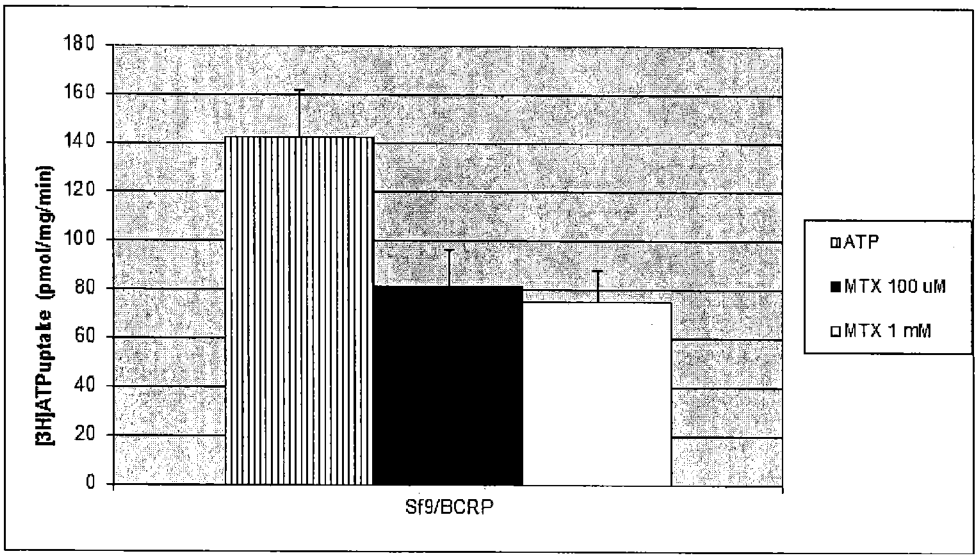
180
160
140
120
100
80
60
40
20
0
[3H]ATPuptake (pmol/mg/min)
Sf9/BCRP
ATP
MTX 100 uM
MTX 1 mM

Fig. 5

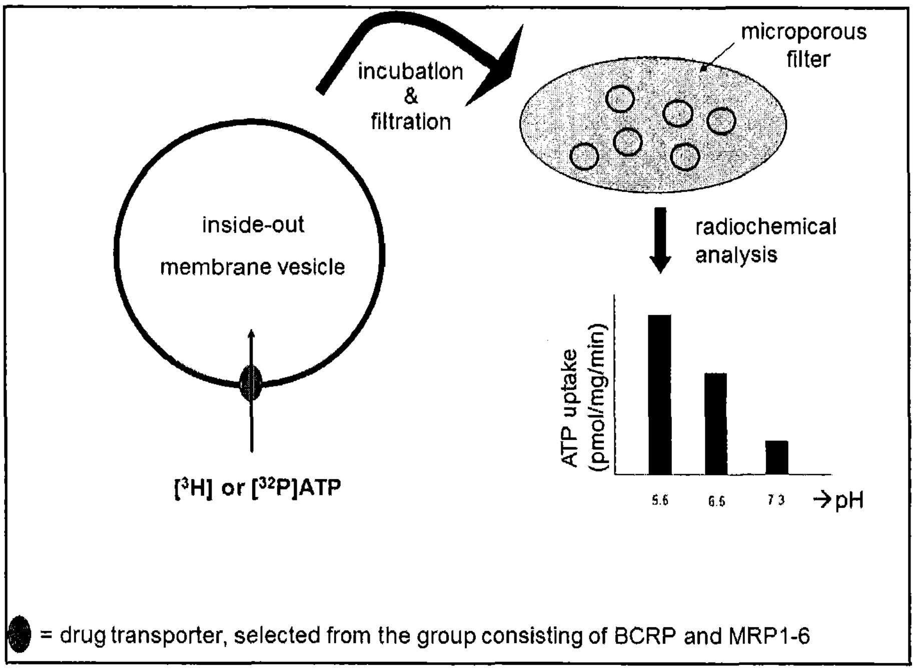
incubation
&
filtration
microporous
filter
inside-out
membrane vesicle
radiochemical
analysis
ATP uptake
(pmol/mg/min)
5.6
6.5
7.3
→pH
[3H] or [32P]ATP
= drug transporter, selected from the group consisting of BCRP and MRP1-6

Fig. 6

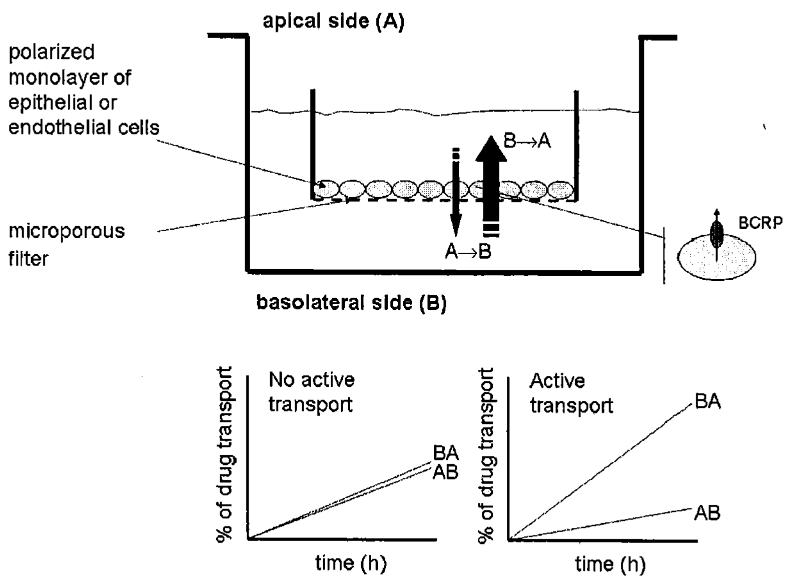
apical side (A)
polarized monolayer of epithelial or endothelial cells
microporous filter
B→A
A→B
BCRP
basolateral side (B)
% of drug transport
No active transport
BA
AB
time (h)
% of drug transport
Active transport
BA
AB
time (h)

Fig. 7

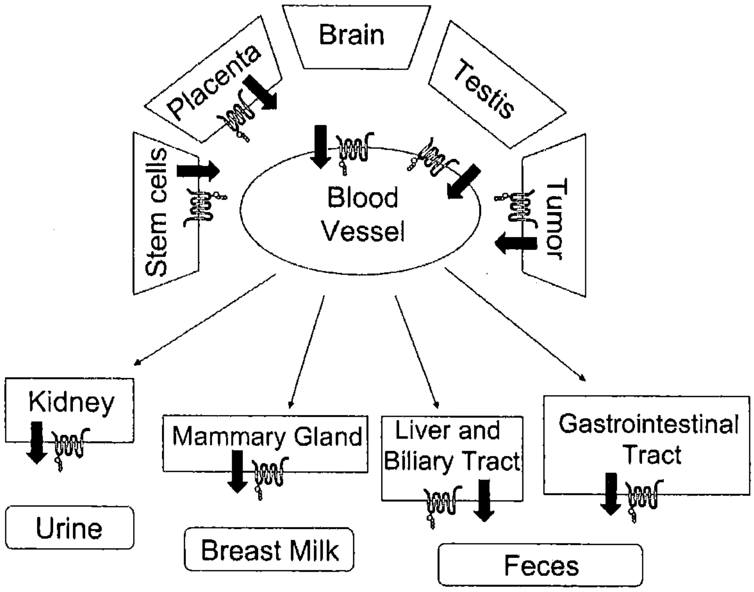
Brain
Placenta
Testis
Stem cells
Blood
Vessel
Tumor
Kidney
Mammary Gland
Liver and
Biliary Tract
Gastrointestinal
Tract
Urine
Breast Milk
Feces

**Fig. 8**

## Table 1

| Type of pump | Cellular localization | Function |
|---|---|---|
| $H^+$-ATPase | Cytoplasm plasmamembrane and acidic organelles | Acidification of extracellular microenvironment and endolysosomal compartment |
| $Na^+/H^+$-ATPase | Cytoplasm plasmamembrane | Alcalinization of cytosol and acidification of extracellular microenvironment |
| MCT1 ($H^+$ / Lactate symporters) | Cytoplasm plasmamembrane | Elimination of lactate as glucose catabolism product and acidification of extracellular milieu |
| Carbonic anhydrase | Cytoplasm plasmamembrane | Regulation of intracellular pH and pH gradients |
| $H^+/K^+$-ATPase | Gastric parietal cells | Regulation of extracellular pH |

**Fig. 9**

**Table 2**

| **Isoform** | **CNS expression** | | | | |
|---|---|---|---|---|---|
| | **WB** | **BBB** | **CP** | **MG** | **AST** |
| MRP1 | H,M,R | H,M,R | H,M,R | R | H,M,R |
| MRP2 | R | H,R | M,R | R | H,R |
| MRP3 | R | | M,R | R | R |
| MRP4 | H,M,R | H,M | H,M,R | R | H,R |
| MRP5 | H,M,R | H,M | M,R | R | H,R |
| MRP6 | H,M | | M,R | R | H,R |

WB, whole brain homogenate; BBB, blood-brain-barrier; CP, choroid plexus; MG, microglia; AST, astrocytes,H, human; M, mouse; R, rat.

## Fig. 10

## Fig. 10 A

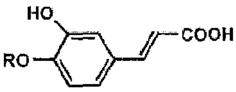

1.caffeic acid R=H
2.isoferulic acid R=$CH_3$

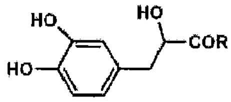

3.Danshensu R=OH
4.3-(3,4-dihydroxyphenyl) lactamide R=$NH_2$

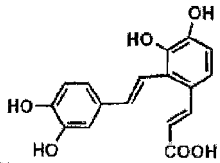

10.salvianolic acid F

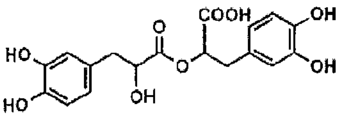

9.salvianic acid C

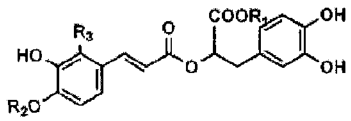

5.rosmarinic acid R1=R2=R3=H
6.methyl rosmarinate R1=$CH_3$,R2=R3=H
7.Salviaflaside R1=H,R2=glc,R3=H
8.salvianolic acid D R1=R2=H,R3=$CH_2COOH$

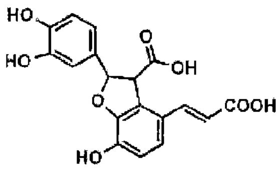

11.prolithospermic acid

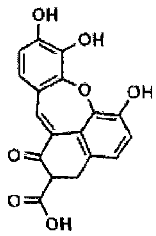

12.Salvianolic acid G

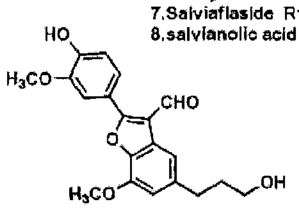

13.Salvinal

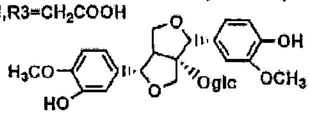

14.1-hydroxy-pinoresinol-1-O-β-D-glucoside

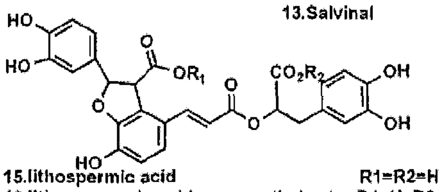

15.lithospermic acid R1=R2=H
16.litherospermic acid monomethyl ester R1=H, R2=$CH_3$
17.litherospermic acid dimethyl ester R1=R2=$CH_3$
18.ethyl lithospermate R1= $C_2H_5$, R2=H

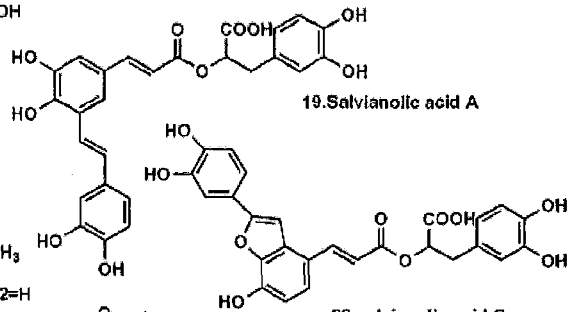

19.Salvianolic acid A

20.salvianolic acid C

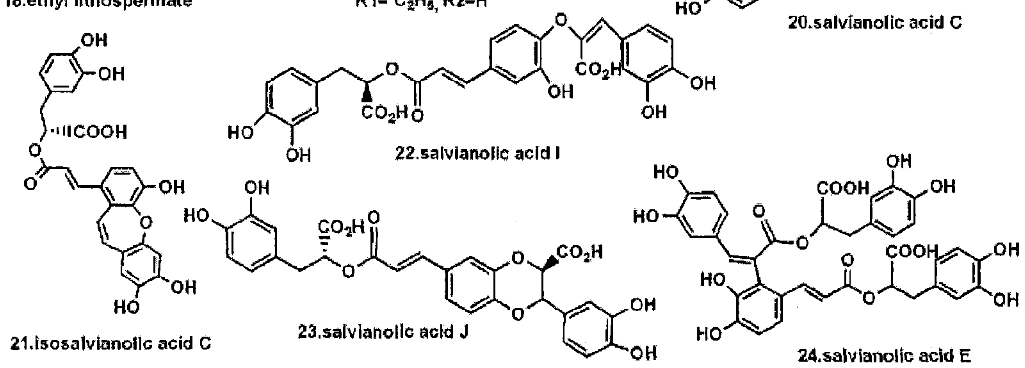

22.salvianolic acid I

21.isosalvianolic acid C

23.salvianolic acid J

24.salvianolic acid E

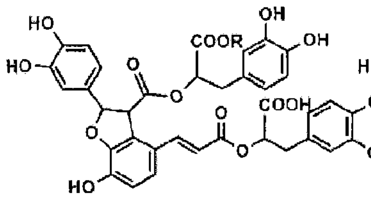

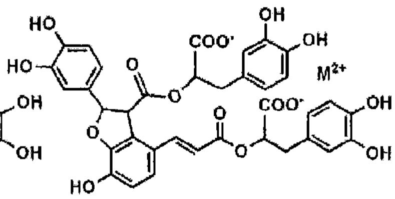

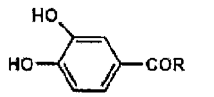

29.protocatechuic acid R=OH
30.protocatechualdehyde R=H

25.salvianolic acid B R=H
26.ethyl lithospermate B R=$C_2H_5$

27.magnesium lithospermate B $M^{2+}$=$Mg^{2+}$
28.ammonium-potassium lithospermate B $M^{2+}$=$NH_4^+$+$K^+$

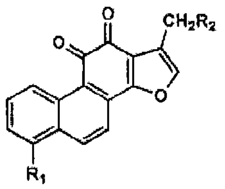

31.tanshinone I R1=$CH_3$,R2=H
32.Tanshinol A R1=$CH_2OH$,R2=H
33.Przewaquinone B R1=$CH_3$,R2=OH
34.Formyltanshinone R1=CHO,R2=H

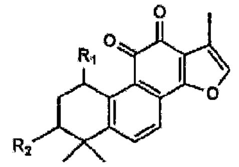

35.tanshinone $II_A$ R1=R2=H
36.hydroxytanshinone $II_A$ ,R1=OH,R2=H
37.3-hydroxytanshinone $II_A$R1=H,R2=OH

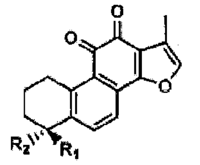

38.tanshinone IIB R1=$CH_2OH$,R2=$CH_3$
39.methyl tanshinonate R1=$CO_2CH_3$,R2=$CH_3$
40.Tanshinaldehyde R1=$CH_3$, R2=CHO
41.tanshindiol A R1=OH, R2=$CH_2OH$

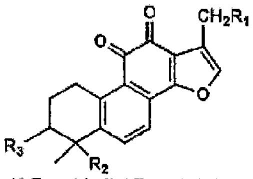

42.Tanshindiol B R1=H, R2=R3=OH
43.Przewaquinone A R1=OH, R2=$CH_3$,R3=H
44.Przewaquinone C R1=H, R2=OH,R3=H

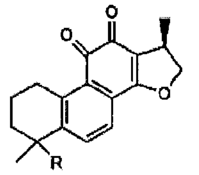

45.Cryptotanshinone R=$CH_3$
46.methyl dihydronortanshinonate R=$COOCH_3$

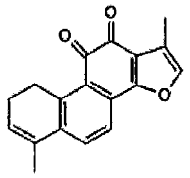

47.1,2-dihydrotanshinquinone

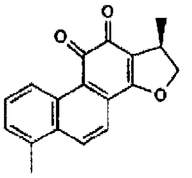

48.dihydrotanshinone I

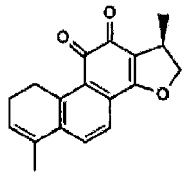

49.tetrahydrotanshinone

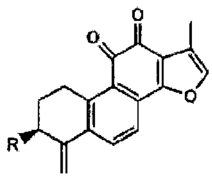

50.methylenetanshinquinone R=H
51.3-hydroxymethylenetanshinquinone R=OH

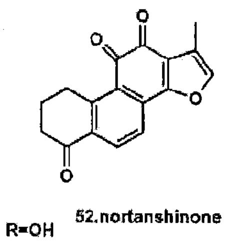

52.nortanshinone

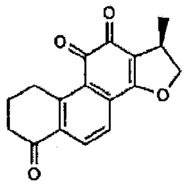

53.Dihydronortanshinone

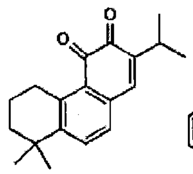

54.miltirone

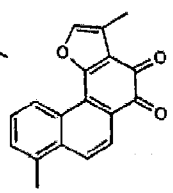

55.Isotanshinone II

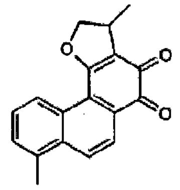

56.dihydroisotanshinone II

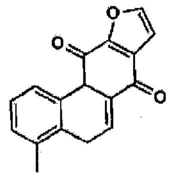

57.Isototanshinone

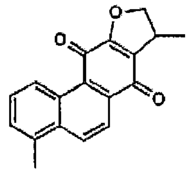

58.dihydroisototanshinone I

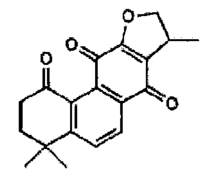

59.1-ketoisocryptotanshinone

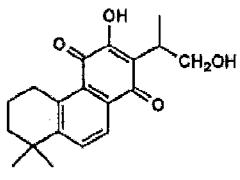

60.Neocryptotanshinone

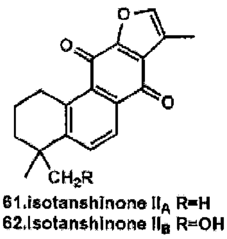

61.isotanshinone $II_A$ R=H
62.Isotanshinone $II_B$ R=OH

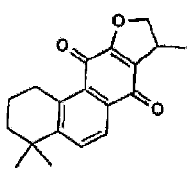

63.Isocryptotanshinone II

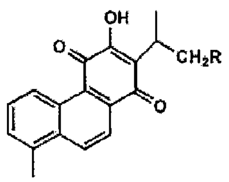

64.Danshenxinkun A R=OH
65.Danshenxinkun B R=H

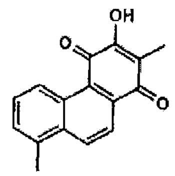

66.Danshenxinkun C

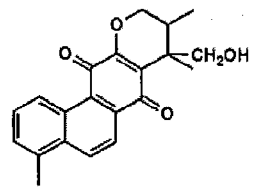

67.Danshenxinkun D

**Fig. 10 B**

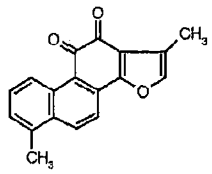

Tanshinone I

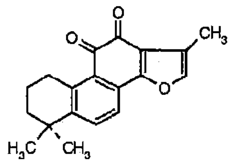

Tanshinone IIA

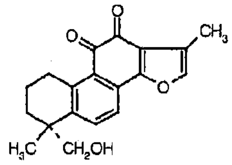

Tanshinone IIB

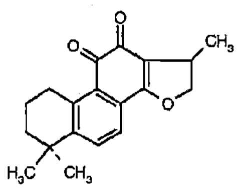

Cryptotanshinone

**Fig. 10 C**

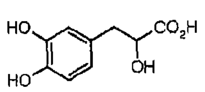

Danshensu
(Salvianic acid A)

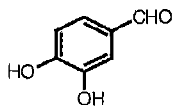

Protocatechuic aldehyde

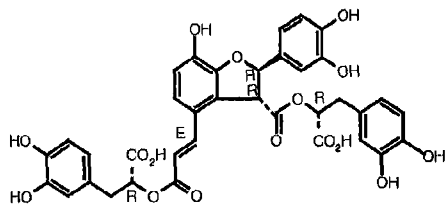

Salvianolic acid B

**Fig. 10 D**

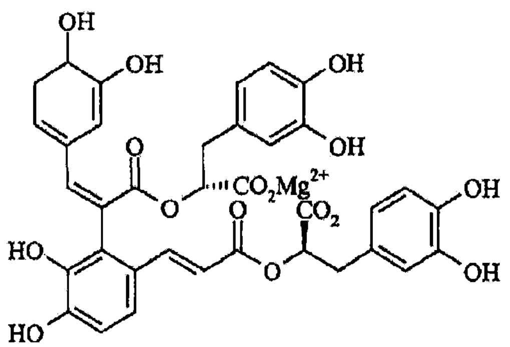

Fig. 10 E

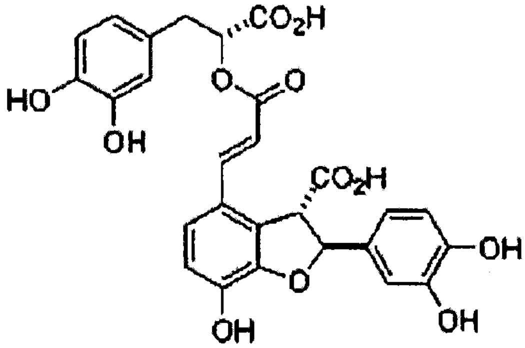

Fig. 10 F

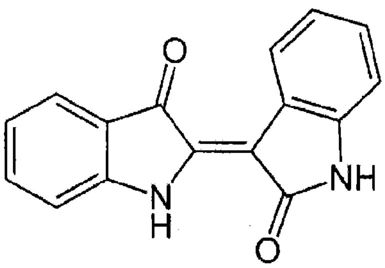

Europäisches Patentamt
European Patent Office
Office européen des brevets

# EUROPEAN SEARCH REPORT

Application Number
EP 13 17 8291

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BOKESCH H R ET AL: "Inhibition of ABCG2-mediated drug efflux by naphthopyrones from marine crinoids", BIOORGANIC & MEDICINAL CHEMISTRY LETTERS, PERGAMON, vol. 20, no. 13, 1 July 2010 (2010-07-01), pages 3848-3850, XP027083676, ISSN: 0960-894X [retrieved on 2010-05-21] * the whole document * * In particular: Title and Abstract. * ----- | 1-9 | INV. G01N33/574 |
| A | YAMAGATA TETSUO ET AL: "Improvement of the oral drug absorption of topotecan through the inhibition of intestinal xenobiotic efflux transporter, breast cancer resistance protein, by excipients", DRUG METABOLISM AND DISPOSITION, NOT AMERICAN SOCIETY FOR PHARMACOLOGY AND EXPERIMENTAL THERAPEUTICS, ETC., vol. 35, no. 7, 1 July 2007 (2007-07-01), pages 1142-1148, XP002711274, ISSN: 0090-9556 * the whole document * * In particular: Title; Abstract; Materials and methods section; Results section first three paragraphs; Fig. 1-5. * ----- | 1-9 | TECHNICAL FIELDS SEARCHED (IPC) G01N |
| | -/-- | | |

The present search report has been drawn up for all claims

| Place of search | Date of completion of the search | Examiner |
|---|---|---|
| The Hague | 4 December 2013 | C.F. Angioni |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 8291

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | BREEDVELD P ET AL: "The Effect of Bcrp1 (Abcg2) on the In vivo Pharmacokinetics and Brain Penetration of Imatinib Mesylate (Gleevec): Implications for the Use of Breast Cancer Resistance Protein and P-Glycoprotein Inhibitors to Enable the Brain Penetration of Imatinib in Patients", CANCER RESEARCH, AMERICAN ASSOCIATION FOR CANCER RESEARCH, US, vol. 65, no. 7, 1 April 2005 (2005-04-01), pages 2577-2582, XP007901255, ISSN: 0008-5472, DOI: 10.1158/0008-5472.CAN-04-2416 * the whole document * * In particular: Title; Abstract; Fig. 1-4. * ----- | 1-9 | |
| A | MAO Q ET AL: "Functional expression of the human breast cancer resistance protein in Pichia pastoris", BIOCHEMICAL AND BIOPHYSICAL RESEARCH COMMUNICATIONS, ACADEMIC PRESS INC. ORLANDO, FL, US, vol. 320, no. 3, 30 July 2004 (2004-07-30), pages 730-737, XP004518011, ISSN: 0006-291X, DOI: 10.1016/J.BBRC.2004.06.012 * the whole document * * In particular: Materials and methods section. * ----- -/-- | 1-9 | TECHNICAL FIELDS SEARCHED (IPC) |

The present search report has been drawn up for all claims

3

| Place of search | Date of completion of the search | Examiner |
|---|---|---|
| The Hague | 4 December 2013 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 8291

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | "An ATP transporter is required for protein translocation into the yeast endoplasmic reticulum.", , 1 January 1993 (1993-01-01), XP55076499, Retrieved from the Internet: URL:http://www.ncbi.nlm.nih.gov/pmc/articles/PMC413250/pdf/emboj00074-0283.pdf [retrieved on 2013-08-26] * the whole document * * In particular: Materials and methods section. * ----- | 1-9 | |
| X | PAULINE BREEDVELD ET AL: "Mechanism of the Pharmacokinetic Interaction between Methotrexate and Benzimidazoles: Potential Role for Breast Cancer Resistance Protein in Clinical Drug-Drug Interactions", CANCER RESEARCH, 1 January 2004 (2004-01-01), pages 5804-5811, XP55091325, * the whole document * * In particular: Title; Abstract; Materials and methods section; p. 5807-5808, Section: Effect of Pantoprazole on the Bcrp1-mediated clearance of MTX in mice. * ----- | 10-12 | TECHNICAL FIELDS SEARCHED (IPC) |

-/--

The present search report has been drawn up for all claims

3

| Place of search | Date of completion of the search | Examiner |
|---|---|---|
| The Hague | 4 December 2013 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 8291

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | OOSTENDORP R L ET AL: "The biological and clinical role of drug transporters at the intestinal barrier", CANCER TREATMENT REVIEWS, SAUNDERS, US, vol. 35, no. 2, 1 April 2009 (2009-04-01), pages 137-147, XP025969473, ISSN: 0305-7372, DOI: 10.1016/J.CTRV.2008.09.004 [retrieved on 2008-11-04] * the whole document * * In particular: Title; Summary; p. 141-142, section: ABCG2-mediated secretory transport. * ----- | 10-12 | |
| X | JULIE SCHOLLER ET AL: "Molecular pharmacokinetic determinants of anticancer kinase inhibitors in humans", ONCOLOGY REVIEWS, SPRINGER MILAN, MILAN, vol. 5, no. 2, 22 February 2011 (2011-02-22), pages 77-92, XP019915032, ISSN: 1970-5565, DOI: 10.1007/S12156-011-0072-5 * the whole document * * In particular: Title; Abstract; p. 87, section on Imatinib; p. 89, section on Warfarin. * ----- | 10-12 | TECHNICAL FIELDS SEARCHED (IPC) |
| A | EFFERTH T ET AL: "Molecular modes of action of artesunate in tumor cell lines", MOLECULAR PHARMACOLOGY, AMERICAN SOCIETY FOR PHARMACOLOGY AND EXPERIMENTAL THERAPEUTICS, US, vol. 64, no. 2, 1 August 2003 (2003-08-01), pages 382-394, XP002324362, ISSN: 0026-895X, DOI: 10.1124/MOL.64.2.382 * the whole document * ----- | 13 | |
| | -/-- | | |

The present search report has been drawn up for all claims

3

| Place of search | Date of completion of the search | Examiner |
|---|---|---|
| The Hague | 4 December 2013 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 13 17 8291

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | "Molecular modes of action of antimalarial artemisinin derivatives as novel anticancer drugs", EUROPEAN JOURNAL OF CANCER, PERGAMON PRESS, OXFORD, GB, vol. 38, 1 November 2002 (2002-11-01), page S99, XP004403771, ISSN: 0959-8049 * the whole document * ----- | 13 | |
| X | KANENO RAMON ET AL: "Chemomodulation of human dendritic cell function by antineoplastic agents in low noncytotoxic concentrations", JOURNAL OF TRANSLATIONAL MEDICINE, BIOMED CENTRAL, LONDON, GB, vol. 7, no. 1, 10 July 2009 (2009-07-10), page 58, XP021059145, ISSN: 1479-5876, DOI: 10.1186/1479-5876-7-58 * the whole document * * In particular: Title; Abstract; last paragraph of introduction; Materials and methods section; Discussion section. * ----- | 15 | |

TECHNICAL FIELDS SEARCHED (IPC)

The present search report has been drawn up for all claims

3

| Place of search | Date of completion of the search | Examiner |
|---|---|---|
| The Hague | 4 December 2013 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 13 17 8291

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION
## SHEET B

Application Number
EP 13 17 8291

The Search Division considers that the present European patent application does not comply with the requirements of unity of invention and relates to several inventions or groups of inventions, namely:

1. claims: 1-9

An in vitro method as defined in claim 1.

---

2. claims: 10-12

A combination of compounds as defined in claim 10 for the treatment of cancer.

---

3. claim: 13

A combination of compounds as defined in claim 13 for the treatment of malaria

---

4. claim: 14

A pharmaceutical composition as defined in claim 14.

---

5. claim: 15

A compound as defined in claim 15.

---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005112947 A


### Non-patent literature cited in the description


- **BENSKY ; CLAVER ; STÖGER.** Chinese Herbal Medicine. Materia Medica. Eastland Press, 1189-1217
- *Herb and Formula Index,* 1219-1242
- *Pinyin-Pharmaceutical cross reference,* 1243-1251
- *English-Pharmaceutical Cross Reference,* 1253-1257
- *Japanes-Pharmaceutical Cross Reference,* 1259-1263
- *Korean Cross Reference,* 1265-1279
- **BREEDVELD et al.** *Mol Pharmacol,* 2007
- **ROBEY et al.** *Curr Pharm Biotechnol,* 2011
- **BURNSTOCK, G.** Purinergic signalling. *Br. J. Pharmacol.,* 2006, vol. 147 (1), S172-S181
- **LDZKO, M., H. HAMMAD ; M. VAN NIMWEGEN ; M. KOOL ; M. A. WILLART ; F. MUSKENS ; H. C. HOOGSTEDEN ; W. LUTTMANN ; D. FERRARI ; F. DI VIRGILIO ; J. C. VIRCHOW, JR.** Extracellular ATP triggers and maintains asthmatic airway inflammation by activating dendritic cells. *Nat. Med.,* 2007, vol. 13, 913-919
- **TANNOCK, I. F. ; D. ROTIN.** Acid pH in tumors and its potential for therapeutic exploitation. *Cancer Res.,* 1989, vol. 49, 4373-4384
- **MARTINS, I. ; A. TESNIERE ; O. KEPP ; M. MICHAUD ; F. SCHLEMMER ; L. SENOVILLA ; C. SEROR ; D. METIVIER ; J. L. PERFETTINI ; L. ZITVOGEL.** Chemotherapy induces ATP release from tumor cells. *Cell Cycle,* 2009, vol. 8, 3723-3728
- **DUEWELL, P. ; H. KONO ; K. J. RAYNER ; C. M. SIROIS ; G. VLADIMER ; F. G. BAUERNFEIND ; G. S. ABELA ; L. FRANCHI ; G. NUNEZ ; M. SCHNURR.** NLRP3 inflammasomes are required for atherogenesis and activated by cholesterol crystals. *Nature,* 2010, vol. 464, 1357-1361
- **LOHMAN, A. W. ; M. BILLAUD ; E. ISAKSON.** Mechanisms of ATP release and signalling in the blood vessel wall. *Cardiovasc. Res.,* 2012, vol. 95, 269-280
- **TU, J. ; G. LE ; H. J. BALLARD.** Involvement of the cystic fibrosis transmembrane conductance regulator in the acidosis-induced efflux of ATP from rat skeletal muscle. *J. Physiol,* 2010, vol. 588, 4563-4578
- **SCHWIEBERT, L. M. ; W. C. RICE ; B. A. KUDLOW ; A. L. TAYLOR ; E. M. SCHWIEBERT.** Extracellular ATP signaling and P2X nucleotide receptors in monolayers of primary human vascular endothelial cells. *Am. J. Physiol Cell Physiol,* 2002, vol. 282, C289-C301
- **QU, Y., S. MISAGHI ; K. NEWTON ; L. L. GILMOUR ; S. LOUIE ; J. E. CUPP ; G. R. DUBYAK ; D. HACKOS ; V. M. DIXIT.** Pannexin-1 is required for ATP release during apoptosis but not for inflammasome activation. *J. Immunol.,* 2011, vol. 186, 6553-6561
- **BRAUNSTEIN, G. M. ; R. M. ROMAN ; J. P. CLANCY ; B. A. KUDLOW ; A. L. TAYLOR ; V. G. SHYLONSKY ; B. JOVOV ; K. PETER ; T. JILLING ; I. I. ISMAILOV.** Cystic fibrosis transmembrane conductance regulator facilitates ATP release by stimulating a separate ATP release channel for autocrine control of cell volume regulation. *J. Biol. Chem.,* 2001, vol. 276, 6621-6630
- **REDDY, M. M. ; P. M. QUINTON ; C. HAWS ; J. J. WINE ; R. GRYGORCZYK ; J. A. TABCHARANI ; J. W. HANRAHAN ; K. L. GUNDERSON ; R. R. KOPITO.** Failure of the cystic fibrosis transmembrane conductance regulator to conduct ATP. *Science,* 1996, vol. 271, 1876-1879
- **LI, C. ; M. RAMJEESINGH ; C. E. BEAR.** Purified cystic fibrosis transmembrane conductance regulator (CFTR) does not function as an ATP channel. *J. Biol. Chem.,* 1996, vol. 271, 11623-11626
- **ABRAHAM, E. H. ; B. SHRIVASTAV ; A. Y. SALIKHOVA ; K. M. STERLING ; N. JOHNSTON ; G. GUIDOTTI ; S. SCALA ; T. LITMAN ; K. C. CHAN ; R. J. ARCECI.** Cellular and biophysical evidence for interactions between adenosine triphosphate and P-glycoprotein substrates: functional implications for adenosine triphosphate/drug cotransport in P-glycoprotein overexpressing tumor cells and in P-glycoprotein low-level expressing erythrocytes. *Blood Cells Mol. Dis.,* 2001, vol. 27, 181-200

- **OSAWA, R. ; K. L. WILLIAMS ; N. SINGH.** The inflammasome regulatory pathway and infections: role in pathophysiology and clinical implications. *J. Infect.,* 2011, vol. 62, 119-129
- **GHIRINGHELLI, F. ; L. APETOH ; A. TESNIERE ; L. AYMERIC ; Y. MA ; C. ORTIZ ; K. VERMAELEN ; T. PANARETAKIS ; G. MIGNOT ; E. ULLRICH.** Activation of the NLRP3 inflammasome in dendritic cells induces IL-1beta-dependent adaptive immunity against tumors. *Nat. Med.,* 2009, vol. 15, 1170-1178
- **TRABANELLI, S. ; D. OCADLIKOVA ; S. GULINELLI ; A. CURTI ; V. SALVESTRINI ; R. P. VIEIRA ; M. LDZKO ; V. F. DI ; D. FERRARI ; R. M. LEMOLI.** Extracellular ATP exerts opposite effects on activated and regulatory CD4+ T cells via purinergic P2 receptor activation. *J. Immunol.,* 2012, vol. 189, 1303-1310
- **SPIES, C. M. ; R. H. STRAUB ; F. BUTTGEREIT.** Energy metabolism and rheumatic diseases: from cell to organism. *Arthritis Res. Ther.,* 2012, vol. 14, 216
- **RAMAKERS, B. P. ; N. P. RIKSEN ; J. G. VAN DER HOEVEN ; P. SMITS ; P. PICKKERS.** Modulation of innate immunity by adenosine receptor stimulation. *Shock,* 2011, vol. 36, 208-215
- **WILHELM, K. ; J. GANESAN ; T. MULLER ; C. DURR ; M. GRIMM ; A. BEILHACK ; C. D. KREMPL ; S. SORICHTER ; U. V. GERLACH ; E. JUTTNER.** Graft-versushost disease is enhanced by extracellular ATP activating P2X7R. *Nat. Med.,* 2010, vol. 16, 1434-1438
- **CAO, X. ; L. P. LI ; Q. WANG ; Q. WU ; H. H. HU ; M. ZHANG ; Y. Y. FANG ; J. ZHANG ; S. J. LI ; W. C. XIONG.** Astrocyte-derived ATP modulates depressive-like behaviors. *Nat. Med.,* 2013, vol. 19, 773-777
- **AKKAYA, C. ; E. SHUMILINA ; D. BOBBALLA ; V. B. BRAND ; H. MAHMUD ; F. LANG ; S. M. HUBER.** The Plasmodium falciparum-induced anion channel of human erythrocytes is an ATP-release pathway. *Pflugers Arch.,* 2009, vol. 457, 1035-1047
- **GUINEA, R. ; L. CARRASCO.** Requirement for vacuolar proton-ATPase activity during entry of influenza virus into cells. *J Virol,* 1995, 69
- **VAN AUBEL, R. A. ; P. H. SMEETS ; J. J. VAN DEN HEUVEL ; F. G. RUSSEL.** Human organic anion transporter MRP4 (ABCC4) is an efflux pump for the purine end metabolite urate with multiple allosteric substrate binding sites. *Am. J. Physiol Renal Physiol,* 2005, vol. 288, F327-F333
- **BREEDVELD, P. ; D. PLUIM ; G. CIPRIANI ; F. DAHLHAUS ; M. A. VAN EIJNDHOVEN ; C. J. DE WOLF ; A. KUIL ; J. H. BEIJNEN ; G. L. SCHEFFER ; G. JANSEN.** The effect of low pH on breast cancer resistance protein (ABCG2)-mediated transport of methotrexate, 7-hydroxymethotrexate, methotrexate diglutamate, folic acid, mitoxantrone, topotecan, and resveratrol in in vitro drug transport models. *Mol. Pharmacol.,* 2007, vol. 71, 240-249
- **DE WOLF. C. ; R. JANSEN ; H. YAMAGUCHI ; H. M. DE ; W. K. VAN DE ; J. WIJNHOLDS ; J. BEIJNEN ; P. BORST.** Contribution of the drug transporter ABCG2 (breast cancer resistance protein) to resistance against anticancer nucleosides. *Mol. Cancer Ther.,* 2008, vol. 7, 3092-3102
- **SCHWIEBERT, E. M. ; M. E. EGAN ; W. B. GUGGINO.** Assays of dynamics, mechanisms, and regulation of ATP transport and release: implications for study of ABC transporter function. *Methods Enzymol.,* 1998, vol. 292, 664-675
- **MANFREDI, G. ; L. YANG ; C. D. GAJEWSKI ; M. MATTIAZZI.** Measurements of ATP in mammalian cells. *Methods,* 2002, vol. 26, 317-326
- **HIGASHI, T. ; A. ISOMOTO ; I. TYUMA ; E. KAKISHITA ; M. UOMOTO ; K. NAGAI.** Quantitative and continuous analysis of ATP release from blood platelets with firefly luciferase luminescence. *Thromb. Haemost.,* 1985, vol. 53, 65-69
- **OZVEGY, C. ; T. LITMAN ; G. SZAKACS ; Z. NAGY ; S. BATES ; A. VARADI ; B. SARKADI.** Functional characterization of the human multidrug transporter, ABCG2, expressed in insect cells. *Biochem. Biophys. Res. Commun.,* 2001, vol. 285, 111-117
- **BREEDVELD, P. ; N. ZELCER ; D. PLUIM ; O. SONMEZER ; M. M. TIBBEN ; J. H. BEIJNEN ; A. H. SCHINKEL ; O. VAN TELLINGEN ; P. BORST ; J. H. M. SCHELLENS.** Mechanism of the Pharmacokinetic Interaction between Methotrexate and Benzimidazoles: Potential Role for Breast Cancer Resistance Protein in Clinical Drug-Drug Interactions. *Cancer Res,* 2004, vol. 64, 5804-5811
- **CANTRILL, C. A. ; R. A. SKINNER ; N. J. ROTHWELL ; J. I. PENNY.** An immortalised astrocyte cell line maintains the in vivo phenotype of a primary porcine in vitro blood-brain barrier model. *Brain Res.,* 2012, vol. 1479, 17-30
- **SZAKACS, G. ; J. K. PATERSON ; J. A. LUDWIG ; C. BOOTH-GENTHE ; M. M. GOTTESMAN.** Targeting multidrug resistance in cancer. *Nat. Rev. Drug Discov.,* 2006, vol. 5, 219-234

- **MALIEPAARD, M. ; G. L. SCHEFFER ; I. F. FANEYTE ; M. A. VAN GASTELEN ; A. C. PIJNENBORG ; A. H. SCHINKEL ; M. J. VAN DE VIJVER ; R. J. SCHEPER ; J. H. SCHELLENS.** Subcellular localization and distribution of the breast cancer resistance protein transporter in normal human tissues. *Cancer Res.,* 2001, vol. 61, 3458-3464
- **ARONICA, E. ; J. A. GORTER ; S. REDEKER ; E. A. VAN VLIET ; M. RAMKEMA ; G. L. SCHEFFER ; R. J. SCHEPER ; D. VAN, V, S. LEENSTRA ; J. C. BAAYEN ; W. G. SPLIET.** Localization of breast cancer resistance protein (BCRP) in microvessel endothelium of human control and epileptic brain. *Epilepsia,* 2005, vol. 46, 849-857
- **ZHOU, S. ; J. D. SCHUETZ ; K. D. BUNTING ; A. M. COLAPIETRO ; J. SAMPATH ; J. J. MORRIS ; I. LAGUTINA ; G. C. GROSVELD ; M. OSAWA ; H. NAKAUCHI.** The ABC transporter Bcrp1/ABCG2 is expressed in a wide variety of stem cells and is a molecular determinant of the side-population phenotype. *Nat. Med.,* 2001, vol. 7, 1028-1034
- **VAN DE. VEN. ; J. J. LINDENBERG ; A. W. REURS ; R. J. SCHEPER ; G. L. SCHEFFER ; T. D. DE GRUIJL.** Preferential Langerhans cell differentiation from CD34(+) precursors upon introduction of ABCG2 (BCRP. *Immunol. Cell Biol.,* 2012, vol. 90, 206-215
- **SZATMARI, I. ; G. VAMOSI ; P. BRAZDA ; B. L. BALINT ; S. BENKO ; L. SZELES ; V. JENEY ; C. OZVEGY-LACZKA ; A. SZANTO ; E. BARTA.** Peroxisome proliferator-activated receptor gamma-regulated ABCG2 expression confers cytoprotection to human dendritic cells. *J. Biol. Chem.,* 2006, vol. 281, 23812-23823
- **SUKOWATI, C. H. ; N. ROSSO ; D. PASCUT ; B. ANFUSO ; G. TORRE ; P. FRANCALANCI ; L. S. CROCE ; C. TIRIBELLI.** Gene and functional up-regulation of the BCRP/ABCG2 transporter in hepatocellular carcinoma. *BMC. Gastroenterol.,* 2012, vol. 12, 160
- **HUANG, L. ; Q. LU ; Y. HAN ; Z. LI ; Z. ZHANG ; X. LI.** ABCG2/V-ATPase was associated with the drug resistance and tumor metastasis of esophageal squamous cancer cells. *Diagn. Pathol.,* 2012, vol. 7, 180
- **JIN, Y. ; Z. Q. BIN ; H. QIANG ; C. LIANG ; C. HUA ; D. JUN ; W. A. DONG ; L. QING.** ABCG2 is related with the grade of glioma and resistance to mitoxantone, a chemotherapeutic drug for glioma. *J. Cancer Res. Clin. Oncol.,* 2009, vol. 135, 1369-1376
- **KOOIJ, G. ; R. BACKER ; J. J. KONING ; A. REIJERKERK ; H. J. VAN ; S. M. VAN DER POL ; J. DREXHAGE ; A. SCHINKEL ; C. D. DIJKSTRA ; J. M. DEN HAAN.** P-glycoprotein acts as an immunomodulator during neuroinflammation. *PLoS. ONE.,* 2009, vol. 4, 8212
- **DE MILITO. A. ; R. CANESE ; M. L. MARINO ; M. BORGHI ; M. LERO ; A. VILLA ; G. VENTURI ; F. LOZUPONE ; E. LESSI ; M. LOGOZZI.** pH-dependent antitumor activity of proton pump inhibitors against human melanoma is mediated by inhibition of tumor acidity. *Int. J. Cancer,* 2002, vol. 127, 207-219
- **LIU, H. T. ; A. H. TOYCHIEV ; N. TAKAHASHI ; R. Z. SABIROV ; Y. OKADA.** Maxianion channel as a candidate pathway for osmosensitive ATP release from mouse astrocytes in primary culture. *Cell Res.,* vol. 18, 558-565
- **BHATIA, P. ; M. BERNIER ; M. SANGHVI ; R. MOADDEL ; R. SCHWARTING ; A. RAMAMOORTHY ; I. W. WAINER.** Breast cancer resistance protein (BCRP/ABCG2) localises to the nucleus in glioblastoma multiforme cells. *Xenobiotica,* 2012, vol. 42, 748-755
- **DALLAS, S. ; D. S. MILLER ; R. BENDAYAN.** Multidrug resistance-associated proteins: expression and function in the central nervous system. *Pharmacol. Rev.,* 2006, vol. 58, 140-161
- **CALATOZZOLO, C. ; M. GELATI ; E. CIUSANI ; F. L. SCIACCA ; B. POLLO ; L. CAJOLA ; C. MARRAS ; A. SILVANI ; L. VITELLARO-ZUCCARELLO ; D. CROCI.** Expression of drug resistance proteins Pgp, MRP1, MRP3, MRP5 and GST-pi in human glioma. *J. Neurooncol.,* 2005, vol. 74, 113-121
- **CALCINOTTO, A. ; P. FILIPAZZI ; M. GRIONI ; M. LERO ; M. A. DE ; A. RICUPITO ; A. COVA ; R. CANESE ; E. JACHETTI ; M. ROSSETTI.** Modulation of microenvironment acidity reverses anergy in human and murine tumor-infiltrating T lymphocytes. *Cancer Res.,* 2012, vol. 72, 2746-2756
- **ROSS, D. D. ; J. E. KARP ; T. T. CHEN ; L. A. DOYLE.** Expression of breast cancer resistance protein in blast cells from patients with acute leukemia. *Blood,* 2000, vol. 96, 365-368
- **KRISHNAMURTHY, P. ; D. D. ROSS ; T. NAKANISHI ; K. BAILEY-DELL ; S. ZHOU ; K. E. MERCER ; B. SARKADI ; B. P. SORRENTINO ; J. D. SCHUETZ.** The stem cell marker Bcrp/ABCG2 enhances hypoxic cell survival through interactions with heme. *J. Biol. Chem.,* 2004, vol. 279, 24218-24225
- **AHMED, F. ; N. ARSENI ; H. GLIMM ; W. HIDDEMANN ; C. BUSKE ; M. FEURING-BUSKE.** Constitutive expression of the ATP-binding cassette transporter ABCG2 enhances the growth potential of early human hematopoietic progenitors. *Stem Cells,* 2008, vol. 26, 810-818
- **LUCIANI, F. ; M. SPADA ; A. DE MILITO ; A. MOLINARI ; L. RIVOLTINI ; A. MONTINARO ; M. MARRA ; L. LUGINI ; M. LOGOZZI ; F. LOZUPONE.** Effect of Proton Pump Inhibitor Pretreatment on Resistance of Solid Tumors to Cytotoxic Drugs. *J Natl Cancer Inst,* 2004, vol. 96, 1702-1713

- **HJELMELAND, A. B. ; Q. WU ; J. M. HEDDLESTON ; G. S. CHOUDHARY ; J. MACSWORDS ; J. D. LATHIA ; R. MCLENDON ; D. LINDNER ; A. SLOAN ; J. N. RICH.** Acidic stress promotes a glioma stem cell phenotype. *Cell Death. Differ.,* 2011, vol. 18, 829-840
- **AARNTZEN, E. H. ; I. J. DE VRIES ; W. J. LESTERHUIS ; D. SCHUURHUIS ; J. F. JACOBS ; K. BOL ; G. SCHREIBELT ; R. MUS ; J. H. DE WILT ; J. B. HAANEN.** Targeting CD4(+) T-helper cells improves the induction of antitumor responses in dendritic cell-based vaccination. *Cancer Res.,* 2013, vol. 73, 19-29
- **RAZIS, E. ; P. SELVIARIDIS ; S. LABROPOULOS ; J. L. NORRIS ; M. J. ZHU ; D. D. SONG ; T. KALEBIC ; M. TORRENS ; A. KALOGERA-FOUNTZILA ; G. KARKAVELAS.** Phase II study of neoadjuvant imatinib in glioblastoma: evaluation of clinical and molecular effects of the treatment. *Clin. Cancer Res.,* 2009, vol. 15, 6258-6266
- **FRITZELL, S. ; E. SANDEN ; S. EBERSTAL ; E. VISSE ; A. DARABI ; P. SIESJO.** Intratumoral temozolomide synergizes with immunotherapy in a T cell-dependent fashion. *Cancer Immunol. Immunother.,* 2013
- **MILANO, F. ; K. K. KRISHNADATH.** Novel therapeutic strategies for treating esophageal adenocarcinoma: the potential of dendritic cell immunotherapy and combinatorial regimens. *Hum. Immunol.,* 2008, vol. 69, 614-624
- **GUPTA, R. K.** Aluminum compounds as vaccine adjuvants. *Adv. Drug Deliv. Rev.,* 1998, vol. 32, 155-172
- **LI, H., S. NOOKALA ; F. RE.** Aluminum hydroxide adjuvants activate caspase-1 and induce IL-1 beta and IL-18 release. *J. Immunol.,* 2007, vol. 178, 5271-5276
- **MARCHESINI, N. ; M. VIEIRA ; S. LUO ; S. N. MORENO ; R. DOCAMPO.** A malaria parasite-encoded vacuolar H(+)-ATPase is targeted to the host erythrocyte. *J. Biol. Chem.,* 2005, vol. 280, 36841-36847
- **VAN DE HOEF, D. L. ; I. COPPENS ; T. HOLOWKA ; M. C. BEN ; O. BRANCH ; A. RODRIGUEZ.** Plasmodium falciparum-derived uric acid precipitates induce maturation of dendritic cells. *PLoS. ONE.,* 2013, vol. 8, 55584
- **REY-LADINO, J. ; A. G. ROSS ; A. W. CRIPPS ; D. P. MCMANUS ; R. QUINN.** Natural products and the search for novel vaccine adjuvants. *Vaccine,* 2011, vol. 29, 6464-6471
- **RIVERA, E. ; S. HU ; C. CONCHA.** Ginseng and aluminium hydroxide act synergistically as vaccine adjuvants. *Vaccine,* 2003, vol. 21, 1149-1157
- **EICHHORN, T. ; T. EFFERTH.** P-glycoprotein and its inhibition in tumors by phytochemicals derived from Chinese herbs. *J. Ethnopharmacol.,* 2012, vol. 141, 557-570
- **YU, X. Y. ; S. G. LIN ; Z. W. ZHOU ; X. CHEN ; J. LIANG ; P. Q. LIU ; W. DUAN ; B. CHOWBAY ; J. Y. WEN ; C. G. LI.** Role of P-Glycoprotein in the intestinal absorption of tanshinone IIA, a major active ingredient in the root of Salvia miltiorrhiza Bunge. *Current Drug Metabolism,* 2007, vol. 8, 325-340
- **MORRIS, M. E. ; S. ZHANG.** Flavonoid-drug interactions: effects of flavonoids on ABC transporters. *Life Sci.,* 2006, vol. 78, 2116-2130
- **JIN, J. ; S. SHAHI ; H. K. KANG ; H. W. VAN VEEN ; T. P. FAN.** Metabolites of ginsenosides as novel BCRP inhibitors. *Biochem. Biophys. Res. Commun.,* 2006, vol. 345, 1308-1314
- **TEN, B. A. ; M. L. KARSTEN ; M. C. DIEKER ; J. J. ZWAGINGA ; S. M. VAN HAM.** The clinical grade maturation cocktail monophosphoryl lipid A plus IFNgamma generates monocyte-derived dendritic cells with the capacity to migrate and induce Th1 polarization. *Vaccine,* 2007, vol. 25, 7145-7152
- **JONULEIT, H. ; U. KUHN ; G. MULLER ; K. STEINBRINK ; L. PARAGNIK ; E. SCHMITT ; J. KNOP ; A. H. ENK.** Pro-inflammatory cytokines and prostaglandins induce maturation of potent immunostimulatory dendritic cells under fetal calf serum-free conditions. *Eur. J. Immunol.,* 1997, vol. 27, 3135-3142
- **DAUER, M. ; B. OBERMAIER ; J. HERTEN ; C. HAERLE ; K. POHL ; S. ROTHENFUSSER ; M. SCHNURR ; S. ENDRES ; A. EIGLER.** Mature dendritic cells derived from human monocytes within 48 hours: a novel strategy for dendritic cell differentiation from blood precursors. *J. Immunol.,* 2003, vol. 170, 4069-4076
- **DE VRIES, N. A. ; S. W. BRUGGEMAN ; D. HULSMAN ; H. I. DE VRIES ; J. ZEVENHOVEN ; T. BUCKLE ; B. C. HAMANS ; W. P. LEENDERS ; J. H. BEIJNEN ; L. M. VAN.** Rapid and robust transgenic high-grade glioma mouse models for therapy intervention studies. *Clin. Cancer Res.,* 2010, vol. 16, 3431-3441